# EUROPÄISCHE PATENTSCHRIFT

(11) **EP 2 379 540 B1**
(45) Veröffentlichungstag und Bekanntmachung des Hinweises auf die Patenterteilung: **22.10.2014**
(21) Anmeldenummer: 09764200.3
(22) Anmeldetag: 03.12.2009
(51) Int. Cl.: C07D 413/12, A61K 31/4439, C07K 5/062, C07K 5/065, C07K 5/068, C07K 5/072, C07K 5/078

(54) **DIPEPTOID-PRODRUGS UND IHRE VERWENDUNG**
DIPEPTOID PRODRUGS AND THE USE THEREOF
PROMÉDICAMENTS DIPEPTOÏDES ET LEUR UTILISATION

(30) Priorität: 16.12.2008 DE 102008062567
(43) Veröffentlichungstag der Anmeldung: 26.10.2011
(73) Patentinhaber: Bayer Intellectual Property GmbH, 40789 Monheim (DE)
(72) Erfinder: LERCHEN, Hans-Georg, 51375 Leverkusen (DE); MEIBOM, Daniel, 51373 Leverkusen (DE); VAKALOPOULOS, Alexandros, 40721 Hilden (DE); ALBRECHT-KÜPPER, Barbara, 42489 Wülfrath (DE); KELDENICH, Jörg, 42113 Wuppertal (DE); ZIMMERMANN, Katja, 40489 Düsseldorf (DE); NELL, Peter, Woodside, CA 94062 (US); KRENZ, Ursula, 42799 Leichlingen (DE)
(74) Vertreter: BIP Patents
(86) Internationale Anmeldenummer: PCT/EP2009/008617
(87) Internationale Veröffentlichungsnummer: WO 2010/072314

(56) Entgegenhaltungen:
- WO-A1-03/053441
- WO-A1-2009/015776
- WO-A1-2009/015811
- WO-A2-01/25210
- BEAUMONT K ET AL: "DESIGN OF ESTER PRODRUGS TO ENHANCE ORAL ABSORPTION OF POORLY PERMEABLE COMPOUNDS: CHALLENGES TO THE DISCOVERY SCIENTIST" CURRENT DRUG METABOLISM, BENTHAM SCIENCE PUBLISHERS, US, Bd. 4, Nr. 6, 1. Januar 2003 (2003-01-01), Seiten 461-485, XP008058352 ISSN: 1389-2002 in der Anmeldung erwähnt

## Beschreibung

Die vorliegende Anmeldung betrifft Dipeptid-ähnliche Prodrug-Derivate von 2-Amino-6-({[2-(4-chlorphenyl)-1,3-oxazol-4-yl]methyl}sulfanyl)-4-(4-{[2,3-dihydroxypropyl]oxy}phenyl)pyridin-3,5-dicarbonitril, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären Erkrankungen.

Prodrugs sind Derivate eines Wirkstoffs, die *in vivo* eine ein- oder mehrstufige Biotransformation enzymatischer und/oder chemischer Art durchlaufen, bevor der eigentliche Wirkstoff freigesetzt wird. Ein Prodrug-Rest wird in der Regel genutzt, um das Eigenschaftsprofil des zugrunde liegenden Wirkstoffs zu verbessern [P. Ettmayer et al., J. Med Chem. 47, 2393-2404 (2004)]. Um ein optimales Wirkprofil zu erreichen, muss dabei das Design des Prodrug-Restes ebenso wie der angestrebte Freisetzungsmechanismus sehr genau auf den individuellen Wirkstoff, die Indikation, den Wirkort und die Applikationsroute abgestimmt werden. Eine große Zahl von Arzneimitteln wird als Prodrugs verabreicht, die gegenüber dem zu Grunde liegenden Wirkstoff eine verbesserte Bioverfügbarkeit aufweisen, beispielsweise erzielt durch eine Verbesserung des physikochemischen Profils, speziell der Löslichkeit, der aktiven oder passiven Absorptionseigenschaften oder der gewebsspezifischen Verteilung. Aus der umfangreichen Literatur über Prodrugs sei beispielhaft genannt: H. Bundgaard (Ed.), Design of Prodrugs: Bioreversible derivatives for various functional groups and chemical entities, Elsevier Science Publishers B.V., 1985.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Wirkungen als hormonähnliche Substanz oder Neurotransmitter aus. Bekannt sind bisher die Rezeptor-Subtypen A1, A2a, A2b und A3 [vgl. K. A. Jacobson und Z.-G. Gao, Nat. Rev. Drug Discover. 5 247-264 (2006)].

Die Aktivierung von A1-Rezeptoren durch spezifische A1-Agonisten führt beim Menschen zu einer frequenzabhängigen Senkung der Herzfrequenz, ohne einen Einfluss auf den Blutdruck zu haben. Selektive A1-Agonisten könnten somit unter anderem für die Behandlung von Angina pectoris und Vorhofflimmern geeignet sein.

Die Aktivierung von A2b-Rezeptoren durch Adenosin oder spezifische A2b-Agonisten führt über die Erweiterung von Gefäßen zu einer Blutdrucksenkung. Die Blutdrucksenkung ist von einem reflektorischen Herzfrequenzanstieg begleitet. Der Herzfrequenzanstieg kann durch die Aktivierung von A1-Rezeptoren durch spezifische A1-Agonisten reduziert werden.

Die kombinierte Wirkung von selektiven Al/A2b-Agonisten auf das Gefäßsystem und die Herzfrequenz resultiert somit in einer systemischen Blutdrucksenkung ohne relevanten Herzfrequenzanstieg. Mit einem solchen pharmakologischen Profil könnten duale A1/A2b-Agonisten zur Behandlung z.B. der Hypertonie beim Menschen eingesetzt werden.

Die Verbindung 2-Amino-6-({[2-(4-chlorphenyl)-1,3-oxazol-4-yl]methyl}sulfanyl)-4-(4-{[2,3-di-hydroxypropyl]oxy}phenyl)pyridin-3,5-dicarbonitril der Formel (A) 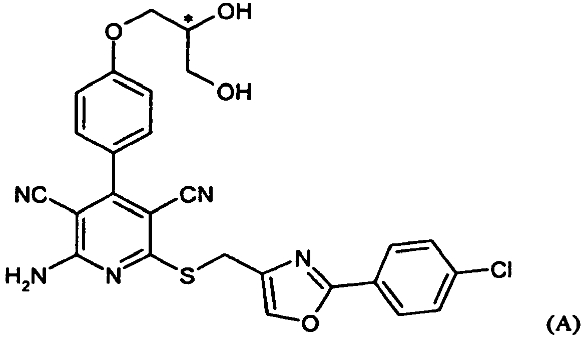 ist ein potenter und selektiver Adenosin Al-Rezeptoragonist mit einer gewissen dualen, A2b-agonistischen Wirkkomponente (siehe PCT-Anmeldung WO2009/015776-A1). Die Substanz befindet sich gegenwärtig in vertiefter Untersuchung als möglicher neuer Arzneimittelwirkstoff für die Prävention und Therapie insbesondere von kardiovaskulären Erkrankungen. Von besonderer Bedeutung hierbei ist die enantiomerenreine Form der Verbindung (A), welche am C*-Kohlen-stoffatom der Propan-1,2-diol-Gruppe eine *R*-Konfiguration besitzt.

Die Verbindung (A) weist jedoch nur eine begrenzte Löslichkeit in Wasser, physiologischen Medien und organischen Lösungsmitteln sowie eine nur geringe Bioverfügbarkeit nach oraler Applikation einer Suspension kristallinen Materials auf. Dies erlaubt einerseits eine intravenöse Applikation des Wirkstoffs nur in sehr geringen Dosierungen; Infusionslösungen auf Basis physiologischer Salzlösungen sind mit gebräuchlichen Lösungsvermittlern nur schwer herstellbar. Andererseits ist eine Formulierung in Tablettenform erschwert.

Aufgabe der vorliegenden Erfindung war daher die Identifizierung von Derivaten oder Prodrugs von Verbindung (A), die eine verbesserte Löslichkeit in den genannten Medien und/oder eine verbesserte Bioverfügbarkeit nach oraler Applikation besitzen und gleichzeitig nach Applikation eine kontrollierte Freisetzung des Wirkstoffs (A) im Körper des Patienten ermöglichen. Durch eine verbesserte intravenöse Applizierbarkeit könnten zudem weitere therapeutische Einsatzgebiete für diesen Wirkstoff erschlossen werden.

Eine Übersicht zu Prodrug-Derivaten auf Basis von Carbonsäureestem und möglichen Eigenschaften solcher Verbindungen ist beispielsweise in K. Beaumont et al., Curr. Drug Metab. 4, 461-485 (2003) gegeben.

Gegenstand der vorliegenden Erfindung sind Verbindungen der allgemeinen Formel (I) 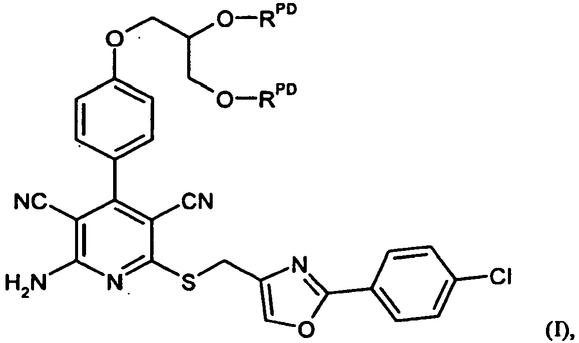 in welcher
- R^{PD}: für eine Gruppe der Formel 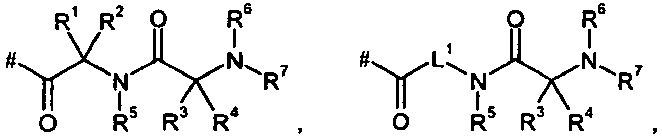 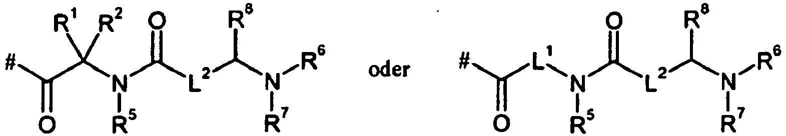 steht, worin
# die Verknüpfungsstelle mit dem jeweiligen O-Atom bedeutet,
L¹ geradkettiges (C₂-C₄)-Alkandiyl bedeutet,
L² geradkettiges (C₁-C₃)-Alkandiyl bedeutet,
R¹ und R³ gleich oder verschieden sind und unabhängig voneinander Wasserstoff oder Methyl (Alanin), Propan-2-yl (Valin), Propan-1-yl (Norvalin), 2-Methylpropan-1-yl (Leucin), 1-Methylpropan-1-yl (Isoleucin), Butan-1-yl (Norleucin), *tert*.-Butyl (2-*tert*,-Butylglycin), Phenyl (2-Phenylglycin), Benzyl (Phenylalanin), *p*-Hydroxy-benzyl (Tyrosin), Indol-3-ylmethyl (Tryptophan), Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (S-Methyeystein), 2-Mercaptoethyl (Homocystein), 2-Methylthioethyl (Methionin), Carbamoylmethyl (Asparagin), 2-Carbamoylethyl (Glutamin), Carboxymethyl (Asparaginsäure), 2-Carboxyethyl (Glutaminsäure), 4-Aminobutan-1-yl (Lysin), 4-Amino-3-hydroxybutan-1-yl (Hydroxylysin), 3-Aminopropan-1-yl (Omithin), 2-Aminoethyl (2,4-Diaminobuttersäure), Aminomethyl (2,3-Diaminopropionsäure), 3-Guanidinopropan-1-yl (Arginin), 3-Ureidoprapan-1-yl (Citrullin) bedeuten,
R² und R⁴ unabhängig voneinander Wasserstoff oder Methyl bedeuten
oder
R¹ und R² oder R³ und R⁴ jeweils miteinander verknüpft sind und zusammen mit dem Kohlenstoffätom, an das sie gemeinsam gebunden sind, einen 3- bis 6-gliedrigen gesättigten Carbocyclus bilden,
R⁵ Wasserstoff oder (C₁-C₄)-Alkyl bedeutet
oder
R⁵ mit R¹ verknüpft ist und beide zusammen mit den Atomen, an die sie gebunden sind, einen Pyrrolidin- oder Piperidin-Ring bilden,
R⁶ und R⁷ gleich oder verschieden sind und unabhängig voneinander Wasserstoff oder (C₁-C₄)-Alkyl, das mit Hydroxy, (C₁-C₄)-Alkoxy, Amino, Mono-(C₁-C₄)-alkylamino oder Di-(C₁-C₄)-alkylamino substituiert sein kann, bedeuten
oder
R⁶ und R⁷ miteinander verknüpft sind und zusammen mit dem Stickstoffatom, an das sie gebunden sind, einen 5- oder 6-gliedrigen gesättigten Heterocyclus bilden, der ein weiteres Ring-Heteroatom aus der Reihe N und O enthalten und ein- oder zweifach, gleich oder verschieden, mit (C₁-C₄)-Alkyl, Amino, Hydroxy und/oder (C₁-C₄)-Alkoxy substituiert sein kann,
oder
R⁶ mit R³ verknüpft ist und beide zusammen mit den Atomen, an die sie gebunden sind, einen Pyrrolidin- oder Piperidin-Ring bilden
und
R⁸ Wasserstoff oder Carboxyl bedeutet,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können. Neben Mono-Salzen sind von der vorliegenden Erfindung auch gegebenenfalls mögliche Mehrfach-Salze wie Di- oder Tri-Salze eingeschlossen.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Cholin, Dicyclohexylamin, Dimethylaminoethanol, Prokain, Dibenzylamin, Morpholin, N-Methylmorpholin, Arginin, Lysin, Ethylendiamin, Piperidin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
(C₁-C₄)-Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, n-Propyl, Isopropyl, *n*-Butyl, Isobutyl, *sec*.-Butyl, *tert.-*Butyl*.*
(C₂-C₄)-Alkandiyl steht im Rahmen der Erfindung für einen geradkettigen, α,ω-divalenten Alkylrest mit 2 bis 4 Kohlenstoffatomen. Beispielhaft und vorzugsweise seien genannt: Ethan-1,2-diyl (1,2-Ethylen), Propan-1,3-diyl (1,3-Propylen), Butan-1,4-diyl (1,4-Butylen).
(C₁-C₃)-Alkandiyl steht im Rahmen der Erfindung für einen geradkettigen, α,ω-divalenten Alkylrest mit 1 bis 3 Kohlenstoffatomen. Beispielhaft und vorzugsweise seien genannt: Methandiyl (Methylen), Ethan-1,2-diyl (1,2-Ethylen), Propan-1,3-diyl (1,3-Propylen).
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy, *tert*.-Butoxy.
Mono-(C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n*-Butylamino, *tert*.-Butylamino.
Di-(C₁-C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N* Diethylamino, *N*-Ethyl-*N*-methylamino, *N*-Methyl-*N*-*n*-propylamino, *N*-Isopropyl-*N-n*-propylamino, *N,N*-Diisopropylamino, *N*-*n*-Butyl-*N*-methylamino, *N*-*tert*.-Butyl-*N*-methylamino.
Ein 3- bis 6-gliedriger Carbocyclus steht im Rahmen der Erfindung für eine monocyclische, gesättigte Cycloalkyl-Gruppe mit 3 bis 6 Ring-Kohlenstoffatomen. Beispielhaft und vorzugsweise seien genannt: Cyclopropyl, Cyclobutyl, Cyclopentyl, Cyclohexyl.
Ein 5- oder 6-gliedriger Heterocyclus steht im Rahmen der Erfindung für eine monocyclische, gesättigte Heterocycloalkyl-Gruppe mit insgesamt 5 oder 6 Ringatomen, die ein Ring-Stickstoffatom enthält und gegebenenfalls ein zweites Ring-Heteroatom aus der Reihe N und 0 enthalten kann. Beispielhaft und vorzugsweise seien genannt: Pyrrolidinyl, Piperidinyl, Piperazinyl, Morpholinyl.
Die Seitengruppe einer α-Aminosäure in der Bedeutung von R¹ und R³ umfasst sowohl die Seitengruppen der natürlich vorkommenden α-Aminosäuren als auch die Seitengruppen von Homologen und Isomeren dieser α-Aminosäuren. Die α-Aminosäure kann hierbei sowohl in der L- als auch in der D-Konfiguration oder auch als Gemisch der L- und D-Form vorliegen. Als Seitengruppen seien beispielhaft genannt: Methyl (Alanin), Propan-2-yl (Valin), Propan-1-yl (Norvalin), 2-Methyl-propan-1-yl (Leucin), 1-Methylpropan-1-yl (Isoleucin), Butan-1-yl (Norleucin), *tert*.-Butyl (2-*tert.-*Butylglycin), Phenyl (2-Phenylglycin), Benzyl (Phenylalanin), *p*-Hydroxybenzyl (Tyrosin), Indol-3-ylmethyl (Tryptophan), Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (*S-*Methylcystein), 2-Mercaptoethyl (Homocystein), 2-Methylthioethyl (Methionin), Carbamoyl-methyl (Asparagin), 2-Carbamoylethyl (Glutamin), Carboxymethyl (Asparaginsäure), 2-Carboxy-ethyl (Glutaminsäure), 4-Aminobutan-1-yl (Lysin), 4-Amino-3-hydroxybutan-1-yl (Hydroxylysin), 3-Aminopropan-1-yl (Omithin), 2-Aminoethyl (2,4-Diaminobuttersäure), Aminomethyl (2,3-Diaminopropionsäure), 3-Guanidinopropan-1-yl (Arginin), 3-Ureidopropan-1-yl (Citrullin).

Bevorzugte α-Aminosäure-Seitengruppen in der Bedeutung von R¹ sind Methyl (Alanin), Propan-2-yl (Valin), 1-Methylpropan-1-yl (Isoleucin), 2-Methylpropan-1-yl (Leucin), Benzyl (Phenylalanin), *p*-Hydroxybenzyl (Tyrosin), Hydroxymethyl (Serin), 1-Hydroxyethyl (Threonin). Bevorzugt ist jeweils die L-Konfiguration.

Bevorzugte α-Aminosäure-Seitengruppen in der Bedeutung von R³ sind Methyl (Alanin), Propan2-yl (Valin), 1-Methylpropan-1-yl (Isoleucin), 2-Methylpropan-1-yl (Leucin), Benzyl (Phenylalanin), Imidazol-4-ylmethyl (Histidin), Hydroxymethyl (Serin), 1-Hydroxyethyl (Threonin), Carbamoylmethyl (Asparagin), 2-Carbamoylethyl (Glutamin), Carboxymethyl (Asparaginsäure), 2-Carboxyethyl (Glutaminsäure), 4-Aminobutan-1-yl (Lysin), 3-Aminopropan-1-yl (Ornithin), 2-Aminoethyl (2,4-Diaminobuttersäure), Aminomethyl (2,3-Diaminopropionsäure), 3-Guanidino-propan-1-yl (Arginin). Bevorzugt ist jeweils die L-Konfiguration.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Hierbei ist eine Substitution mit einem oder mit zwei gleichen oder verschiedenen Substituenten bevorzugt; besonders bevorzugt ist die Substitution mit einem Substituenten.

Bevorzugt im Rahmen der vorliegenden Erfindung sind Verbindungen der Formel (I), in welcher
- R^{PD}: für eine Gruppe der Formel 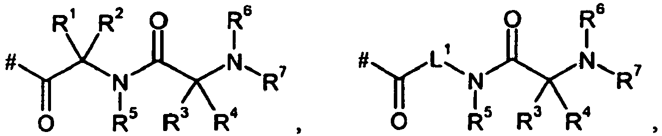 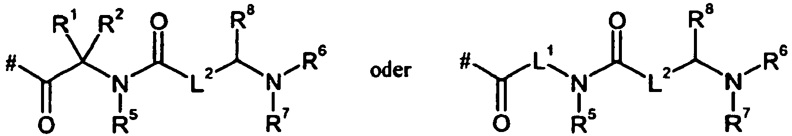 steht, worin
# die Verknüpfungsstelle mit dem jeweiligen O-Atom bedeutet,
L¹ Ethan-1,2-diyl bedeutet,
L² Methandiyl oder Ethan-1,2-diyl bedeutet,
R¹ Wasserstoff, Methyl, Propan-2-yl, 1-Methylpropan-1-yl, 2-Methylpropan-1-yl, Benzyl, *p*-Hydroxybenzyl, Hydroxymethyl oder 1-Hydroxyethyl bedeutet,
R² Wasserstoff bedeutet,
R³ Wasserstoff, Methyl, Propan-2-yl, 1-Methylpropan-1-yl, 2-Methylpropan-1-yl, Benzyl, Imidazol-4-ylmethyl, Hydroxymethyl, 1-Hydroxyethyl, Carbamoylmethyl, 2-Carbamoylethyl, Carboxymethyl, 2-Carboxyethyl, 4-Aminobutan-1-yl, 3-Amino-propan-1-yl, 2-Aminoethyl, Aminomethyl oder 3-Guanidinopropan-1-yl bedeutet,
R⁴ Wasserstoff bedeutet,
R⁵ Wasserstoff oder Methyl bedeutet oder
R⁵ mit R¹ verknüpft ist und beide zusammen mit den Atomen, an die sie gebunden sind, einen Pyrrolidin-Ring bilden,
R⁶ Wasserstoff oder Methyl bedeutet
oder
R⁶ mit R³ verknüpft ist und beide zusammen mit den Atomen, an die sie gebunden sind, einen Pyrrolidin-Ring bilden,
R⁷ Wasserstoff oder Methyl bedeutet und
R⁸ Wasserstoff oder Carboxyl bedeutet,
sowie ihre Salze, Solvate und Solvate der Salze.

Besonders bevorzugt im Rahmen der vorliegenden Erfindung sind Verbindungen der Formel (I), in welcher
- R^{PD}: für eine Gruppe der Formel 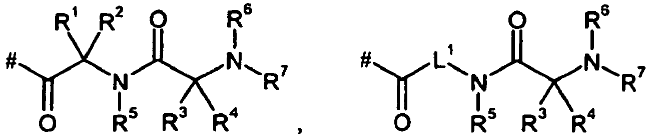 oder 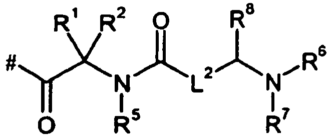 steht, worin
# die Verknüpfungsstelle mit dem jeweiligen O-Atom bedeutet,
L¹ Ethan-1,2-diyl bedeutet,
L² Methandiyl bedeutet,
R¹ Wasserstoff, Methyl, Propan-2-yl, 1-Methylpropan-1-yl, 2-Methylpropan-1-yl, Hydroxymethyl oder 1-Hydroxyethyl bedeutet,
R² Wasserstoff bedeutet,
R³ Wasserstoff, Methyl, Propan-2-yl, 1-Methylpropan-1-yl, 2-Methylpropan-1-yl, Imidazol-4-ylmethyl, Hydroxymethyl, 1-Hydroxyethyl, 2-Carboxyethyl, 4-Amino-butan-1-yl, 3-Aminopropan-1-yl oder 2-Aminoethyl bedeutet,
R⁴ Wasserstoff bedeutet,
R⁵ Wasserstoff bedeutet,
R⁶ Wasserstoff oder Methyl bedeutet
oder
R⁶ mit R³ verknüpft ist und beide zusammen mit den Atomen, an die sie gebunden sind, einen Pyrrolidin-Ring bilden,
R⁷ Wasserstoff bedeutet
und
R⁸ Wasserstoff bedeutet,
sowie ihre Salze, Solvate und Solvate der Salze.

Die beiden Prodrug-Gruppen R^{PD} in den Verbindungen der Formel (I) können innerhalb des oben angegebenen Bedeutungsumfangs gleich oder verschieden sein. Bevorzugt sind Verbindungen der Formel (I) mit jeweils identischen Prodrug-Gruppen R^{PD}.

Von besonderer Bedeutung sind die Verbindungen der Formeln (I-A) und (I-B) 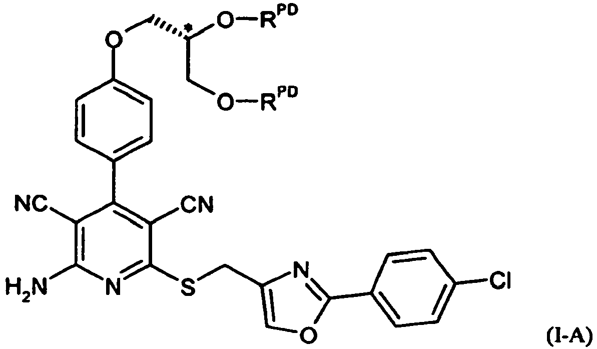 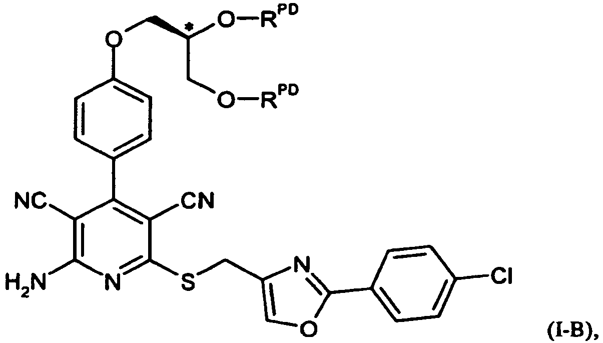 in welchen R^{PD} die oben angegebene Bedeutung hat,
mit einer S- bzw. R-Konfiguration am C*-Kohlenstoffatom der Propan-1,2,3-triyl-Gruppe sowie deren Salze, Solvate und Solvate der Salze.

Bevorzugt im Rahmen der vorliegenden Erfindung sind die Verbindungen der Formel (I-A) mit einer S-Konfiguration am C*-Kohlenstoffatom der Propan-1,2,3-triyl-Gruppe sowie deren Salze, Solvate und Solvate der Salze.

Besonders bevorzugt im Rahmen der vorliegenden Erfindung sind Verbindungen der Formel (I-A), in welchen die beiden Prodrug-Gruppen R^{PD} jeweils identisch sind, sowie deren Salze, Solvate und Solvate der Salze.

Weiterer Gegenstand der Erfindung ist ein Verfahren zur Herstellung der erfindungsgemäßen Verbindungen der Formel (I), in welchen die beiden Prodrug-Gruppen R^{PD} jeweils identisch sind, dadurch gekennzeichnet, dass man die Verbindung (A) 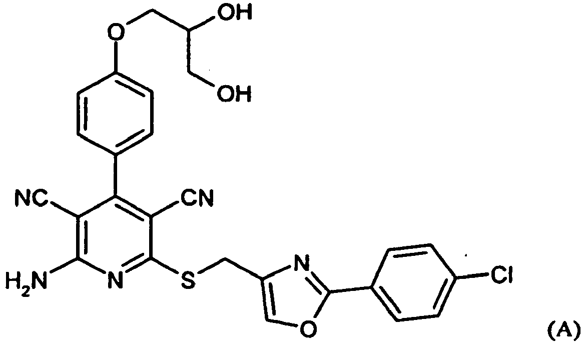 entweder
[A] in einem inerten Lösungsmittel in Gegenwart eines Kondensationsmittels zunächst mit zwei oder mehr Äquivalenten einer Aminosäure der Formel (II) oder (III) 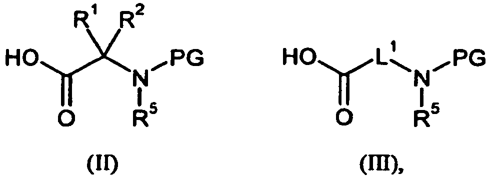 in welchen L¹, R¹, R² und R⁵ die oben angegebenen Bedeutungen haben
   und
   - PG: für eine temporäre Amino-Schutzgruppe wie beispielsweise tert.-Butoxycarbonyl steht,
   zu einer Verbindung der Formel (IV) bzw. (V) 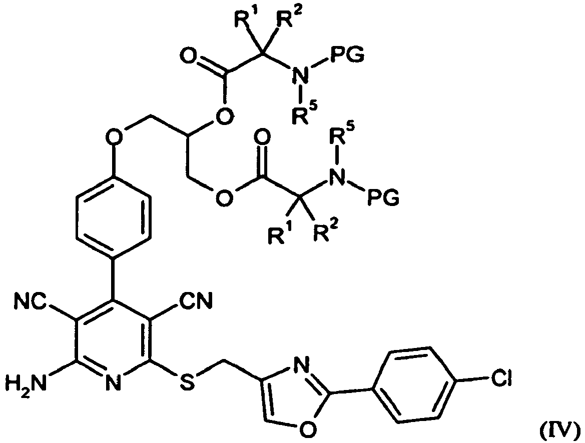 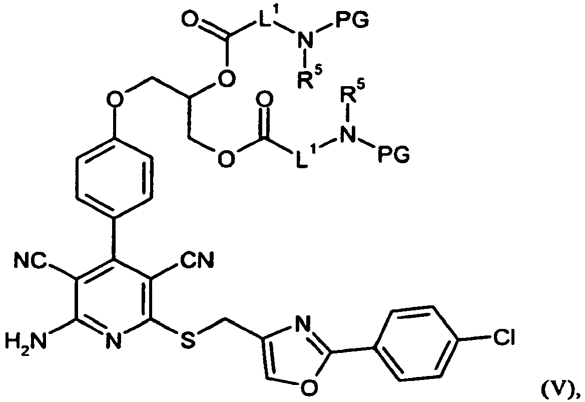 in welchen L¹, PG, R¹, R² und R⁵ die oben angegebenen Bedeutungen haben,
   verestert, diese nach Abspaltung der Schutzgruppen PG dann in einem inerten Lösungsmittel in Gegenwart eines Kondensationsmittels mit zwei oder mehr Äquivalenten einer Aminosäure der Formel (VI) oder (VII) 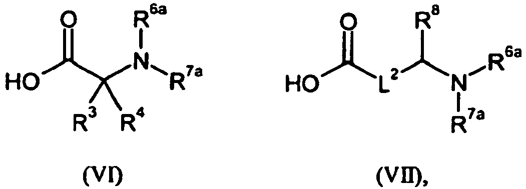 in welchen L², R³, R⁴ und R⁸ die oben angegebenen Bedeutungen haben und
   - R^{6a} und R^{7a}: gleich oder verschieden sind und die oben angegebenen Bedeutungen von R⁶ bzw. R⁷ haben oder für eine temporäre Amino-Schutzgruppe stehen,
   zu einer Verbindung der Formel (VIII), (IX), (X) bzw. (XI) 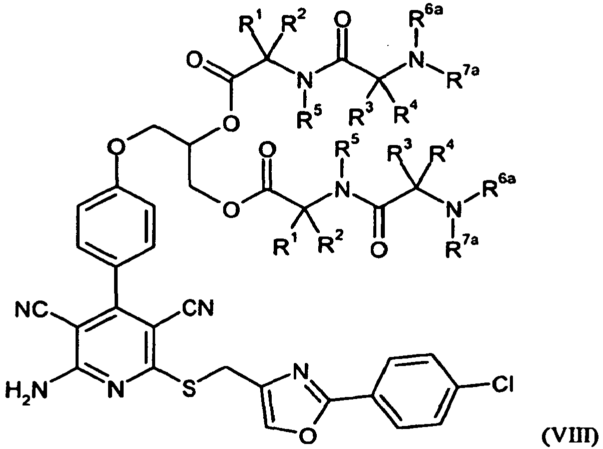 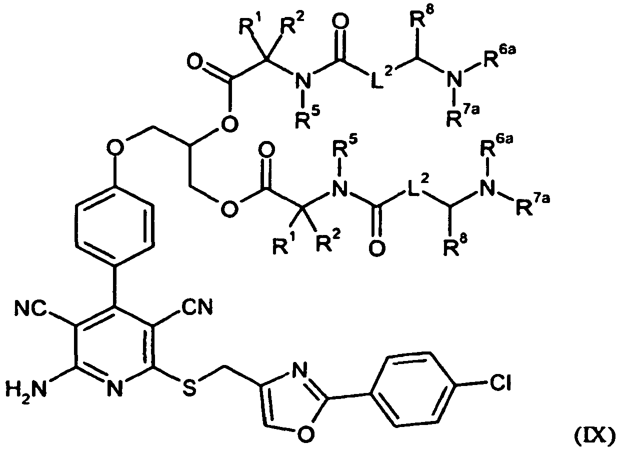 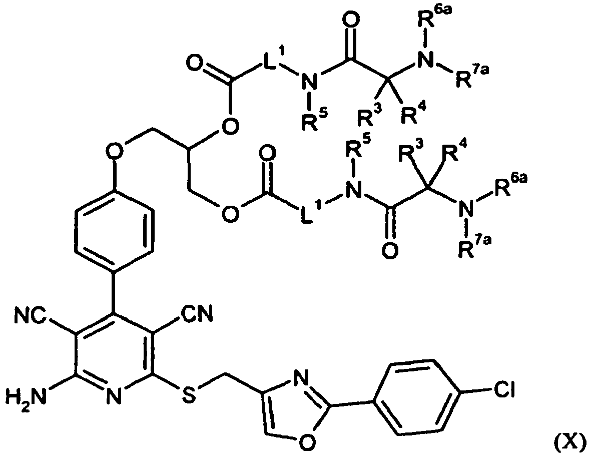 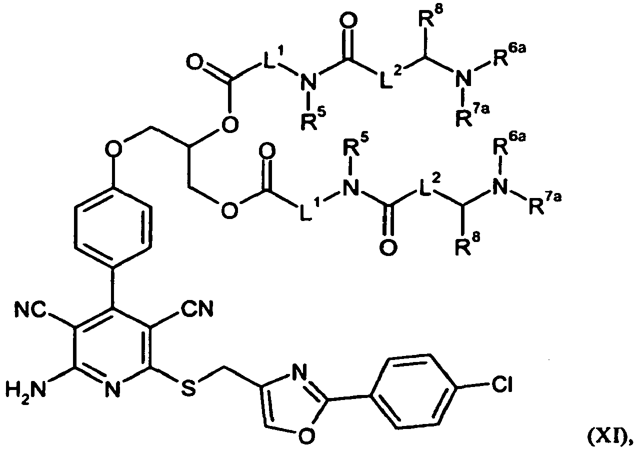 in welchen L¹, L², R¹, R², R³, R⁴, R⁵, R^{6a}, R^{7a} und R⁸ jeweils die oben angegebenen Bedeutungen haben,
   kuppelt und anschließend gegebenenfalls vorhandene Schutzgruppen wieder entfernt
   oder
[B] in einem inerten Lösungsmittel in Gegenwart eines Kondensationsmittels mit zwei oder mehr Äquivalenten einer Carbonsäure der Formel (XII), (XIII), (XIV) oder (XV) 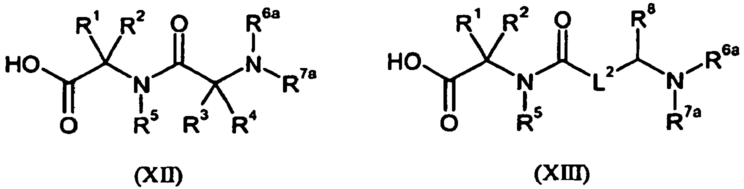 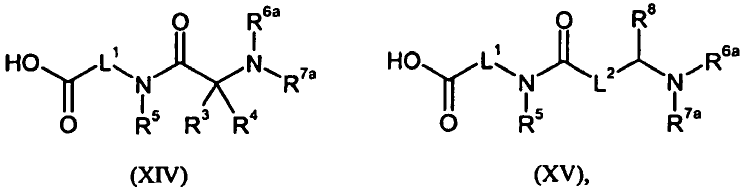 in welchen L¹, L², R¹, R², R³, R⁴, R⁵ und R⁸ die zuvor angegebenen Bedeutungen haben
   und
   - R^{6a} und R^{7a}: gleich oder verschieden sind und die oben angegebenen Bedeutungen von R⁶ bzw. R⁷ haben oder für eine temporäre Amino-Schutzgruppe stehen,
   zu einer der oben aufgeführten Verbindungen (VIII), (IX), (X) bzw. (XI) kuppelt und anschließend gegebenenfalls vorhandene Schutzgruppen wieder entfernt
und die jeweils resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Die Transformation (A) → (I) erfolgt somit entweder durch sequentielle Kupplung der einzelnen, gegebenenfalls geeignet geschützten Aminosäure-Komponenten (Verfahrensvariante [A]) oder durch direkte Acylierung mit einem geeignet geschützten Dipeptoid-Derivat (Verfahrensvariante [B]). Die Kupplungsreaktionen (Ester- bzw. Amid-Bildung) werden hierbei nach bekannten Methoden der Peptidchemie durchgeführt [vgl. z.B. M. Bodanszky, Principles of Peptide Synthesis, Springer-Verlag, Berlin, 1993; H.-D. Jakubke und H. Jeschkeit, Aminosäure, Peptide, Proteine, Verlag Chemie, Weinheim, 1982].

Inerte Lösungsmittel für die Kupplungsreaktionen sind beispielsweise Ether wie Diethylether, *tert*.-Butyl-methylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Pyridin, Dimethylsulfoxid, Dimethylformamid, *N,N*'-Dimethylpropylenharnstoff (DMPU), *N*-Methylpyrrolidon (NMP) oder Acetonitril. Ebenso ist es möglich, Gemische der genannten Lösungsmittel zu verwenden. Bevorzugt sind Dichlormethan, Dimethylformamid oder Gemische dieser beiden Lösungsmittel.

Als Kondensationsmittel bei diesen Kupplungsreaktionen eignen sich beispielsweise Carbodiimide wie *N,N*'-Diethyl-, *N,N*'-Dipropyl-, *N,N'*-Diisopropyl-, *N,N*'-Dicyclohexylcarbodiimid (DCC) oder *N*-(3-Dimethylaminoisopropyl)-*N*'-ethylcarbodiimid-Hydrochlorid (EDC), Phosgen-Derivate wie *N,N*'-Carbonyldiimidazol (CDI), 1,2-Oxazoliumverbindungen wie 2-Ethyl-5-phenyl-1,2-oxazolium-3-sulfat oder 2-*tert*.-Butyl-5-methylisoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris-(pyrrolidino)phosphonium-hexafluorophosphat (PyBOP), *O*-(Benzotriazol-1-yl)-*N,N,N',N*'-tetramethyluronium-tetrafluoroborat (TBTU), *O*-(Benzotriazol-1-yl)-*N,N,N',N*'-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N'N*'-tetramethyluronium-hexafluorophosphat (HATU) oder *O*-(1H-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder organische Aminbasen wie Triethylamin, *N*-Methylmorpholin, *N*-Methyl-piperidin, *N,N*-Diisopropylethylamin oder 4-*N,N*-Dimethylaminopyridin. Zur Ester-Bildung wird bevorzugt *N*-(3-Dimethylaminoisopropyl)-*N'*-ethylcarbodiimid-Hydrochlorid (EDC) in Kombination mit 4-*N,N*-Dimethylaminopyridin eingesetzt. Für die Amid-Bildung wird vorzugsweise *N*-(3-Dimethylaminoisopropyl)-N'-ethylcarbodiimid-Hydrochlorid (EDC) in Kombination mit 1-Hydroxybenzotriazol (HOBt) oder *N*-Hydroxysuccinimid (HOSu) und gegebenenfalls einer Base wie *N,N*-Diisopropylethylamin verwendet.

Die Kupplungen werden im Allgemeinen in einem Temperaturbereich von 0°C bis +60°C, bevorzugt bei +10°C bis +30°C durchgeführt. Die Reaktionen können bei normalem, bei erhöhtem oder bei erniedrigtem Druck erfolgen (z.B. von 0.5 bis 5 bar). Im Allgemeinen arbeitet man bei Normal-druck.

Die Verbindungen der Formel (I) können bei der Herstellung nach den oben beschriebenen Verfahren auch direkt in Form von Salzen entstehen. Diese Salze können gegebenenfalls durch Behandlung mit einer Base bzw. Säure in einem inerten Lösungsmittel, über chromatographische Methoden oder mittels Ionenaustauscherharzen in die jeweiligen freien Basen bzw. Säuren überführt werden. Weitere Salze der erfindungsgemäßen Verbindungen können gegebenenfalls auch durch Austausch von Gegenionen mit Hilfe der Ionenaustausch-Chromatographie, beispielsweise mit Amberlite^{®}-Harzen, hergestellt werden.

In den Verbindungen der Formeln (II), (VI), (VII), (XII), (XIII), (XIV) und (XV) bzw. in den Resten R¹, R³, R⁶, R⁷ und/oder R⁸ gegebenenfalls vorhandene funktionelle Gruppen - wie insbesondere Amino-, Guanidino-, Hydroxy-, Mercapto- und Carboxylgruppen - können bei den zuvor beschriebenen Reaktionssequenzen, falls zweckmäßig oder erforderlich, auch in temporär geschützter Form vorliegen. Die Einführung und Entfernung solcher Schutzgruppen erfolgt hierbei nach üblichen, aus der Peptidchemie bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984].

Als Amino- und Guanidino-Schutzgruppe wird bevorzugt *tert*.-Butoxycarbonyl (Boc) oder Benzyloxycarbonyl (Z) verwendet. Als Schutzgruppe für eine Hydroxy- oder Carboxyl-Funktion wird vorzugsweise *tert*.-Butyl oder Benzyl eingesetzt. Die Abspaltung dieser Schutzgruppen wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Dichlormethan oder Essigsäure durchgeführt; gegebenenfalls kann die Abspaltung auch ohne ein zusätzliches inertes Lösungsmittel erfolgen. Im Falle von Benzyl und Benzyloxycarbonyl als Schutzgruppe können diese auch Hydrogenolyse in Gegenwart eines Palladium-Katalysators entfernt werden. Die Abspaltung der genannten Schutzgruppen kann gegebenenfalls simultan in einer Eintopf-Reaktion oder in separaten Reaktionsschritten vorgenommen werden.

Die Verbindungen der Formeln (II), (III), (VI), (VII), (XII), (XIII), (XIV) und (XV) sind kommerziell erhältlich oder literaturbekannt, oder sie können nach literaturüblichen Methoden hergestellt werden.

Erfindungsgemäße Verbindungen der Formel (I), in welchen die beiden Prodrug-Gruppen R^{PD} nicht identisch sind, können in Analogie zu dem oben beschriebenen Verfahren dadurch hergestellt werden, dass man die Verbindung (A) in separaten Schritten mit jeweils einem Äquivalent von entsprechend unterschiedlichen Verbindungen der Formeln (II), (III), (VI), (VII), (XII), (XIII), (XIV) und/oder (XV) kuppelt und hierbei anfallende Produktgemische dann - gegebenenfalls vor oder nach der Abspaltung temporärer Schutzgruppen - in die einzelnen Komponenten auftrennt. Für eine solche Trennung werden bevorzugt chromatographische Methoden, wie die Chromatographie an Kieselgel oder Aluminiumoxid oder die HPLC-Chromatographie an Umkehrphasen, oder die Umkristallisation aus wässrigen oder nicht-wässrigen Lösungsmittel-Gemischen verwendet.

Die Verbindung 2-Amino-6-({[2-(4-chlorphenyl)-1,3-oxazol-4-yl]methyl}sulfanyl)-4-(4-{[2,3-dihydroxypropyl]oxy}phenyl)pyridin-3,5-dicarbonitril der Formel (A) wird hergestellt, indem man zunächst den Benzaldehyd der Formel (XVI) 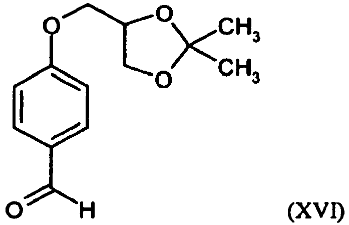 mit zwei Äquivalenten 2-Cyanothioacetamid in Gegenwart einer Base wie *N*-Methylmorpholin zum Pyridin-Derivat (XVII) 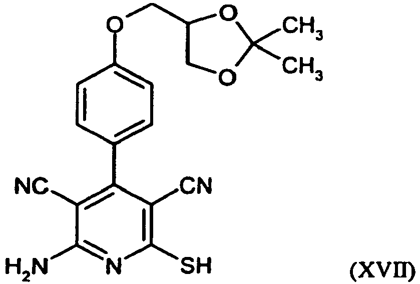 kondensiert, dieses dann in Gegenwart einer Base wie Natriumhydrogencarbonat mit 4-(Chlormethyl)-2-(4-chlorphenyl)-1,3-oxazol der Formel (XVIII) 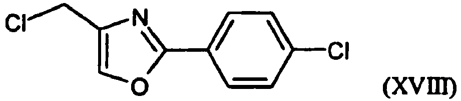 zur Verbindung der Formel (XIX) 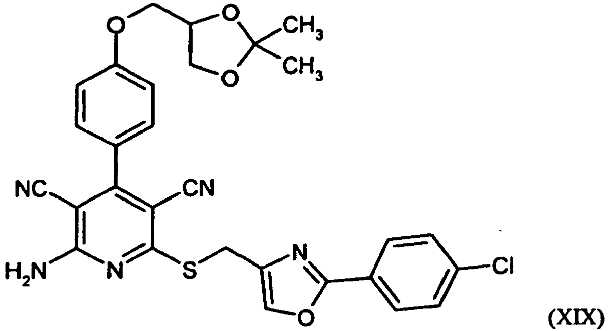 alkyliert und abschließend die Acetonid-Schutzgruppe mit Hilfe einer wässrigen Säure, wie Salzsäure oder Essigsäure, abspaltet [siehe auch das nachfolgende Reaktionsschema 2 sowie die Beschreibung der Intermediate 1A-9A im Experimentellen Teil].

Die Verbindung der Formel (XVI) ihrerseits ist durch Umsetzung von 4-Hydroxybenzaldehyd mit dem 3-Chlor-1,2-propandiol-Acetonid der Formel (XX) 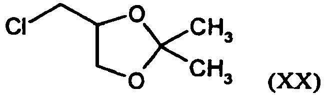 in Gegenwart einer Base wie Kaliumcarbonat zugänglich. Werden hierbei die enantiomerenreinen, R- bzw. S-konfigurierten 3-Chlor-1,2-propandiol-Acetonide eingesetzt, so lassen sich gemäß der oben beschriebenen Reaktionssequenz die entsprechenden Enantiomere der Wirkstoff-Verbindung (A) sowie - aus diesen abgeleitet - die korrespondierenden Prodrug-Verbindungen der Formeln (I-A) und (I-B) erhalten.

Das 2-Phenyl-1,3-oxazol-Derivat der Formel (XVIII) ist über literaturbekannte Kondensationsreaktionen herstellbar [vgl. das nachfolgende Reaktionsschema 3].

Die Herstellung der erfindungsgemäßen Verbindungen (I) und der Wirkstoff-Verbindung (A) kann durch die folgenden Syntheseschemata beispielhaft veranschaulicht werden:

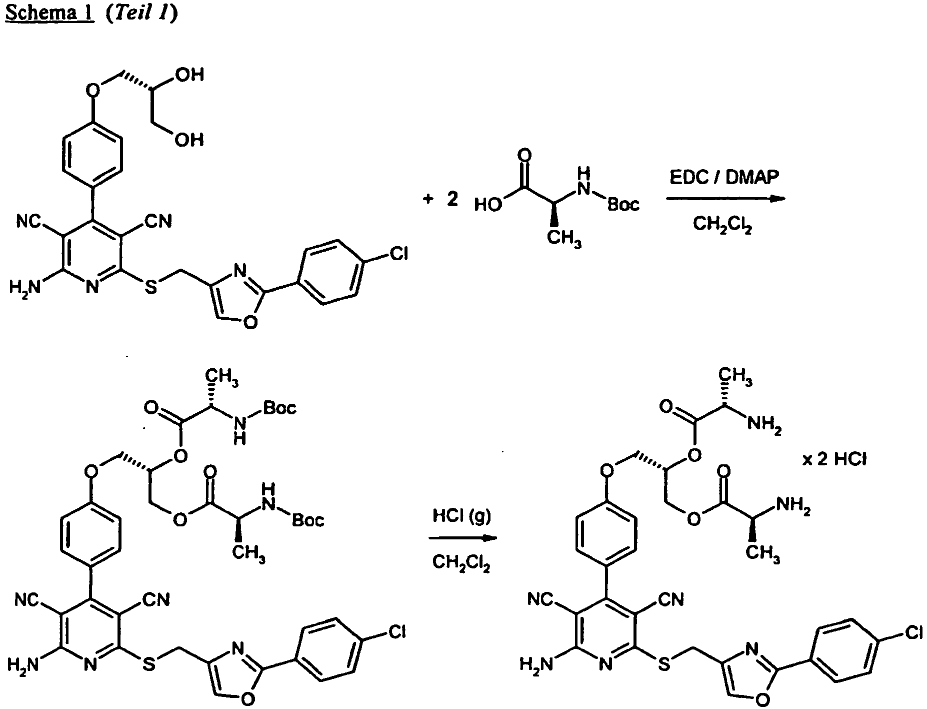

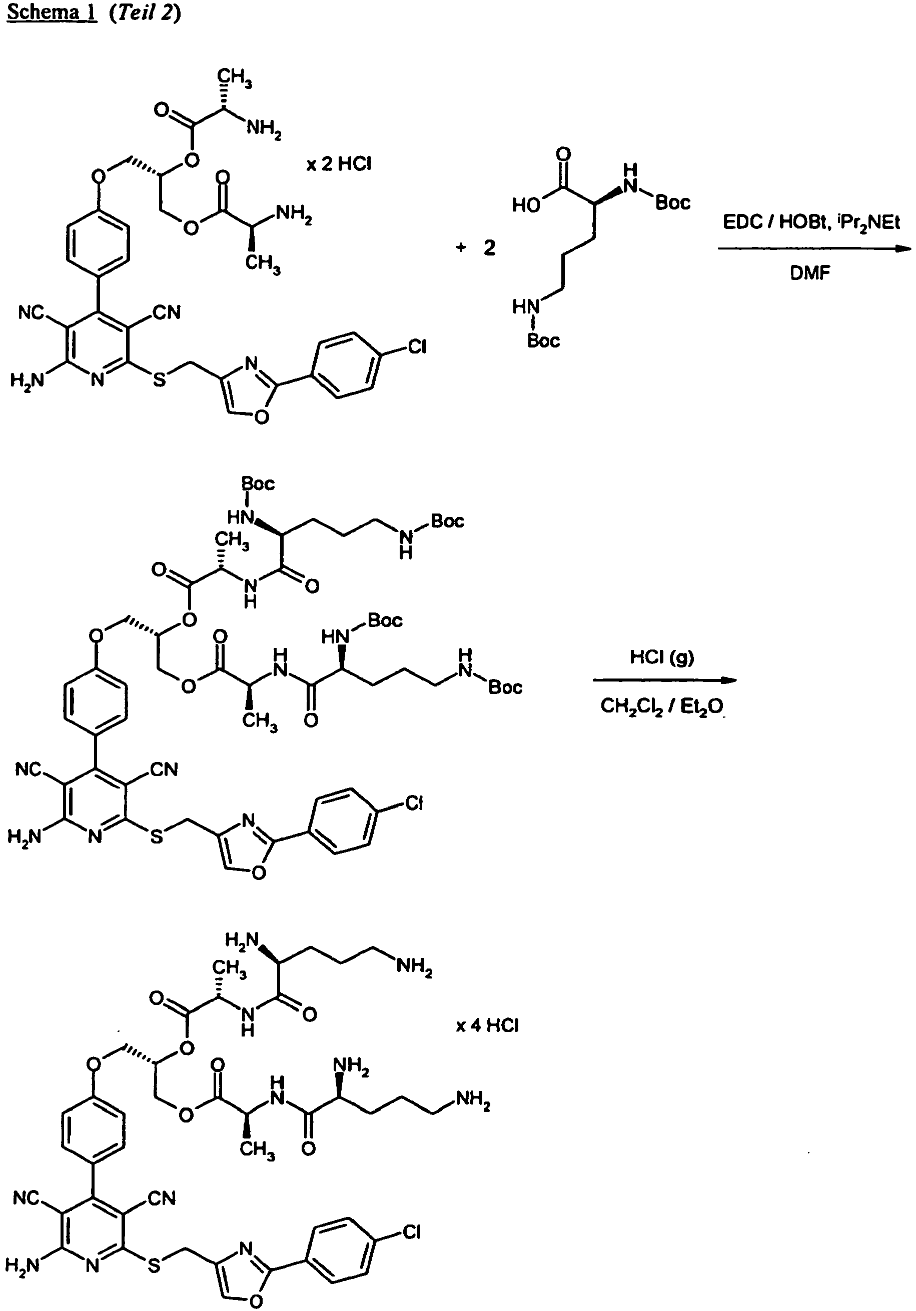

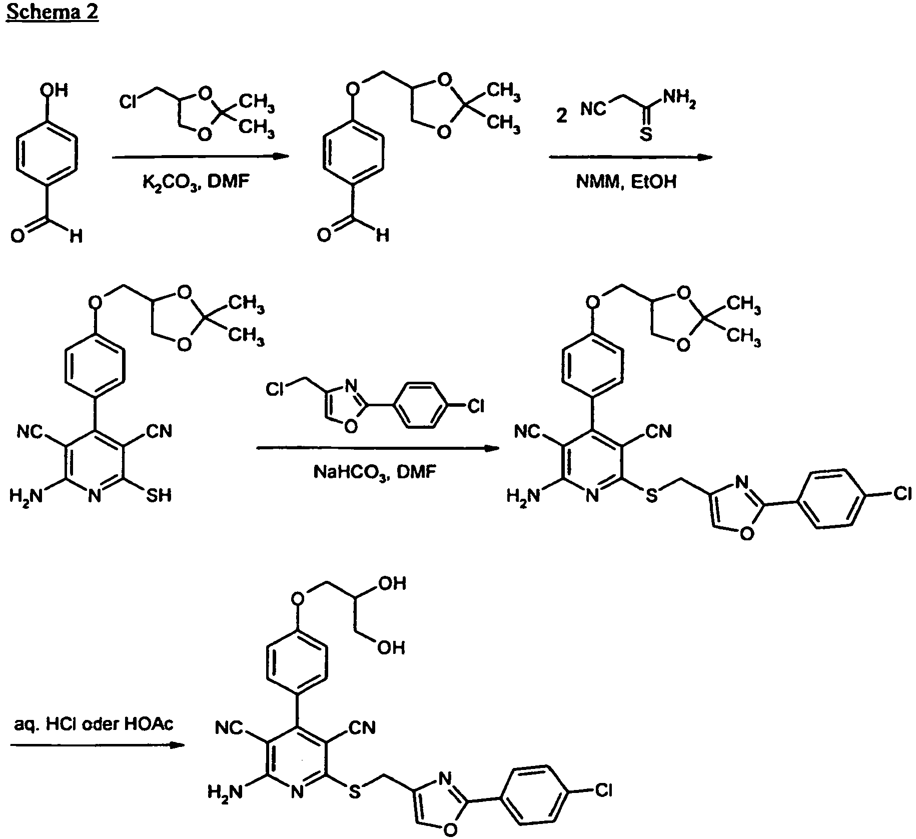

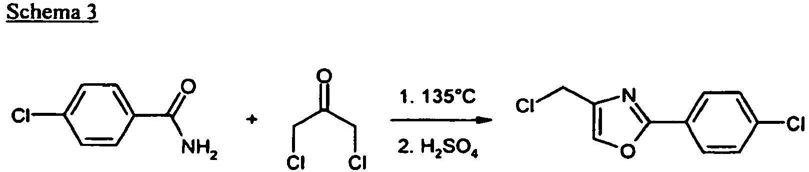

Die erfindungsgemäßen Verbindungen und ihre Salze stellen nützliche Prodrugs der Wirkstoff-Verbindung (A) dar. Sie weisen einerseits eine gute Stabilität bei verschiedenen pH-Werten auf und zeigen andererseits eine effiziente Konversion zur Wirksubstanz (A) bei einem physiologischen pH-Wert und insbesondere *in vivo.* Darüber hinaus besitzen die erfindungsgemäßen Verbindungen verbesserte Löslichkeiten in wässrigen oder anderen physiologisch verträglichen Medien, was sie für die therapeutische Anwendung insbesondere bei intravenöser Applikation geeignet macht. Außerdem ist die Bioverfügbarkeit aus Suspension nach oraler Applikation gegenüber der Muttersubstanz (A) verbessert.

Die Verbindungen der Formel (I) sind allein oder in Kombination mit einem oder mehreren anderen Wirkstoffen zur Prophylaxe und/oder Behandlung verschiedener Erkrankungen geeignet, so beispielsweise insbesondere von Erkrankungen des Herzkreislauf-Systems (kardiovaskulären Erkrankungen), zur Kardioprotektion nach Schädigungen des Herzens sowie von Stoffwechsel-Erkrankungen.

Im Sinne der vorliegenden Erfindung sind unter Erkrankungen des Herzkreislauf-Systems bzw. kardiovaskulären Erkrankungen beispielsweise die folgenden Erkrankungen zu verstehen: Hypertonie (Bluthochdruck), periphere und kardiale Gefäßerkrankungen, koronare Herzerkrankung, koronare Restenose wie z.B. Restenose nach Ballondilatation von peripheren Blutgefäßen, Myokardinfarkt, akutes Koronarsyndrom, akutes Koronarsyndrom mit ST-Erhebung, akutes Koronarsyndrom ohne ST-Erhebung, stabile und instabile Angina pectoris, Herzmuskelschwäche, Prinzmetal-Angina, persistierende ischämische Dysfunktion ("hibernating myocardium"), vorübergehende postischämische Dysfunktion ("stunned myocardium"), Herzinsuffizienz, Tachykardien, atriale Tachykardie, Arrhythmien, Vorhof- und Kammerflimmern, persistierendes Vorhofflimmern, permanentes Vorhofflimmern, Vorhofflimmern mit normaler linksventrikulärer Funktion, Vorhofflimmern mit eingeschränkter linksventrikulärer Funktion, Wolff-Parkinson-White-Syndrom, periphere Durchblutungsstörungen, erhöhte Spiegel von Fibrinogen und von LDL geringer Dichte sowie erhöhte Konzentrationen von Plasminogenaktivator-Inhibitor 1 (PAI-1), insbesondere Hypertonie, koronare Herzerkrankung, akutes Koronarsyndrom, Angina pectoris, Herzinsuffizienz, Myokardinfarkt und Vorhofflimmern.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akute dekompensierte Herzinsuffizienz, Rechtsherzinsuffizienz, Linksherzinsuffizienz, Globalinsuffzienz, ischämische Kardiomyopathie, dilatative Kardiomyopathie, angeborene Herzfehler, Henklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Weiterhin eignen sich die erfindungsgemäßen Verbindungen insbesondere auch zur Reduktion des von einem Infarkt betroffenen Myokardbereichs sowie zur Prophylaxe von Sekundärinfarkten.

Des weiteren sind die erfindungsgemäßen Verbindungen insbesondere zur Prophylaxe und/oder Behandlung von thromboembolischen Erkrankungen, Reperfusionsschäden nach Ischämie, mikro- und makrovaskulären Schädigungen (Vaskulitis), arteriellen und venösen Thrombosen, Ödemen, Ischämien wie Myokardinfarkt, Hirnschlag und transitorischen ischämischen Attacken, zur Kardioprotektion bei Koronararterien-Bypass-Operationen (CABG), primären perkutan-transluminalen Koronarangioplastien (PTCAs), PTCAs nach Thrombolyse, Rescue-PTCA, Herztransplantationen und Operationen am offenen Herzen, sowie zur Organprotektion bei Transplantationen, Bypass-Operationen, Katheteruntersuchungen und anderen operativen Eingriffen geeignet.

Weitere Indikationsgebiete, für die die erfindungsgemäßen Verbindungen verwendet werden können, sind beispielsweise die Prophylaxe und/oder Behandlung von Erkrankungen des Urogenitalbereiches, wie z.B. akutes Nierenversagen, Reizblase, urogenitale Inkontinenz, erektile Dysfunktion und weibliche sexuelle Dysfunktion, daneben aber auch die Prophylaxe und/oder Behandlung von inflammatorischen Erkrankungen, wie z.B. entzündliche Dermatosen und Arthritis, insbesondere rheumatische Arthritis, von Erkrankungen des Zentralen Nervensystems und neurodegenerativen Störungen (Zustände nach Schlaganfall, Alzheimer'sche Krankheit, Parkinson'sche Krankheit, Demenz, Chorea Huntington, Epilepsie, Depressionen, Multiple Sklerose), von Schmerzzuständen und Migräne, Leberfibrose und Leberzirrhose, von Krebserkrankungen und von Übelkeit und Erbrechen in Verbindung mit Krebstherapien, sowie zur Wundheilung.

Ein weiteres Indikationsgebiet ist beispielsweise die Prophylaxe und/oder Behandlung von Erkrankungen der Atemwege wie beispielsweise Asthma, chronisch-obstruktive Atemwegserkrankungen (COPD, chronische Bronchitis), Lungenemphysem, Bronchiektasien, zystische Fibrose (Mukoviszidose) und pulmonale Hypertonie, insbesondere pulmonale arterielle Hypertonie.

Schließlich kommen die erfindungsgemäßen Verbindungen auch für die Prophylaxe und/oder Behandlung von Stoffwechsel-Erkrankungen in Betracht, wie beispielsweise Diabetes, insbesondere Diabetes mellitus, Gestationsdiabetes, Insulin-abhängiger Diabetes und Nicht-Insulin-abhängiger Diabetes, diabetische Folgeerkrankungen wie z.B. Retinopathie, Nephropathie und Neuropathie, metabolische Erkrankungen wie z.B. Metabolisches Syndrom, Hyperglykämie, Hyperinsulinämie, Insulinresistenz, Glukose-Intoleranz und Fettsucht (Adipositas), sowie Arteriosklerose und Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), insbesondere von Diabetes, Metabolischem Syndrom und Dyslipidämien.

Beschrieben wird die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Beschrieben wird die Verwendung der erfindungsgemäßen Verbindungen in einem Verfahren zur Behandlung und/oder Prophylaxe von Erkrankungen, insbesondere der zuvor genannten Erkrankungen.

Beschrieben wird auch ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen.

Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/ oder antithrombotisch wirkende Mittel, Antiarrhythmika, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten) sowie Analgetika wie beispielsweise Aspirin.

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff, einem Antiarrhythmikum und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylhamstoffe, Biguanide, Meglitinid-Derivate, Glukosidase-Inhibitoren, Inhibitoren der Dipeptidyl-Peptidase IV (DPP-IV-Inhibitoren), Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin AII-Antagonisten, ACE-Hemmer, Renin-Inhibitoren, beta-Adrenozeptor-Antagonisten, alpha-Adrenozeptor-Antagonisten, Diuretika, Aldosteron-Antagonisten, Mineralocorticoid-Rezeptor-Antagonisten, ECE-Inhibitoren sowie der Vasopeptidase-Inhibitoren;
- antithrombotisch wirkenden Mitteln, beispielhaft und vorzugsweise aus der Gruppe der Thrombozytenaggregationshemmer oder der Antikoagulantien;
- Antiarrhythmika, insbesondere solchen zur Behandlung von supraventrikulären Arrhythmien und Tachykardien;
- Substanzen zur Prophylaxe und Behandlung von Ischämie- und Reperfusionsschäden;
- Vasopressin-Rezeptor-Antagonisten;
- organischen Nitraten und NO-Donatoren;
- positiv-inotrop wirksamen Verbindungen;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Agonisten des Prostacyclin-Rezeptors (IP-Rezeptors), wie beispielsweise Iloprost, Beraprost und Cicaprost;
- Calcium-Sensitizem, wie beispielhaft und vorzugsweise Levosimendan;
- Kalium-Supplements;
- NO- und Häm-unabhängigen Aktivatoren der Guanylatcyclase, wie insbesondere den in WO 01/19355, WO 01/19776, WO 01/19778, WO 01/19780, WO 02/070462 und WO 02/070510 beschriebenen Verbindungen;
- NO-unabhängigen, jedoch Häm-abhängigen Stimulatoren der Guanylatcyclase, wie insbesondere den in WO 00/06568, WO 00/06569, WO 02/42301 und WO 03/095451 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren, insbesondere Sorafenib, Imatinib, Gefitinib und Erlotinib;
- den Energiestoffwechsel des Herzens beeinflussenden Verbindungen, wie beispielweise Etomoxir, Dichloracetat, Ranolazine und Trimetazidine;
- Schmerzmitteln; und/oder
- Substanzen zur Prophylaxe und Behandlung von Übelkeit und Erbrechen
kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantien/Radikalfänger sowie der Cannabinoid-Rezeptor 1-Antagonisten verstanden.

Bei einer bevorzugten Ausführungsform der Erfindung werden die erfindungsgemäßen Yerbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Torcetrapib, JTT-705, BAY 60-5521, BAY 78-7499 oder CETP vaccine (Avant), verabreicht.

Bei einer bevorzugten Ausrührungsform der Erfindung werden die erfindungsgemäßen Verbindungen in Kombination mit einem PPAR-γ-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylhamstoffe, Biguanide, Meglitinid-Derivate, Glukosidase-Inhibitoren, DPP-IV-Inhibitoren und PPAR-γ-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem DPP-IV-Inhibitor, wie beispielhaft und vorzugsweise Sitagliptin oder Vildagliptin, verabreicht.

Bei einer bevorzugten Ausführungsform der Erfindung werden die erfindungsgemäßen Verbindungen in Kombination mit einem PPAR-γ-Agonisten beispielsweise aus der Klasse der Thiazolidindione, wie beispielhaft und vorzugsweise Pioglitazone oder Rosiglitazone, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Renin-Inhibitoren, beta-Adrenozeptor-Antagonisten, alpha-Adrenozeptor-Antagonisten und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Valsartan, Candesartan, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindurigen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem beta-Adrenozeptor-Antagonisten, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Adrenozeptor-Antagonisten, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem Aldosteron- oder Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Vasopressin-Rezeptor-Antagonisten, wie beispielhaft und vorzugsweise Conivaptan, Tolvaptan, Lixivaptan oder SR-121463, verabreicht.

Bei einer bevorzugten Ausführungsform der Erfindung werden die erfindungsgemäßen Verbindungen in Kombination mit einem organischen Nitrat oder NO-Donator, wie beispielhaft und vorzugsweise Natriumnitroprussid, Glycerinnitrat, Isosorbidmononitrat, Isosorbiddinitrat, Molsidomin oder SIN-1, oder in Kombination mit inhalativem NO verabreicht.

Bei einer bevorzugten Ausführungsform der Erfindung werden die erfindungsgemäßen Verbindungen in Kombination mit einer positiv-inotrop wirksamen Verbindung, wie beispielhaft und vorzugsweise Herzglycosiden (Digoxin) sowie beta-adrenergen und dopaminergen Agonisten, wie Isoproterenol, Adrenalin, Noradrenalin, Dopamin oder Dobutamin, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, oder in Kombination mit Kaliumkanal-Agonisten wie Minoxidil, Diazoxid, Dihydralazin oder Hydralazin verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter Antiarrhythmika werden vorzugsweise Substanzen aus der Gruppe der Klasse la-Anti-arrhythmika (z.B. Chinidin), der Klasse Ic-Antiarrhythmika (z.B. Flecainid, Propafenon), der Klasse II-Antiarrhythmika (z.B. Metoprolol, Atenolol, Sotalol, Oxprenolol und andere beta-Rezeptoren-Blocker), der Klasse III-Antiarrhythmika (z.B. Sotalol, Amiodaron) und der Klasse IV-Antiarrhythmika (z.B. Digoxin sowie Verapamil, Diltiazem und andere Calcium-Antagonisten) verstanden.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Adrenozeptor-Antagonisten, alpha-Adrenozeptor-Antagonisten, organische Nitrate und NO-Donatoren, Calcium-Antagonisten, ACE-Inhibitoren, Angiotensin AII-Antagonisten, Aldosteron- und Mineralocorticoid-Rezeptor-Antagonisten, Vasopressin-Rezeptor-Antagonisten, Thrombozytenaggregationshemmer, Antikoagulantien und Antiarrhythmika, sowie deren Verwendung zur Behandlung und/oder Prophylaxe der zuvor genannten Erkrankungen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen, enthalten sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent. Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecyl-sulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- Ac: Acetyl
- aq.: wässrig, wässrige Lösung
- Boc: *tert*.-Butoxycarbonyl
- DMAP: 4-*N,N*-Dimethylaminopyridin
- DMF: *N,N*-Dimethylformamid
- DMSO: Dimethylsulfoxid
- d. Th.: der Theorie (bei Ausbeute)
- EDC: 1-(3-Dimethylaminopropyl)-3-ethylcarbodiimid-Hydrochlorid
- ESI: Elektrospray-Ionisation (bei MS)
- Et: Ethyl
- ges.: gesättigt
- h: Stunde(n)
- HOAc: Essigsäure
- HOBt: 1-Hydroxy-1*H*-benzotriazol-Hydrat
- HPLC: Hochdruck-, Hochleistungsflüssigchromatographie
- ⁱPr: Isopropyl
- konz.: konzentriert
- LC-MS: Flüssigchromatographie-gekoppelte Massenspektrometrie
- min: Minute(n)
- MS: Massenspektrometrie
- NMM: *N*-Methylmorpholin
- NMR: Kernresonanzspektrometrie
- *p*: para
- quant.: quantitativ (bei Ausbeute)
- RT: Raumtemperatur
- Rₜ: Retentionszeit (bei HPLC)
- *tert.*: tertiär
- TFA: Trifluoressigsäure
- THF: Tetrahydrofuran
- UV: Ultraviolett-Spektrometrie
- v/v: Volumen-zu-Volumen-Verhältnis (einer Lösung)
- Z: Benzyloxycarbonyl

### LC-MS-Methoden:

### Methode 1:

Gerätetyp MS: Micromass ZQ; Gerätetyp HPLC: Waters Alliance 2795; Säule: Merck Chromolith SpeedROD RP-18e 50 mm x 4.6 mm; Eluent A: 1 Wasser + 0.5 ml 50%-ige Ameisensäure, Eluent B: 1 1 Acetonitril + 0.5 ml 50%-ige Ameisensäure; Gradient: 0.0 min 10% B → 3.0 min 95% B → 4.0 min 95% B; Fluss: 0.0 min 1.0 ml/min → 3.0 min 3.0 ml/min → 4.0 min 3.0 ml/min; Ofen: 35°C; UV-Detektion: 210 nm.

### Methode 2:

Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 3:

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 4:

Instrument: Micromass Quattro LCZ mit HPLC Agilent Serie 1100; UV DAD; Säule: Phenomenex Gemini 3µ 30 mm x 3.00 mm; Eluent A: 1 1 Wasser + 0.5 ml 50%-ige Ameisensäure, Eluent B: 1 I Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 5:

Instrument: Micromass Quattro Premier mit Waters UPLC Acquity; Säule: Thermo Hypersil GOLD 1.9 µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen:50°C; UV-Detektion: 210 nm.

### Methode 6:

Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 11 Wasser + 0.5 ml 50%-ige Ameisensäure, Eluent B: 1 I Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 7:

Gerätetyp MS: Micromass ZQ; Gerätetyp HPLC: HP 1100 Series; UV DAD; Säule: Phenomenex Gemini 3µ 30 mm x 3.00 mm; Eluent A: 1 I Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 8:

Instrument: Waters Acquity SQD UPLC System; Säule: Waters Acquity UPLC HSS T3 1.8µ 50 mm x 1 mm; Eluent A: 11 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 9:

Gerätetyp MS: M-40 DCI (NH₃); Gerätetyp HPLC: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 10:

Instrument: Micromass Quattro Micro MS mit HPLC Agilent Serie 1100; Säule: Thermo Hypersil GOLD 3µ 20 mm x 4 mm; Eluent A: 1 I Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 11:

Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Ausgangsverbindungen und Intermediate:

### Beispiel 1A

### 4-{[(4S)-2,2-Dimethyl-1,3-dioxolan-4-yl]methoxy}benzaldehyd

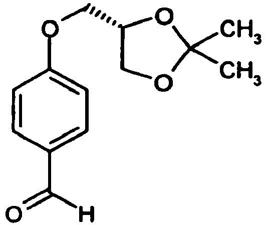

12.5 g (102.4 mmol) 4-Hydroxybenzaldehyd wurden unter Argon in 166 ml trockenem DMF vorgelegt und bei RT mit 42.4 g (307.1 mmol) Kaliumcarbonat sowie 20.05 g (133.1 mmol) (*R*)-(-)-3-Chlor-1,2-propandiol-Acetonid versetzt. Es wurde 16 h bei 160°C gerührt. Der Ansatz wurde dann mit Wasser versetzt und zweimal mit Ethylacetat extrahiert. Die vereinigten organischen Phasen wurden mit ges. wässriger Natriumchlorid-Lösung gewaschen und über Magnesiumsulfat getrocknet. Nach Filtration wurde das Lösungsmittel am Rotationsverdampfer entfernt und der Rückstand mittels Säulenchromatographie an Kieselgel gereinigt (Laufmittel: Cyclohexan/Ethylacetat 10:2).
Ausbeute: 20.0 g (82% d. Th.)
¹H-NMR (400 MHz, DMSO-d₆): 8 = 9.89 (s, 1H), 7.85 (d, 2H), 7.03 (d, 2H), 4.50 (q, 1H), 4.22-4.09 (m, 2H), 4.04 (dd, 1H), 3.92 (dd, 1H), 1.48 (s, 3H), 1.41 (s, 3H).
LC-MS (Methode 9): Rₜ = 4.02 min; MS (ESIpos): m/z = 254 [M+NH₄]⁺.

### Beispiel 2A

### 4-{[(4R)-2,2-Dimethyl-1,3-dioxolan-4-yl]methoxy} benzaldehyd

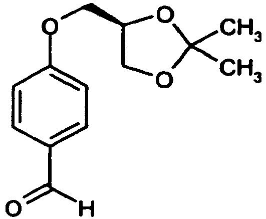

31.2 g (255.4 mmol) 4-Hydroxybenzaldehyd wurden in 400 ml trockenem DMF vorgelegt und bei RT mit 105.7 g (766.1 mmol) Kaliumcarbonat sowie 50.0 g (332.0 mmol) (*S*)-(-)-3-Chlor-1,2-propandiol-Acetonid versetzt. Es wurde 16 h bei 160°C gerührt. Der Ansatz wurde dann mit 4000 ml Wasser versetzt und dreimal mit je 500 ml Ethylacetat extrahiert. Die vereinigten organischen Phasen wurden jeweils einmal mit 500 ml Wasser und 500 ml ges. wässriger Natriumchlorid-Lösung gewaschen. Nach Trocknen über Magnesiumsulfat wurde das Lösungsmittel am Rotationsverdampfer entfernt und der Rückstand mittels Säulenchromatographie an Kieselgel 60 aufgereinigt (Laufmittel-Gradient: Ethylacetat/Petrolether 1:9 → 2:8).
Ausbeute: 40.4 g (63% d. Th.)
¹H-NMR (400 MHz, DMSO-d₆): δ = 9.90 (s, 1H), 7.85 (d, 2H), 7.03 (d, 2H), 4.50 (q, 1H), 4.22-4.09 (m, 2H), 4.04 (dd, 1H), 3.92 (dd, 1H), 1.48 (s, 3H), 1.41 (s, 3H).
LC-MS (Methode 9): Rₜ = 3.97 min; MS (ESIpos): m/z = 254 [M+NH₄]⁺.

### Beispiel 3A

### 2-Amino-4-(4-{[(4S)-2,2-dimethyl-1,3-dioxolan-4-yl]methoxy}phenyl)-6-mercaptopyridin-3,5-dicarbonitril

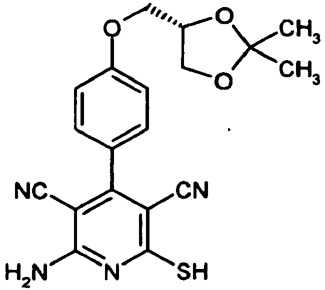

44.0 g (186.2 mmol) der Verbindung aus Beispiel 1A und 37.3 g (372.5 mmol) Cyanothioacetamid wurden in 800 ml Ethanol vorgelegt. Das Reaktionsgemisch wurde bei Raumtemperatur mit 37.6 g (372.5 mmol) 4-Methylmorpholin versetzt und 3 h unter Rühren zum Rückfluss erhitzt. Nach Abkühlen auf RT wurde weitere 16 h bei dieser Temperatur nachgerührt. Der ausgefallene Niederschlag wurde abgesaugt, mit Ethanol gewaschen und im Vakuum getrocknet. Das Produkt wurde ohne weitere Reinigung in der Folgereaktion eingesetzt.
Ausbeute: 22.8 g (32% d. Th.)
¹H-NMR (400 MHz, DMSO-d₆): δ = 7.69-7.37 (br. s, 2H), 7.42 (d, 2H), 7.10 (d, 2H), 4.48-4.39 (m, 1H), 4.15-4.02 (m, 2H), 3.78 (dd, 1H), 3.66 (dd, 1H), 2.77-2.68 (br. s, 1H), 1.37 (s, 3H), 1.31 (s, 3H).
LC-MS (Methode 1): Rₜ = 1.75 min; MS (ESIpos): m/z = 383 [M+H]⁺.

### Beispiel 4A

### 2-Amino-4-(4-{[(4R)-2,2-dimethyl-1,3-dioxolan-4-yl]methoxy}phenyl)-6-mercaptopyridin-3,5-dicarbonitril

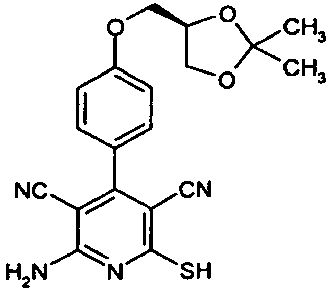

40.4 g (171.0 mmol) der Verbindung aus Beispiel 2A und 34.2 g (342.0 mmol) Cyanothioacetamid wurden in 700 ml Ethanol vorgelegt. Das Reaktionsgemisch wurde mit 34.5 g (342.0 mmol) 4-Methylmorpholin versetzt und 3 h unter Rühren zum Rückfluss erhitzt. Nach Abkühlen auf RT wurde weitere 16 h bei dieser Temperatur nachgerührt. Der ausgefallene Niederschlag wurde abgesaugt, mit ca. 100 ml Ethanol gewaschen und im Trockenschrank getrocknet. Das Produkt wurde ohne weitere Reinigung in der Folgereaktion eingesetzt.
Ausbeute: 19.5 g (29% d. Th.)
¹H-NMR (400 MHz, DMSO-d₆): 8 = 7.63-7.31 (br. s, 2H), 7.41 (d, 2H), 7.09 (d, 2H), 4.49-4.38 (m, 1H), 4.15-3.99 (m, 2H), 3.78 (dd, 1 H), 3.66 (dd, 1H), 2.77-2.68 (br. s, 1H), 1.37 (s, 3H), 1.32 (s, 3H).
LC-MS (Methode 11): Rₜ = 1.95 min; MS (ESIpos): m/z = 424 [M+H+CH₃CN]⁺.

### Beispiel 5A

### 4-(Chlormethyl)-2-(4-chlorphenyl)-1,3-oxazol

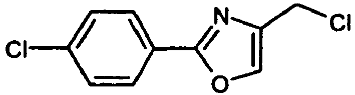

123.8 g (795.5 mmol) 4-Chlorbenzolcarboxamid und 101.0 g (795.5 mmol) 1,3-Dichloraceton wurden eine Stunde bei 135°C gerührt. Es bildete sich eine Schmelze. Der Ansatz wurde danach unter Rühren auf RT abgekühlt, bei dieser Temperatur vorsichtig mit 200 ml konz. Schwefelsäure versetzt und 30 min gerührt. Die erhaltene Suspension wurde auf Eiswasser gegossen und weitere 30 min gerührt. Der entstandene Niederschlag wurde dann abgesaugt, mit Wasser gewaschen und mittels Flashchromatographie an Kieselgel gereinigt (Laufmittel: Dichlormethan). Das Lösungsmittel wurde am Rotationsverdampfer entfernt und der Rückstand im Vakuum getrocknet. Es wurden 95.5 g (53% d. Th.) der Zielverbindung erhalten.
¹H-NMR (400 MHz, DMSO-d₆): δ = 8.30 (s, 1H), 7.99 (d, 2H), 7.62 (d, 2H), 4.75 (s, 2H).
LC-MS (Methode 2): Rₜ = 3.78 min; MS (ESIpos): m/z = 228 [M+H]⁺.

### Beispiel 6A

### 2-Amino-6-({[2-(4-chlorphenyl)-1,3-oxazol-4-yl]methyl}sulfanyl)-4-(4-{[(4S)-2,2-dimethyl-1,3-dioxolan-4-yl]methoxy}phenyl)pyridin-3,5-dicarbonitril

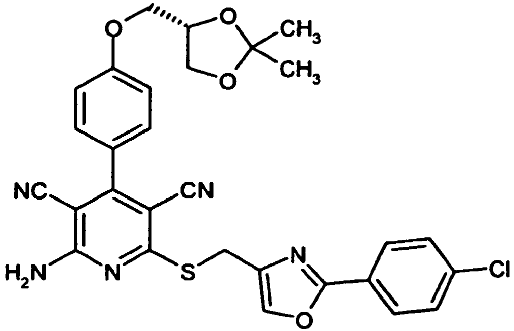

150 mg (0.39 mmol) der Verbindung aus Beispiel 3A und 98 mg (0.43 mmol) der Verbindung aus Beispiel 5A wurden zusammen mit 99 mg (1.18 mmol) Natriumhydrogencarbonat in 2 ml trockenem DMF suspendiert. Das Reaktionsgemisch wurde 20 h bei RT gerührt. Der Ansatz wurde danach direkt mittels präparativer HPLC aufgereinigt (Säule: YMC GEL ODS-AQ S-5 / 15 µm; Laufmittel-Gradient: Acetonitril/Wasser 10:90 → 95:5).
Ausbeute: 147 mg (65% d. Th.)
¹H-NMR (400 MHz, DMSO-d₆): δ = 8.37 (s, 1H), 8.29-7.91 (br. s, 2H), 7.97 (d, 2H), 7.61 (d, 2H), 7.47 (d, 2H), 7.12 (d, 2H), 4.48-4.39 (m, 1H), 4.42 (s, 2H), 4.16-4.03 (m, 3H), 3.77 (dd, 1H), 1.37 (s, 3H), 1.31 (s, 3H).
LC-MS (Methode 3): Rₜ = 4.23 min; MS (ESIpos): m/z = 574 [M+H]⁺.

### Beispiel 7A

### 2-Amino-6-({[2-(4-chlorphenyl)-1,3-oxazol-4-yl]methyl}sulfanyl)-4-(4-{[(4R)-2,2-dimethyl-1,3-dioxolan-4-yl]methoxy}phenyl)pyridin-3,5-dicarbonitril

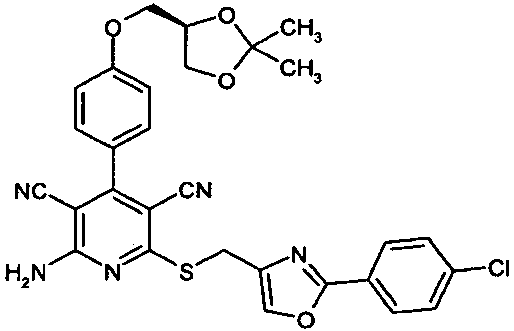

70 mg (0.18 mmol) der Verbindung aus Beispiel 4A und 46 mg (0.20 mmol) der Verbindung aus Beispiel 5A wurden zusammen mit 46 mg (0.55 mmol) Natriumhydrogencarbonat in 1.9 ml trockenem DMF suspendiert. Das Reaktionsgemisch wurde 20 h bei RT gerührt. Der Ansatz wurde danach am Rotationsverdampfer vom Lösungsmittel befreit und der Rückstand mittels präparativer HPLC gereinigt (Säule: YMC GEL ODS-AQ S-5 / 15 µm; Laufmittel-Gradient: Acetonitril/Wasser 10:90 → 95:5).
Ausbeute: 79 mg (75% d. Th.)
¹H-NMR (400 MHz, DMSO-d₆): 8 = 8.37 (s, 1H), 8.30-8.01 (br. s, 2H), 7.97 (d, 2H), 7.60 (d, 2H), 7.48 (d, 2H), 7.12 (d, 2H), 4.48-4.40 (m, 1H), 4.42 (s, 2H), 4.16-4.03 (m, 3H), 3.78 (dd, 1H), 1.37 (s, 3H), 1.31 (s, 3H).
LC-MS (Methode 7): Rₜ = 2.99 min; MS (ESIpos): m/z = 574 [M+H]⁺.

### Beispiel 8A

### 2-Amino-6-({[2-(4-chlorphenyl)-1,3-oxazol-4-yl]methyl}sulfanyl)-4-(4-{[(2R)-2,3-dihydroxypropyl]oxy}phenyl)pyridin-3,5-dicarbonitril

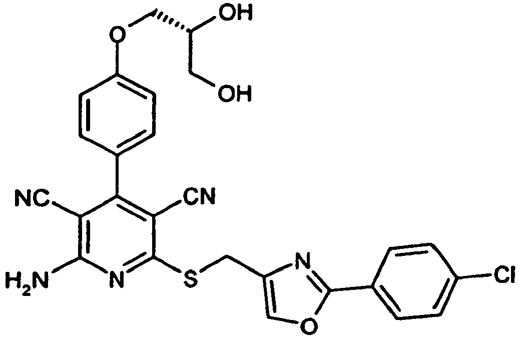

127.1 g (221.4 mmol) der Verbindung aus Beispiel 6A wurden in 800 ml Ethanol suspendiert und mit 800 ml 37%-iger Salzsäure versetzt. Das Gemisch wurde über Nacht unter Rückfluss gerührt. Nach dem Abkühlen auf Raumtemperatur wurde der entstandene Niederschlag abgesaugt, mit Ethanol gewaschen und bei 50°C im Vakuum über Nacht getrocknet. Es wurden 108.3 g (92% d. Th.) der Zielverbindung erhalten.
¹H-NMR (400 MHz, DMSO-d₆): δ = 8.37 (s, 1H), 8.30-7.89 (br. s, 2H), 7.98 (d, 2H), 7.61 (d, 2H), 7.48 (d, 2H), 7.10 (d, 2H), 5.00 (d, 1 H), 4.70 (t, 1H), 4.42 (s, 2H), 4.09 (dd, 1H), 3.98-3.92 (m, 1H), 3.81 (q, 1H), 3.50-3.43 (m, 2H).
LC-MS (Methode 4): Rₜ = 2.51 min; MS (ESIpos): m/z = 534 [M+H]⁺.

### Beispiel 9A

### 2-Amino-6-({[2-(4-chlorphenyl)-1,3-oxazol-4-yl]methyl}sulfanyl)-4-(4-{[(2S)-2,3-dihydroxypropyl]oxy}phenyl)pyridin-3,5-dicarbonitril

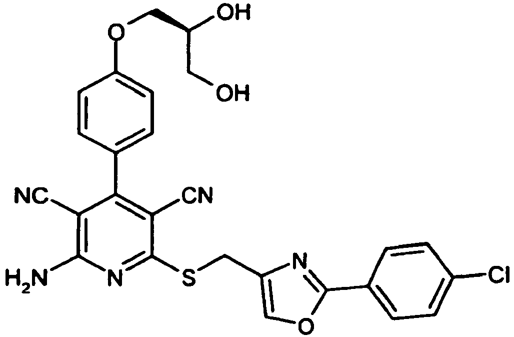

400 mg (0.70 mmol) der Verbindung aus Beispiel 7A wurden in 17 ml Essigsäure vorgelegt und anschließend vorsichtig mit 8.6 ml Wasser versetzt. Es wurde 12 h bei RT gerührt. Nach Einengen des Reaktionsgemisches am Rotationsverdampfer wurde der Rückstand direkt mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhielt man das Produkt als weißen Feststoff.
Ausbeute: 340 mg (91% d. Th.)
¹H-NMR (400 MHz, DMSO-d₆): δ = 8.37 (s, 1H), 8.27-7.91 (br. s, 2H), 7.98 (d, 2H), 7.60 (d, 2H), 7.47 (d, 2H), 7.10 (d, 2H), 5.00 (d, 1H), 4.70 (t, 1 H), 4.42 (s, 2H), 4.09 (dd, 1H), 3.96 (dd, 1H), 3.70 (q, 1H), 3.46 (t, 2H).
LC-MS (Methode 7): Rₜ = 2.48 min; MS (ESIpos): m/z = 534 [M+H]⁺.

### Beispiel 10A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis{2-[(tert.-butoxycarbonyl)amino]propanoat}

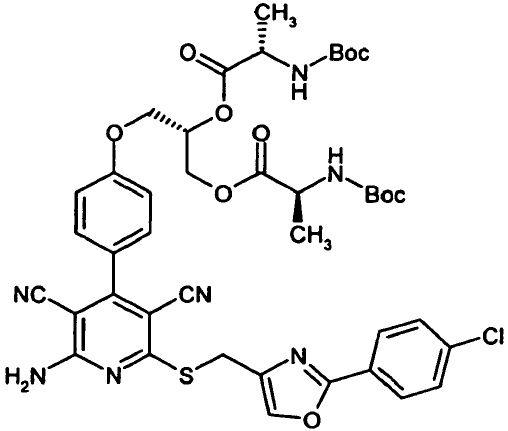

5 g (9.36 mmol) der Verbindung aus Beispiel 8A, 7.09 g (37.45 mmol) *N*-Boc-L-Alanin, 8.975 g (46.82 mmol) 1-(3-Dimethylaminopropyl)-3-ethylcarbodiimid-Hydrochlorid sowie 1.144 g (9.36 mmol) 4-*N*,*N*-Dimethylaminopyridin wurden in 500 ml Dichlormethan zusammengegeben und 30 min lang im Ultraschallbad behandelt. Der Ansatz wurde danach mit 10%-iger Zitronensäure-Lösung und anschließend mit 10%-iger Natriumhydrogencarbonat-Lösung ausgeschüttelt, bis kein *N*-Boc-L-Alanin in der organischen Phase mehr nachweisbar war. Die organische Phase wurde dann über Magnesiumsulfat getrocknet und im Vakuum eingeengt. Der Rückstand wurde in Dichlormethan aufgenommen und mit Diethylether versetzt. Der entstandene Niederschlag wurde abgesaugt. Nach Trocknung des Feststoffs verblieben 6.01 g (73% d. Th.) der Titelverbindung.
¹H-NMR (400 MHz, DMSO-d₆): δ = 8.36 (s, 1H), 8.33-8.02 (br. m, 2H), 7.97 (d, 2H), 7.60 (d, 2H), 7.48 (d, 2H), 7.30 (m, 2H), 7.12 (d, 2H), 5.34 (m, 1H), 4.42 (s, 2H), 4.38-4.21 (m, 4H), 4.03 (m, 2H), 1.36 (s, 18H), 1.26-1.22 (m, 6H).
LC-MS (Methode 5): Rₜ = 1.61 min; MS (ESIpos): m/z = 876 [M+H]⁺.

### Beispiel 11A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-aminopropanoat)-Dihydrochlorid

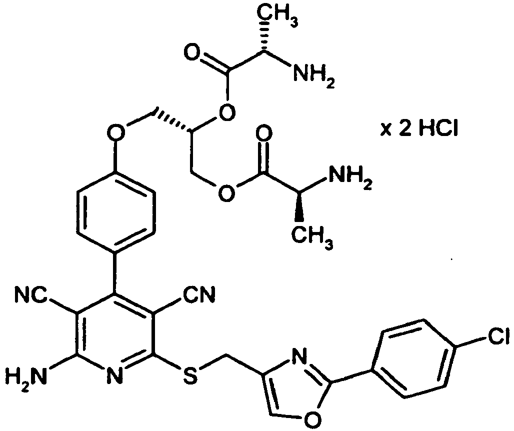

In eine Lösung von 6014 mg (6.862 mmol) der Verbindung aus Beispiel 10A in 500 ml Dichlormethan wurde über 30 min Chlorwasserstoff-Gas eingeleitet, wobei die Temperatur unter +20°C gehalten wurde. Der ausgefallene Feststoff wurde abgesaugt, mit Dichlormethan und mit Diethylether gewaschen und über Nacht im Hochvakuum bei +80°C getrocknet. Es wurden 5080 mg (99% d. Th.) der Zielverbindung erhalten.
¹H-NMR (400 MHz, DMSO-d₆): δ = 8.7 (br. s, 6H), 8.4 (s, 1H), 8.0 (d, 2H), 7.60 (d, 2H), 7.50 (d, 2H), 7.15 (d, 2H), 5.5 (m, 1H), 4.60-4.50 (m, 2H), 4.44 (s, 2H), 4.40 (d, 2H), 4.15 (m, 2H), 1.5-1.4 (m, 6H).
LC-MS (Methode 7): Rₜ = 1.53 min; MS (ESIpos): m/z = 676 [M+H]⁺.

### Beispiel 12A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-aminopropanoat)-Bis(trifluoressigsäure)-Salz

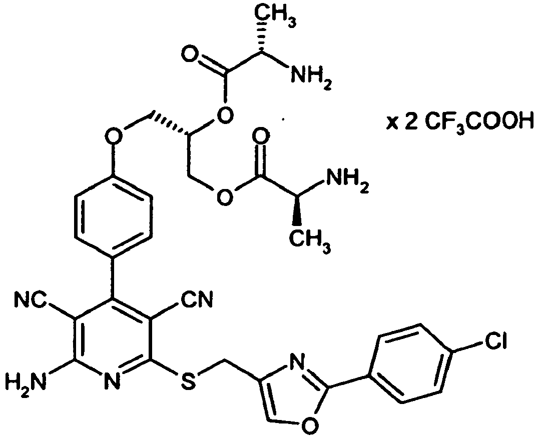

6.520 g (7.440 mmol) der Verbindung aus Beispiel 10A wurden in 45 ml Dichlormethan vorgelegt, mit 5.732 ml (74.396 mmol) Trifluoressigsäure versetzt und über Nacht bei Raumtemperatur gerührt. Anschließend wurde das Reaktionsgemisch eingeengt und der Rückstand zweimal mit Dichlormethan aufgeschlämmt und erneut eingeengt. Der Rückstand wurde dann mit Diethylether verrührt, der verbliebene Feststoff abfiltriert mit Diethylether gewaschen und im Hochvakuum getrocknet. Es wurden 4.8 g (69% d. Th.) der Zielverbindung erhalten.
¹H-NMR (400 MHz, DMSO-d₆): δ = 8.42 (br. s, 4H), 8.36 (s, 1H), 8.19 (m, 2H), 7.97 (d, 2H), 7.60 (d, 2H), 7.50 (d, 2H), 7.13 (d, 2H), 5.52-5.47 (m, 1H), 4.59-4.50 (m, 2H), 4.41 (s, 2H), 4.39-4.31 (m, 2H), 4.19-4.15 (m, 2H), 1.42-1.34 (m, 6H).
LC-MS (Methode 5): Rₜ = 0.94 min; MS (ESIpos): m/z = 676 [M+H]⁺.

### Beispiel 13A

### (6S,9S,12S)-12-({4-[2-Amino-6-({[2-(4-chlorphenyl)-1,3-oxazol-4-yl]methyl}sulfanyl)-3,5-dicyanopyridin-4-yl]phenoxy}methyl)-2,2,9-trimethyl-6-(2-methylpropyl)-4,7,10-trioxo-3,11-dioxa-5,8-diazatridecan-13-yl-(2S)-2-({(2S)-2-[(tert.-butoxycarbonyl)amino]-4-methylpentanoyl}amino)-propanoat

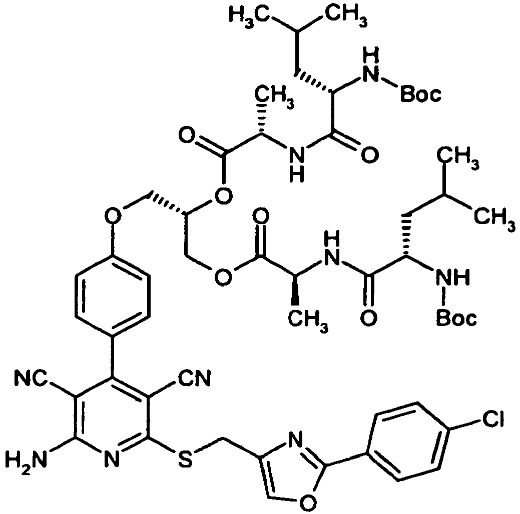

139 mg (0.60 mmol) *N*-(*tert*.-Butoxycarbonyl)-L-leucin wurden gemeinsam mit 81 mg (0.60 mmol) 1-Hydroxy-1*H*-benzotriazol-Hydrat und 80 mg (0.421 mmol) 1-(3-Dimethylaminopropyl)-3-ethyl-carbodiimid-Hydrochlorid für 5 min in 3 ml DMF gerührt. Anschließend wurden 150 mg (0.20 mmol) der Verbindung aus Beispiel 11A sowie 0.174 ml (1.00 mmol) *N*,*N*-Diisopropylethylamin zugegeben und das Reaktionsgemisch über Nacht bei RT gerührt. Danach wurden nochmals die gleichen Mengen an *N*-(*tert*.-Butoxycarbonyl)-L-leucin, 1-Hydroxy-1*H*-benzotriazol-Hydrat, 1-(3-Dimethylaminopropyl)-3-ethylcarbodiimid-Hydrochlorid sowie *N*,*N*-Diisopropylethylamin hinzugefügt und die Mischung weitere 3 h bei RT gerührt. Der Reaktionsansatz wurde dann direkt mittels präparativer HPLC aufgereinigt (Laufmittel-Gradient: Acetonitril/Wasser 10:90 → 95:5). Es wurden 188 mg (85% d. Th.) der Zielverbindung erhalten.
¹H-NMR (400 MHz, DMSO-d₆): δ = 8.36 (s, 1H), 8.24 (d, 2H), 7.99 (d, 2H), 7.60 (d, 2H), 7.48 (d, 2H), 7.12 (d, 2H), 6.80 (t, 2H), 5.33-5.29 (m, 1H), 4.41 (s, 2H), 4.37-4.19 (m, 6H), 4.03-3.97 (m, 2H), 1.64-1.56 (m, 2H), 1.44-1.38 (m, 4H), 1.36 (2s, 18H), 1.30-1.27 (m, 6H), 0.86-0.83 (m, 12H).
LC-MS (Methode 6): Rₜ = 2.88 min; MS (ESIpos): m/z = 1102 [M+H]⁺.

### Beispiel 14A

### (6S,9S,13S)-14-{4-[2-Amino-6-({[2-(4-chlorphenyl)-1,3-oxazol-4-yl]methyl}sulfanyl)-3,5-dicyanopyridin-4-yl]phenoxy}-6-[(2S)-butan-2-yl]-2,2,9-trimethyl-4,7,10-trioxo-3,11-dioxa-5,8-diazatetradecan-13-yl-(2S)-2-({(2S,3S)-2-[(tert.-butoxycarbonyl)amino]-3-methylpentanoyl}-amino)propanoat

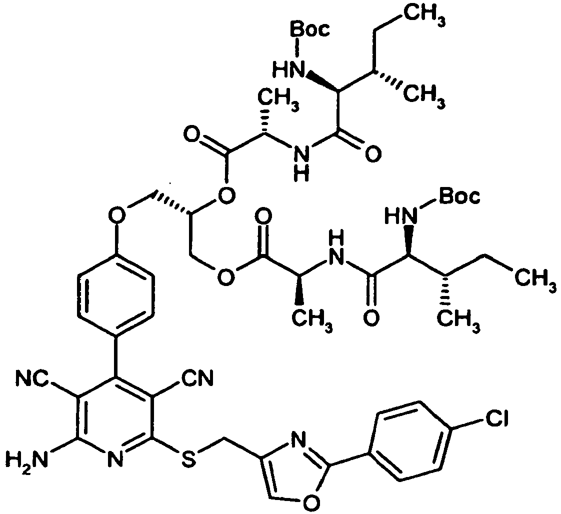

Die Herstellung der Titelverbindung erfolgte analog zur Herstellung von Beispiel 13A ausgehend von der Verbindung aus Beispiel 11A und *N*-(*tert*.-Butoxycarbonyl)-L-isoleucin.
Ausbeute: 82% d. Th.
¹H-NMR (400 MHz, DMSO-d₆): δ = 8.36 (s, 1H), 8.32-8.31 (m, 2H), 7.97 (d, 2H), 7.61 (d, 2H), 7.48 (d, 2H), 7.11 (d, 2H), 6.65-6.61 (m, 2H), 5.42-5.31 (m, 1 H), 4.42 (s, 2H), 4.39-4.19 (m, 6H), 3.85 (m, 2H), 1.67-1.65 (m, 2H), 1.52-1.40 (m, 2H), 1.35 (2s, 18H), 1.29 (t, 6H), 1.12-1.01 (m, 2H), 0.84-0.77 (m, 12H).
LC-MS (Methode 6): Rₜ = 2.87 min; MS (ESIpos): m/z = 1102 [M+H]⁺.

### Beispiel 15A

### (8S,11S,15S)-16-{4-[2-Amino-6-({[2-(4-chlorphenyl)-1,3-oxazol-4-yl]methyl}sulfanyl)-3,5-dicyanopyridin-4-yl]phenoxy}-8-[(tert.-butoxycarbonyl}amino]-2,2,11-trimethyl-4,9,12-trioxo-3,13-dioxa-5,10-diazahexadecan-15-yl-(2S)-2-({(2S)-2,4-bis[(tert.-butoxycarbonyl)amino]butanoyl}-amino)propanoat

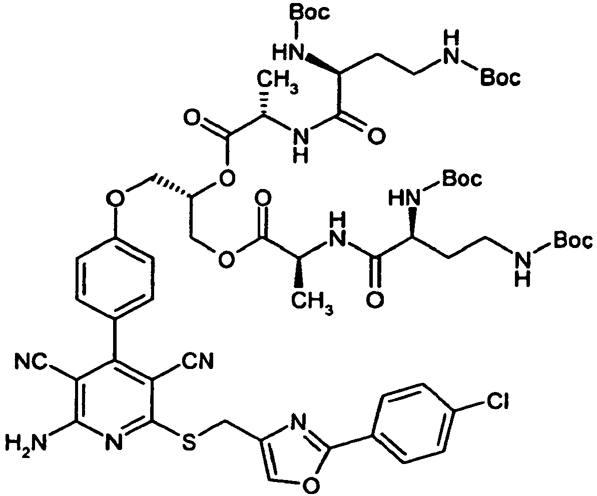

250 mg (0.501 mmol) (2*S*)-2,4-Bis[(*tert*.-butoxycarbonyl)amino]butansäure-Dicyclohexylamin-Salz wurden gemeinsam mit 68 mg (0.501 mmol) 1-Hydroxy-1*H-*benzotriazol-Hydrat und 67 mg (0.350 mmol) 1-(3-Dimethylaminopropyl)-3-ethylcarbodiimid-Hydrochlorid für 5 min in 3 ml DMF gerührt. Anschließend wurden 125 mg (0.200 mmol) der Verbindung aus Beispiel 11A sowie 0.145 ml (0.834 mmol) *N*,*N*-Diisopropylethylamin zugegeben und das Gemisch über Nacht bei RT gerührt. Der Reaktionsansatz wurde danach direkt mittels präparativer HPLC aufgereinigt (Laufmittel-Gradient: Acetonitril/Wasser 10:90 → 95:5). Es wurden 166 mg (76% d. Th.) der Zielverbindung erhalten.
¹H-NMR (400 MHz, DMSO-d₆): δ = 8.36 (s, 1H), 8.25 (m, 2H), 8.12-8.00 (m, 2H), 7.97 (d, 2H), 7.60 (d, 2H), 7.47 (d, 2H), 7.11 (s, 2H), 6.88-6.84 (m, 2H), 6.75-6.64 (m, 2H), 5.37-5.28 (m, 1H), 4.42 (s, 2H), 4.38-4.19 (m, 6H), 3.98-3.93 (m, 2H), 3.06-2.91 (m, 4H), 1.77-1.71 (m, 2H), 1.61-1.51 (m, 2H), 1.35 (s, 36H), 1.31-1.27 (m, 6H).
LC-MS (Methode 7): Rₜ = 3.17 min; MS (ESIpos): m/z = 1276 [M+H]⁺.

### Beispiel 16A

### (9S,12S,16S)-17-{4-[2-Amino-6-({[2-(4-chlorphenyl)-1,3-oxazol-4-yl]methyl}sulfanyl)-3,5-dicyanopyridin-4-yl]phenoxy}-9-[(tert.-butoxycarbonyl)amino]-2,2,12-trimethyl-4,10,13-trioxo-3,14-dioxa-5,11-diazaheptadecan-16-yl-(2S)-2-({(2S)-2,5-bis[(tert.-butoxycarbonyl)amino]-pentanoyl}amino)propanoat

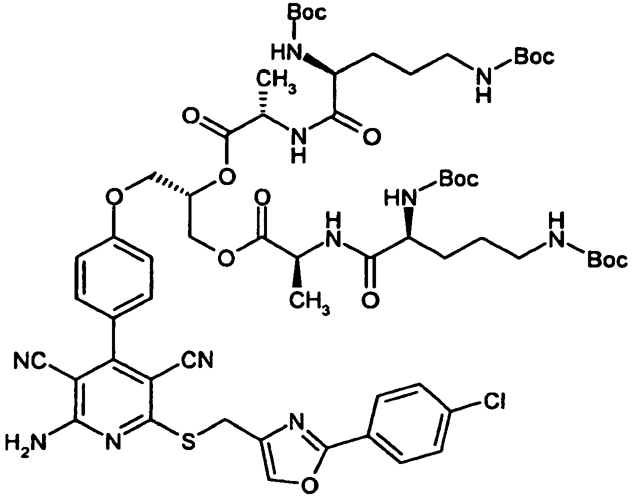

Die Herstellung der Titelverbindung erfolgte analog zur Herstellung von Beispiel 15A ausgehend von der Verbindung aus Beispiel 11A und käuflichem *N*²,*N*⁵-Bis(*tert*.-butoxycarbonyl)-L-ornithin.
Ausbeute: 86% d. Th.
¹H-NMR (400 MHz, DMSO-d₆): δ = 8.36 (s, 1H), 8.21 (br. d, 2H), 8.15-8.01 (m, 2H), 7.97 (d, 2H), 7.60 (d, 2H), 7.48 (d, 2H), 7.12 (d, 2H), 6.82-6.71 (m, 4H), 5.37-5.28 (m, 1H), 4.42 (s, 2H), 4.38-4.19 (m, 6H), 3.96-3.86 (m, 2H), 2.94-2.81 (m, 4H), 1.47-1.23 (m, 50H).
LC-MS (Methode 7): Rₜ = 3.17 min; MS (ESIpos): m/z = 1304 [M+H]⁺.

### Beispiel 17A

### (6S,9S,13S)-14-{4-[2-Amino-6-({[2-(4-chlorphenyl)-1,3-oxazol-4-yl]methyl}sulfanyl)-3,5-di-cyanopyridin-4-yl]phenoxy}-6-(tert.-butoxymethyl}-2,2,9-trimethyl-4,7,10-trioxo-3,11-dioxa-5,8-diazatetradecan-13-yl-(2S)-2-({(2S)-3-tert-butoxy-2-[(tert.-butoxycarbonyl)amino]propanoyl}-amino)propanoat

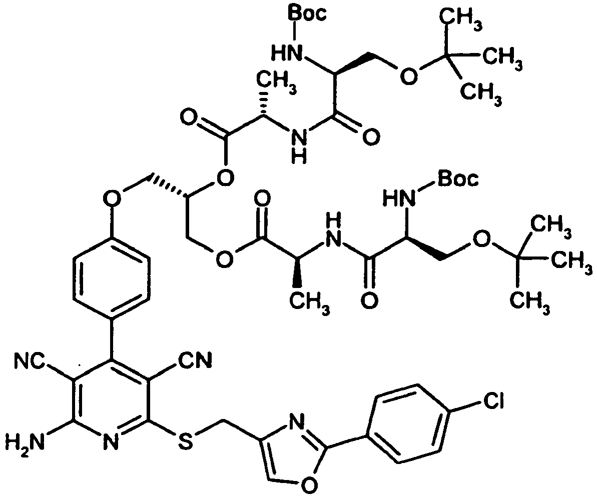

Die Herstellung der Titelverbindung erfolgte analog zur Herstellung von Beispiel 15A ausgehend von der Verbindung aus Beispiel 11A und käuflichem *N*-(*tert*.-Butoxycarbonyl)-O-*tert*.-butyl-L-serin-Dicyclohexylamin-Salz.
Ausbeute: 80% d. Th.
¹H-NMR (400 MHz, DMSO-d₆): δ = 8.66 (s, 1H), 8.28-8.21 (m, 2H), 8.18-8.02 (m, 2H), 7.97 (d, 2H), 7.60 (d, 2H), 7.48 (d, 2H), 7.12-7.10 (d, 2H), 6.64-6.58 (m, 2H), 5.37-5.30 (m, 1H), 4.42 (s, 2H), 4.39-4.18 (m, 6H), 4.08-4.01 (m, 2H), 3.49-3.35 (m, 4H), 1.36 (2s, 18H), 1.29 (t, 6H), 1.09 (2s, 18H).
LC-MS (Methode 7): Rₜ = 3.27 min; MS (ESIpos): m/z = 1162 [M+H]⁺.

### Beispiel 18A

### (6S,9S,13S)-14-{4-[2-Amino-6-({[2-(4-chlorphenyl)-1,3-oxazol-4-yl]methyl}sulfanyl)-3,5-dicyanopyridin-4-yl]phenoxy}-6-benzyl-2,2,9-trimethyl-4,7,10-trioxo-3,11-dioxa-5,8-diazatetradecan-13-yl-(2S)-2-({(2S)-2-[(tert.-butoxycarbonyl)amino]-3-phenylpropanoyl}amino)propanoat

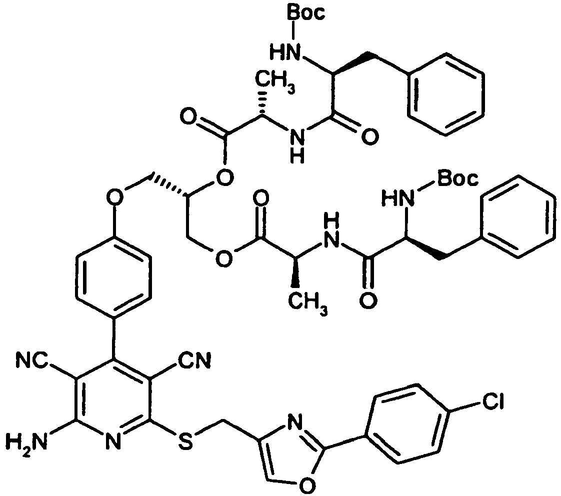

Die Herstellung der Titelverbindung erfolgte analog zur Herstellung von Beispiel 15A ausgehend von der Verbindung aus Beispiel 11A und käuflichem *N-*(*tert*.-Butoxycarbonyl)-L-phenylalanin.
Ausbeute: 83% d. Th.
¹H-NMR (400 MHz, DMSO-d₆): δ = 8.47-8.40 (m, 2H), 8.37 (s, 1H), 8.31-8.01 (m, 2H), 7.97 (d, 2H), 7.60 (d, 2H), 7.44 (d, 2H), 7.33-7.21 (m, 8H), 7.20-7.13 (m, 2H), 7.09 (d, 2H), 6.91-6.87 (m, 2H), 5.39-5.32 (m, 1H), 4.42 (s, 2H), 4.40-4.07 (m, 8H), 3.02-2.90 (m, 2H), 2.74-2.61 (m, 2H), 1.34-1.31 (m, 6H), 1.26 (2s, 14H), 1.22-1.18 (m, 4H).
LC-MS (Methode 7): Rₜ = 3.22 min; MS (ESIpos): m/z = 1170 [M+H]⁺.

### Beispiel 19A

### (9S,12S)-12-({4-[2-Amino-6-({[2-(4-chlorphenyl)-1,3-oxazol-4-yl]methyl}sulfanyl)-3,5-dicyano-pyridin-4-yl]phenoxy}methyl)-2,2,9-trimethyl-4,7,10-trioxo-3,11-dioxa-5,8-diazatridecan-13-yl-(2S)-2-({[(tert.-butoxycarbonyl)amino]acetyl}amino)propanoat

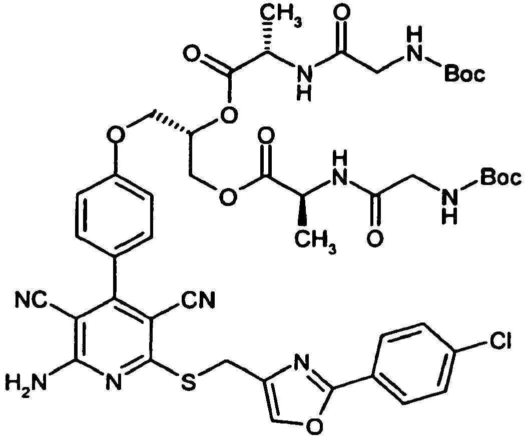

1.535 g (8.765 mmol) *N*-(*tert*.-Butoxycarbonyl)glycin wurden gemeinsam mit 711 mg (5.259 mmol) 1-Hydroxy-1*H*-benzotriazol-Hydrat und 807 mg (4.207 mmol) 1-(3-Dimethylaminopropyl)-3-ethylcarbodiimid-Hydrochlorid für 5 min in 50 ml DMF gerührt. Anschließend wurden 3.17 g (3.506 mmol) der Verbindung aus Beispiel 12A sowie 2.44 ml (14.023 mmol) *N*,*N*-Diisopropylethylamin zugegeben und das Gemisch über Nacht bei RT gerührt. Danach wurden weitere 768 mg (4.382 mmol) *N*-(*tert*.-Butoxycarbonyl)glycin, 1.221 ml (7.012 mmol) *N*,*N*-Diisopropylethylamin, 404 mg (2.104 mmol) 1-(3-Dimethylaminopropyl)-3-ethylcarbodiimid-Hydrochlorid sowie 356 mg (2.630 mmol) 1-Hydroxy-1*H*-benzotriazol-Hydrat hinzugefügt und die Reaktionsmischung erneut für 4 h bei RT gerührt. Der Ansatz wurde danach direkt mittels präparativer HPLC aufgereinigt (Laufmittel-Gradient: Acetonitril/Wasser 10:90 → 95:5). Es wurden 2.37 g (66% d. Th.) der Zielverbindung erhalten.
¹H-NMR (400 MHz, DMSO-d₆): 8 = 8.36 (s, 1H), 8.22 (d, 2H), 8.17-8.01 (m, 2H), 7.97 (d, 2H), 7.60 (d, 2H), 7.48 (d, 2H), 7.13 (d, 2H), 6.93-6.87 (m, 2H), 5.37-5.30 (m, 1H), 4.42 (s, 2H), 4.36-4.21 (m, 6H), 3.64-3.45 (m, 4H), 1.36 (s, 18H), 1.28 (t, 6H).
LC-MS (Methode 6): Rₜ = 2.42 min; MS (ESIpos): m/z = 990 [M+H]⁺.

### Beispiel 20A

### Di-tert-butyl-(2S,2'S)-2,2'-([(2S)-3-{4-[2-amino-6-({[2-(4-chlorphenyl)-1,3-oxazol-4-yl]methyl}-sulfanyl)-3,5-dicyanopyridin-4-yl]phenoxy}propan-1,2-diyl]-bis{oxy[(2S)-1-oxopropan-1,2-diyl]-carbamoyl})dipyrrolidin-1-carboxylat

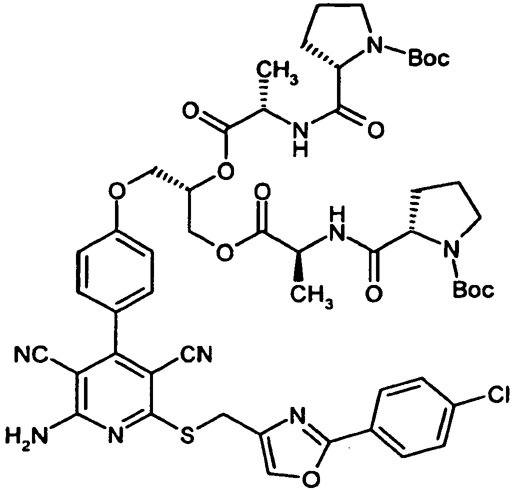

Die Herstellung der Titelverbindung erfolgte analog zur Herstellung von Beispiel 19A ausgehend von der Verbindung aus Beispiel 11A und käuflichem 1-(*tert*.-Butoxycarbonyl)-L-prolin.
Ausbeute: 84% d. Th.
¹H-NMR (400 MHz, DMSO-d₆): δ = 8.36 (s, 1H), 8.34-8.26 (m, 2H), 8.19-8.02 (m, 2H), 7.97 (d, 2H), 7.61 (d, 2H), 7.48 (d, 2H), 7.11 (d, 2H), 5.41-5.31 (m, 1H), 4.42 (s, 2H), 4.39-4.18 (m, 6H), 4.14-4.02 (m, 2H), 3.27-3.19 (m, 2H), 2.12-1.96 (m, 2H), 1.86-1.64 (m, 6H), 1.40-1.28 (m, 24H).
LC-MS (Methode 7): Rₜ = 2.98 min; MS (ESIpos): m/z = 1070 [M+H]⁺.

### Beispiel 21A

### (9S,12S)-12-({4-[2-Amino-6-({[2-(4-chlorphenyl)-1,3-oxazol-4-yl]methyl}sulfanyl)-3,5-dicyano-pyridin-4-yl]phenoxy}methyl)-2,2,5,9-tetramethyl-4,7,10-trioxo-3,11-dioxa-5,8-diazatridecan-13-yl-(2S)-2-({[(tert.-butoxycarbonyl)(methyl)amino]acetyl}amino)propanoat

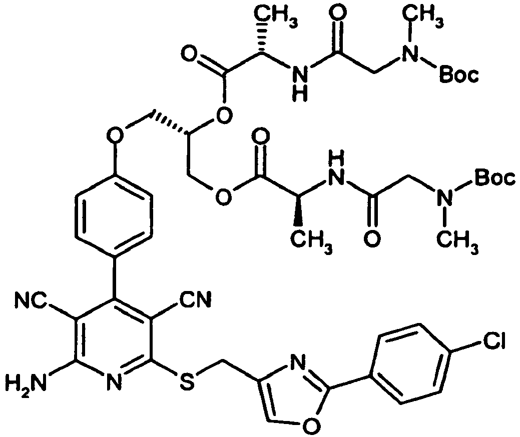

Die Herstellung der Titelverbindung erfolgte analog zur Herstellung von Beispiel 19A ausgehend von der Verbindung aus Beispiel 12A und käuflichem *N*-(*tert*.-Butoxycarbonyl)-*N*-methylglycin.
Ausbeute: 60% d. Th.
¹H-NMR (400 MHz, DMSO-d₆): δ = 8.36 (s, 1H), 8.34-8.29 (m, 2H), 8.21-8.02 (m, 2H), 7.97 (d, 2H), 7.60 (d, 2H), 7.49 (d, 2H), 7.12 (d, 2H), 5.39-5.31 (m, 1H), 4.42 (s, 2H), 4.38-4.19 (m, 6H), 3.91-3.68 (m, 4H), 2.77 (s, 3H), 2.75 (s, 3H), 1.37-1.23 (m, 24H).
LC-MS (Methode 5): Rₜ = 1.52 min; MS (ESIpos): m/z = 1018 [M+H]⁺.

### Beispiel 22A

### Di-tert.-butyl-(2S,6S,9S,13S,17S)-9-({4-[2-amino-6-{{[2-(4-chlorphenyl)-1,3-oxazol-4-yl]methyl}-sulfanyl)-3,5-dicyanopyridin-4-yl]phenoxy}methyl)-2,17-bis[(tert.-butoxycarbonyl)amino]-6,13-dimethyl-4,7,12,15-tetraoxo-8,11-dioxa-5,14-diazaoctadecan-1,18-dioat

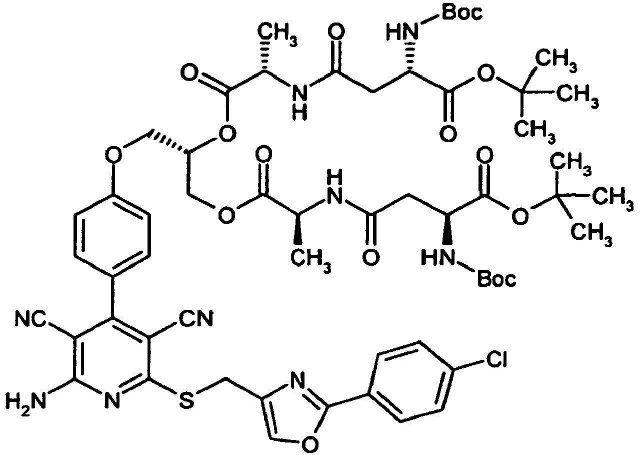

Die Herstellung der Titelverbindung erfolgte analog zur Herstellung von Beispiel 19A ausgehend von der Verbindung aus Beispiel 12A und käuflicher (3*S*)4-*tert*.-Butoxy-3-[(*tert*.-butoxycarbonyl)amino]-4-oxobutan säure.
Ausbeute: 71% d. Th.
¹H-NMR (400 MHz, DMSO-d₆): δ = 8.36 (s, 1H), 8.35 (d, 2H), 8.29-8.02 (m, 2H), 7.97 (d, 2H), 7.60 (d, 2H), 7.48 (d, 2H), 7.13 (d, 2H), 6.91-6.87 (m, 2H), 5.38-5.30 (m, 1H), 4.41 (s, 2H), 4.39-4.07 (m, 8H), 2.52-2.41 (m, 4H), 1.36 (s, 36H), 1.29-1.23 (m, 6H).
LC-MS (Methode 5): Rₜ = 1.69 min; MS (ESIpos): m/z = 1218 [M+H]⁺.

### Beispiel 23A

### (6S,9S,13S)-14-{4-[2-Amino-6-({[2-(4-chlorphenyl)-1,3-oxazol-4-yl]methyl}sulfanyl)-3,5-dicyanopyridin-4-yl]phenoxy}-6-(2-amino-2-oxoethyl)-2,2,9-trimethyl-4,7,10-trioxo-3,11-dioxa-5,8-diazatetradecan-13-yl-(2S)-2-({(2S)-4-amino-2-[(tert.-butoxycarbonyl)amino]-4-oxobutanoyl}-amino)propanoat

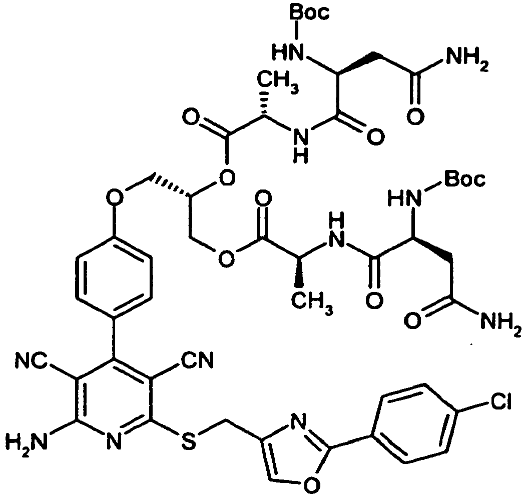

115 mg (0.498 mmol) *N*²-(*tert*.-Butoxycarbonyl)-L-asparagin wurden gemeinsam mit 40 mg (0.299 mmol) 1-Hydroxy-1*H*-benzotriazol-Hydrat und 46 mg (0.239 mmol) 1-(3-Dimethylaminopropyl)-3-ethylcarbodiimid-Hydrochlorid für 5 min in 3 ml DMF gerührt. Anschließend wurden 240 mg (0.199 mmol) der Verbindung aus Beispiel 12A sowie 0.139 ml (0.796 mmol) *N*,*N*-Diisopropylethylamin zugegeben und die Mischung über Nacht bei RT gerührt. Der Reaktionsansatz wurde danach direkt mittels präparativer HPLC aufgereinigt (Laufmittel-Gradient: Acetonitril/Wasser 10:90 → 95:5). Es wurden 66 mg (22% d. Th.) der Zielverbindung erhalten.
¹H-NMR (400 MHz, DMSO-d₆): δ = 8.36 (s, 1H), 8.32-8.16 (m, 4H), 7.97 (d, 2H), 7.60 (d, 2H), 7.48 (d, 2H), 7.25-7.17 (m, 2H), 7.13 (d, 2H), 6.95-6.82 (m, 4H), 5.36-5.29 (m, 1H), 4.42 (s, 2H), 4.38-4.16 (m, 8H), 2.47-2.31 (m, 4H), 1.35 (2s, 18H), 1.28 (t, 6H).
LC-MS (Methode 6): Rₜ = 2.15 min; MS (ESIpos): m/z = 1104 [M+H]⁺.

### Beispiel 24A

### Di-tert.-butyl-(3S,6S,9S,13S,16S)-9-({4-[2-amino-6-({[2-(4-chlorphenyl)-1,3-oxazol-4-yl]methyl}-sulfanyl)-3,5-dicyanopyridin-4-yl]phenoxy}methyl)-3,16-bis[(tert.-butoxycarbonyl)amino]-6,13-dimethyl-4,7,12,15-tetraoxo-8,11-dioxa-5,14-diazaoctadecan-1,18-dioat

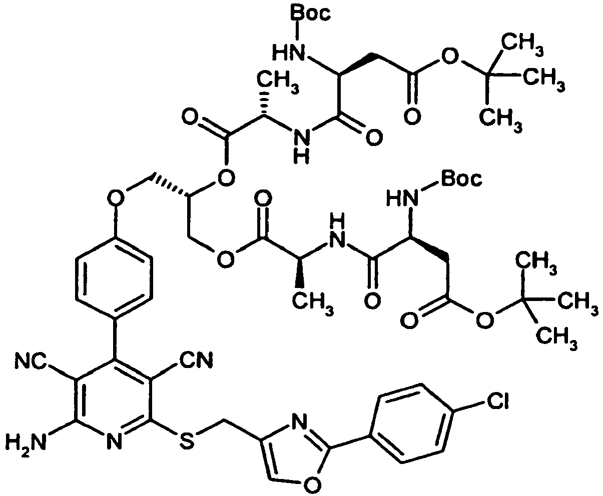

320 mg (1.106 mmol) (2*S*)-4-*tert*.-Butoxy-2-[(*tert*.-butoxycarbonyl)amino]-4-oxobutansäure wurden gemeinsam mit 90 mg (0.664 mmol) 1-Hydroxy-1*H*-benzotriazol-Hydrat und 102 mg (0.531 mmol) 1-(3-Dimethylaminopropyl)-3-ethylcarbodiimid-Hydrochlorid für 5 min in 3 ml DMF gerührt. Anschließend wurden 400 mg (0.442 mmol) der Verbindung aus Beispiel 12A sowie 0.308 ml (1.770 mmol) *N*,*N*-Diisopropylethylamin zugegeben und das Gemisch über Nacht bei RT gerührt. Danach wurden nochmals die gleichen Mengen an (2*S*)-4-*tert*.-Butoxy-2-[(*tert*.-butoxycarbonyl)amino]-4-oxobutansäure, 1-Hydroxy-1*H-*benzotriazol-Hydrat, 1-(3-Dimethylaminopropyl)-3-ethylcarbodiimid-Hydrochlorid sowie *N*,*N*-Diisopropylethylamin hinzugefügt und die Reaktionsmischung weitere 3 h bei RT gerührt. Der Ansatz wurde danach direkt mittels präparativer HPLC aufgereinigt (Laufmittel-Gradient: Acetonitril/Wasser 10:90 → 95:5). Es wurden 418 mg (77% d. Th.) der Zielverbindung erhalten.
¹H-NMR (400 MHz, DMSO-d₆): δ = 8.37 (s, 1H), 8.28-8.24 (m, 2H), 8.20-8.00 (m, 2H), 7.97 (d, 2H), 7.60 (d, 2H), 7.48 (d, 2H), 7.13 (d, 2H), 7.00 (br. d, 2H), 5.34-5.30 (m, 1H), 4.42 (s, 2H), 4.38-4.19 (m, 8H), 2.65-2.57 (m, 2H), 2.44-2.37 (m, 2H), 1.39-1.35 (m, 36H), 1.31-1.26 (m, 6H).
LC-MS (Methode 5): Rₜ = 1.72 min; MS (ESIpos): m/z = 1218 [M+H]⁺.

### Beispiel 25A

### Di-tert.-butyl-(4S,7S,10S,14S,17S)-10-({4-[2-amino-6-({[2-(4-chlorphenyl)-1,3-oxazol-4-yl]-methyl}sulfanyl)-3,5-dicyanopyridin-4-yl]phenoxy}methyl}-4,17-bis[(tert.-butoxycarbonyl)-amino]-7,14-dimethyl-5,8,13,16-tetraoxo-9,12-dioxa-6,15-diazaicosan-1,20-dioat

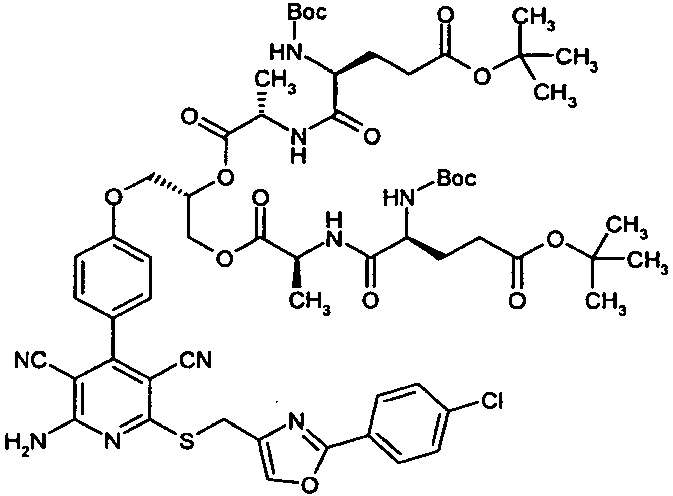

Die Herstellung der Titelverbindung erfolgte analog zur Herstellung von Beispiel 24A ausgehend von der Verbindung aus Beispiel 12A und käuflicher (2*S*)-5-*tert*.-Butoxy-2-[(*tert*.-butoxycarbonyl)amino]-5-oxopentansäure.
Ausbeute: 67% d. Th.
¹H-NMR (400 MHz, DMSO-d₆): δ = 8.37 (s, 1H), 8.30-8.27 (m, 2H), 8.20-8.01 (m, 2H), 7.97 (d, 2H), 7.60 (d, 2H), 7.48 (d, 2H), 7.12 (d, 2H), 6.83 (br. t, 2H), 5.38-5.30 (m, 1H), 4.42 (s, 2H), 4.40-4.21 (m, 6H), 4.00-3.94 (m, 2H), 2.32-2.16 (m, 4H), 1.92-1.79 (m, 2H), 1.74-1.64 (m, 2H), 1.37-1.35 (m, 36H), 1.31-1.28 (m, 6H).
LC-MS (Methode 6): Rₜ = 2.95 min; MS (ESIpos): m/z = 1246 [M+H]⁺.

### Beispiel 26A

### (6S,9S,13S)-14-{4-[2-Amino-6-({[2-(4-chlorphenyl)-1,3-oxazol-4-yl]methyl}sulfanyl)-3,5-dicyanopyridin-4-yl]phenoxy}-6-(1H-imidazol-4-ylmethyl)-2,2,9-trimethyl-4,7,10-trioxo-3,11-dioxa-5,8-diazatetradecan-13-yl-(2S)-2-{[(2S)-2-[(tert.-butoxycarbonyl)amino]-3-(1H-imidazol-4-yl)-propanoyl]amino}propanoat

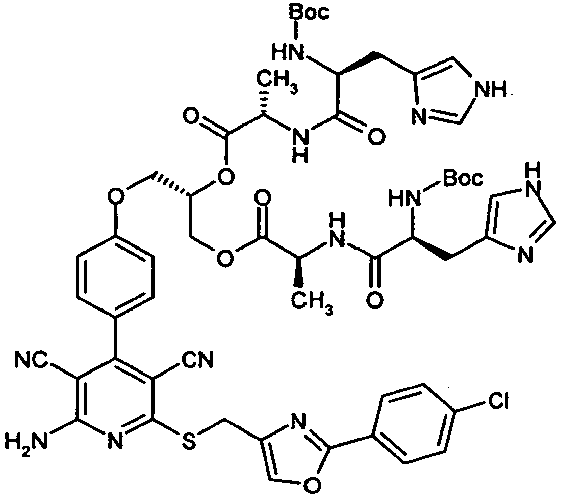

Die Herstellung der Titelverbindung erfolgte analog zur Herstellung von Beispiel 24A ausgehend von der Verbindung aus Beispiel 12A und käuflichem *N*-(*tert*.-Butoxycarbonyl)-L-histidin.
Ausbeute: 52% d. Th.
¹H-NMR (400 MHz, DMSO-d₆): δ = 8.63-8.42 (m, 2H), 8.37 (s, 1H), 7.97 (d, 2H), 7.60 (d, 2H), 7.52 (br. s, 2H), 7.46 (d, 2H), 7.10 (d, 2H), 6.93-6.71 (m, 4H), 4.42 (s, 2H), 5.41-5.26 (m, 1 H), 4.41 (s, 2H), 4.38-4.00 (m, 8H), 2.97-2.67 (m, 4H), 1.37-1.20 (m, 24H).
LC-MS (Methode 6): Rₜ = 1.40 min; MS (ESIpos): m/z = 1150 [M+H]⁺.

### Beispiel 27A

### Di-tert.-butyl-(2S,7S,10S,14S,19S)-10-({4-[2-amino-6-({[2-(4-chlorphenyl)-1,3-oxazol-4-yl]-methyl}sulfanyl)-3,5-dicyanopyridin-4-yl]phenoxy}methyl)-2,19-bis[(tert.-butoxycarbonyl)-amino]-7,14-dimethyl-5,8,13,16-tetraoxo-9,12-dioxa-6,15-diazaicosan-1,20-dioat

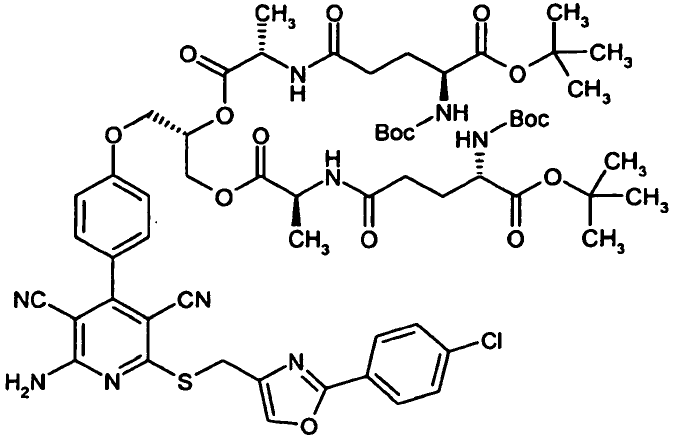

Die Herstellung der Titelverbindung erfolgte analog zur Herstellung von Beispiel 24A ausgehend von der Verbindung aus Beispiel 11A und käuflicher (4*S*)-5-*tert*.-Butoxy-4-[(*tert*.-butoxycarbonyl)amino]-5-oxopentansäure.
Ausbeute: 73% d. Th.
¹H-NMR (400 MHz, DMSO-d₆): δ = 8.36 (s, 1H), 8.25 (d, 2H), 8.20-8.01 (m, 2H), 7.97 (d, 2H), 7.60 (d, 2H), 7.48 (d, 2H), 7.14-7.09 (m, 4H), 5.35-5.30 (m, 1H), 4.42 (s, 2H), 4.34 (d, 2H), 4.29-4.20 (m, 4H), 3.80-3.67 (m, 2H), 2.22-2.15 (m, 4H), 1.94-1.82 (m, 2H), 1.76-1.59 (m, 2H), 1.41-1.32 (m, 36H), 1.28-1.22 (m, 6H).
LC-MS (Methode 5): Rₜ = 1.69 min; MS (ESIpos): m/z = 1246 [M+H]⁺.

### Beispiel 28A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-bis{3-[(tert.-butoxycarbonyl)amino]propanoat}

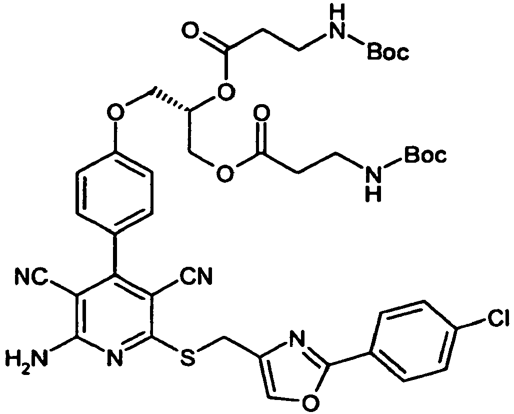

200 mg (0.375 mmol) der Verbindung aus Beispiel 8A wurden in 10 ml Dichlormethan/DMF (1:1) vorgelegt, mit 213 mg (1.124 mmol) *N*-(*tert*.-Butoxycarbonyl)-β-alanin, 215 mg (1.124 mmol) 1-(3-Dimethylaminopropyl)-3-ethylcarbodümid-Hydrochlorid sowie 4.6 mg (0.037 mmol) 4-*N,N-*Dimethylaminopyridin versetzt und über Nacht bei Raumtemperatur gerührt. Die Reaktionsmischung wurde danach direkt mittels präparativer HPLC aufgereinigt (Acetonitril/Wasser-Gradient 10:90 → 95:5). Es wurden 126 mg (38% d. Th.) der Zielverbindung erhalten.
LC-MS (Methode 6): Rₜ = 2.67 min; MS (ESIpos): m/z = 876 [M+H]⁺.

### Beispiel 29A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-bis(3-aminopropanoat)-Dihydrochlorid

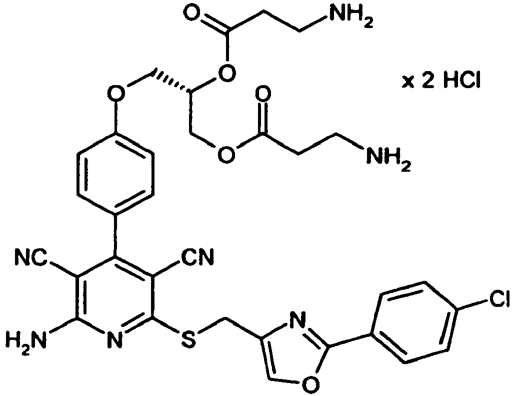

123 mg (0.140 mmol) der Verbindung aus Beispiel 28A wurden in 2 ml Dichlormethan vorgelegt und mit 1.4 ml (2.807 mmol) einer 2 M Lösung von Chlorwasserstoff-Gas in Diethylether versetzt. Nach 1 h Rühren wurde der ausgefallene Feststoff abfiltriert, mit Dichlormethan und Diethylether gewaschen und im Vakuum getrocknet. Man erhielt 79 mg (75% d. Th.) der Zielverbindung.
¹H-NMR (400 MHz, DMSO-d₆): δ = 8.38 (s, 1H), 8.34-8.12 (br. s, 2H), 8.03 (br. m, 6H), 7.97 (d, 2H), 7.61 (d, 2H), 7.49 (d, 2H), 7.14 (d, 2H), 5.40 (m, 1H), 4.42 (s, 2H), 4.41-4.29 (m, 4H), 3.04 (br. m, 4H), 2.75 (br. m, 4H).
LC-MS (Methode 6): Rₜ = 1.17 min; MS (ESIpos): m/z = 676 [M+H]⁺.

### Beispiel 30A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-bis(3-aminopropanoat)-Bis(trifluoressigsäure)-Salz

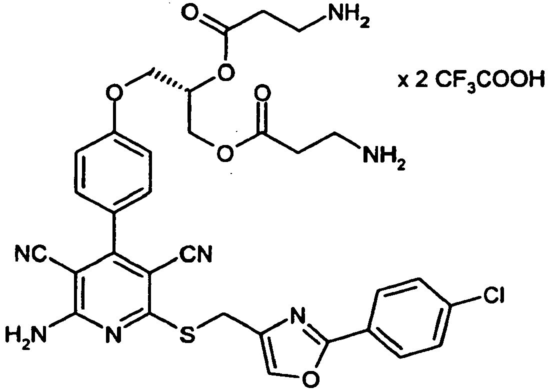

860 mg (0.736 mmol) der Verbindung aus Beispiel 28A wurden in 3 ml Dichlormethan vorgelegt und mit 0.57 ml (7.360 mmol) Trifluoressigsäure versetzt. Nach 2 h Rühren bei RT wurde das Dichlormethan im Vakuum abgezogen und der Rückstand mittels präparativer HPLC gereinigt (Laufmittel-Gradient: Acetonitril/Wasser 10:90 → 95:5 unter Zusatz von 0.1% TFA). Es wurden 532 mg (80% d. Th.) der Zielverbindung erhalten.
LC-MS (Methode 6): Rₜ = 1.09 min; MS (ESIpos): m/z = 676 [M+H]⁺.

### Beispiel 31A

### (10S,18S)-19-{4-[2-Amino-6-({[2-(4-chlorphenyl)-1,3-oxazol-4-yl]methyl}sulfanyl)-3,5-dicyano-pyridin-4-yl]phenoxy}-10-[(tert.-butoxycarbonyl)amino]-2,2-dimethyl-4,11,15-trioxo-3,16-dioxa-5,12-diazanonadecan-18-yl-3-({(2S)-2,6-bis[(tert.-butoxycarbonyl)amino]hexanoyl}amino)-propanoat

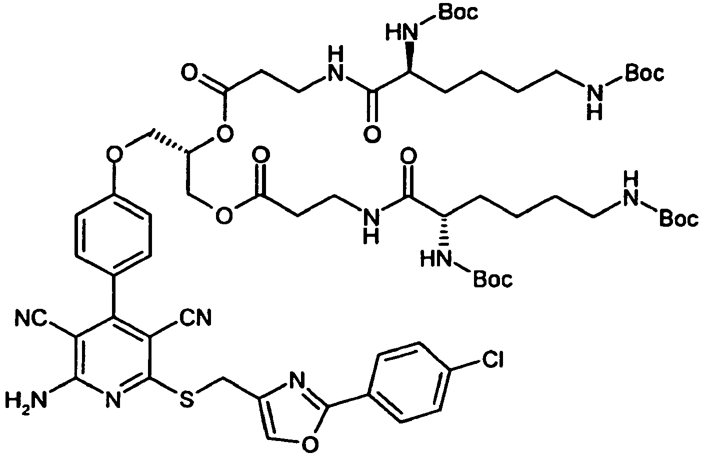

192 mg (0.553 mmol) *N²*,*N⁶*-Bis(*tert*.-butoxycarbonyl)-L-lysin wurden in 2 ml DMF vorgelegt, mit 51 mg (0.265 mmol) 1-(3-Dimethylaminopropyl)-3-ethylcarbodiimid-Hydrochlorid sowie 45 mg (0.332 mmol) 1-Hydroxy-1*H*-benzotriazol-Hydrat versetzt und 10 min gerührt. Anschließend wurden 200 mg (0.221 mmol) der Verbindung aus Beispiel 30A sowie 0.154 ml (0.885 mmol) *N*,*N*-Diisopropylethylamin zugegeben und die Mischung über Nacht bei RT gerührt. Der Reaktionsansatz wurde danach direkt mittels präparativer HPLC aufgereinigt (Laufmittel-Gradient: Acetonitril/Wasser 10:90 → 95:5). Es wurden 77 mg (26% d. Th.) der Zielverbindung erhalten.
LC-MS (Methode 4): Rₜ = 3.15 min; MS (ESIpos): m/z = 1332 [M+H]⁺.

### Beispiel 32A

### (14S)-15-{4-[2-Amino-6-({[2-(4-chlorphenyl)-1,3-oxazol-4-yl]methyl}sulfanyl)-3,5-dicyano-pyridin-4-yl]phenoxy}-2,2-dimethyl-4,7,11-trioxo-3,12-dioxa-5,8-diazapentadecan-14-yl-3-({[(tert.-butoxycarbonyl)amino]acetyl}amino)propanoat

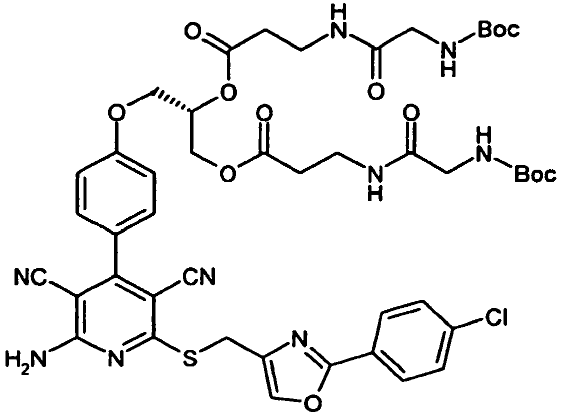

Die Herstellung der Titelverbindung erfolgte analog zur Herstellung von Beispiel 31A ausgehend von der Verbindung aus Beispiel 30A und käuflichem *N*-(*tert.*-Butoxycarbonyl)glycin.
Ausbeute: 34% d. Th.
LC-MS (Methode 4): Rₜ = 2.73 min; MS (ESIpos): m/z = 990 [M+H]⁺.

### Beispiel 33A

### (6S,14S)-15-{4-[2-Amino-6-({[2-(4-chlorphenyl)-1,3-oxazol-4-yl]methyl}sulfanyl)-3,5-dicyano-pyridin-4-yl]phenoxy}-2,2-dimethyl-6-(2-methylpropyl)-4,7,11-trioxo-3,12-dioxa-5,8-diazapenta-decan-14-yl-3-({(2S)-2-[(tert.-butoxycarbonyl)amino]-4-methylpentanoyl}amino)propanoat

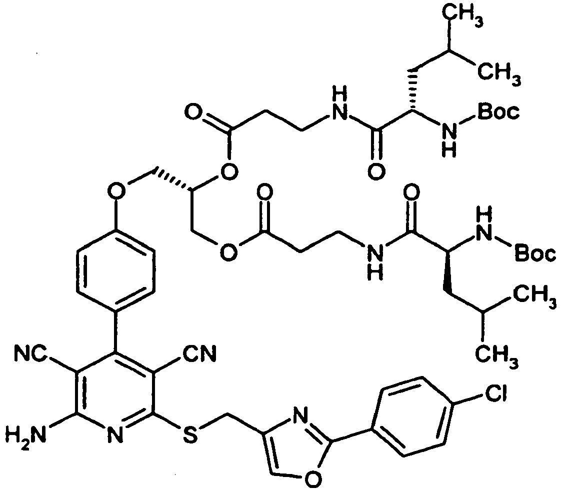

Die Herstellung der Titelverbindung erfolgte analog zur Herstellung von Beispiel 31A ausgehend von der Verbindung aus Beispiel 30A und käuflichem *N*-*tert*.-Butoxycarbonyl)-L-leucin.
Ausbeute: 34% d. Th.
LC-MS (Methode 5): Rₜ = 1.64 min; MS (ESIpos): m/z = 1102 [M+H]⁺.

### Beispiel 34A

### (6S,14S)-15-{4-[2-Amino-6-({[2-(4-chlorphenyl)-1,3-oxazol-4-yl]methyl}sulfanyl)-3,5-dicyano-pyridin-4-yl]phenoxy}-2,2-dimethyl-4,7,11-trioxo-6-(propan-2-yl)-3,12-dioxa-5,8-diazapenta-decan-14-yl-3-({(2S)-2-[(tert.-butoxycarbonyl)amino]-3-methylbutanoyl}amino)propanoat

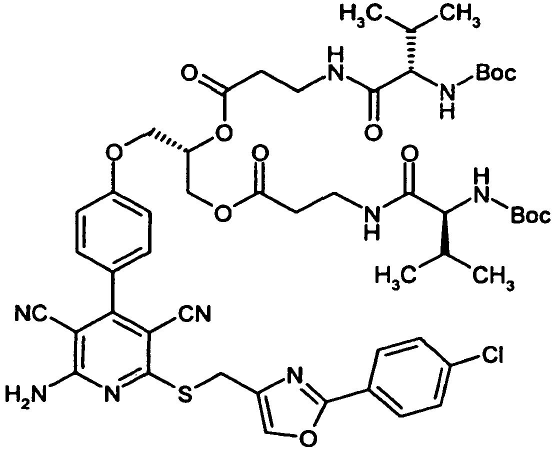

Die Herstellung der Titelverbindung erfolgte analog zur Herstellung von Beispiel 31A ausgehend von der Verbindung aus Beispiel 30A und käuflichem N-(tert.-Butoxycarbonyl)-L-valin.
Ausbeute: 31% d. Th.
LC-MS (Methode 5): Rₜ = 1.57 min; MS (ESIpos): m/z = 1074 [M+H]⁺.

### Beispiel 35A

### (2S)-3-{4-[2-Amino-6-({[2-(4-chlorphenyl}-1,3-oxazol-4-yl]methyl}sulfanyl)-3,5-dicyanopyridin-4-yl]phenoxy}propan-1,2-diyl-(2S,2'S)-bis{2-[(tert.-butoxycarbonyl)amino]-4-methylpentanoat}

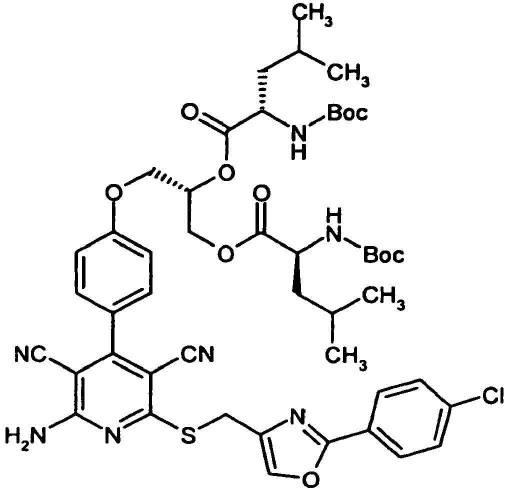

250 mg (0.468 mmol) der Verbindung aus Beispiel 8A wurden in 10 ml Dichlormethan vorgelegt, mit 271 mg (1.170 mmol) *N*-(*tert*.-Butoxycarbonyl)-L-leucin, 269 mg (1.405 mmol) 1-(3-Dimethylaminopropyl)-3-ethylcarbodiimid-Hydrochlorid sowie 5.7 mg (0.047 mmol) 4-*N*,*N*-Dimethylaminopyridin versetzt und über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5). Es wurden 327 mg (73% d. Th.) der Zielverbindung erhalten.
LC-MS (Methode 10): Rₜ = 3.29 min; MS (ESIpos): m/z = 960 [M+H]⁺.

### Beispiel 36A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-amino-4-methylpentanoat)-Bis(trifluoressigsäure)-Salz

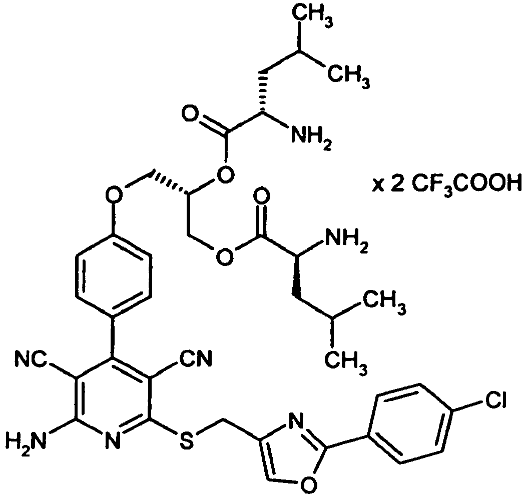

327 mg (0.340 mmol) der Verbindung aus Beispiel 35A wurden in 1 ml Dichlormethan vorgelegt, mit 0.262 ml (3.404 mmol) Trifluoressigsäure versetzt und 3 h bei RT gerührt. Das Reaktionsgemisch wurde danach im Vakuum eingeengt und der Rückstand mittels präparativer HPLC gereinigt (Laufmittel-Gradient: Acetonitril/Wasser 10:90 → 95:5 unter Zusatz von 0.1% TFA). Es wurden 217 mg (64% d. Th.) der Zielverbindung erhalten.
LC-MS (Methode 10): Rₜ = 1.81 min; MS (ESIpos): m/z = 760 [M+H]⁺.

### Beispiel 37A

### (10S,14S)-15-{4-[2-Amino-6-({[2-(4-chlorphenyl)-1,3-oxazol-4-yl]methyl}sulfanyl)-3,5-dicyano-pyridin-4-yl]phenoxy}-2,2-dimethyl-10-(2-methylpropyl)-4,8,11-trioxo-3,12-dioxa-5,9-diazapenta-decan-14-yl-(2S)-2-(3-[(tert.-butoxycarbonyl)amino]propanoyl}amino)-4-methylpentanoat

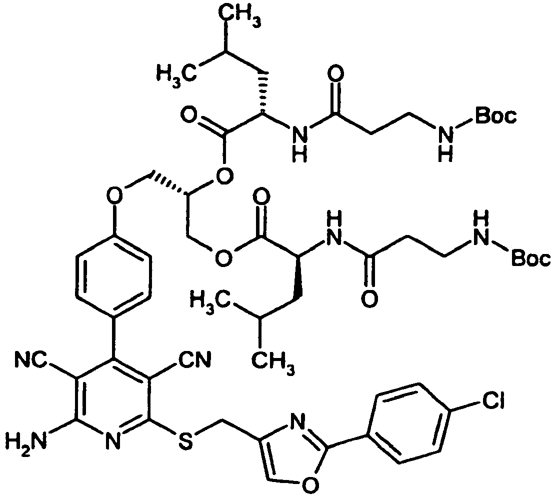

104 mg (0.549 mmol) *N*-(*tert*.-Butoxycarbonyl)-β-alanin wurden in 2 ml DMF vorgelegt, mit 51 mg (0.265 mmol) 1-(3-Dimethylaminopropyl)-3-ethylcarbodiimid-Hydrochlorid sowie 45 mg (0.332 mmol) 1-Hydroxy-1*H*-benzotriazol-Hydrat versetzt und 10 min gerührt. Anschließend wurden 217 mg (0.221 mmol) der Verbindung aus Beispiel 36A sowie 0.154 ml (0.885 mmol) *N,N-*Diisopropylethylamin zugegeben und die Mischung 7 h bei RT gerührt. Danach wurden nochmals die gleichen Mengen an *N*-(*tert*.-Butoxycarbonyl)-β-alanin, 1-(3-Dimethylaminopropyl)-3-ethylcarbodiimid-Hydrochlorid, 1-Hydroxy-1*H*-benzotriazol-Hydrat sowie *N*,*N*-Diisopropylethylamin hinzugefügt und das Gemisch über Nacht bei RT gerührt. Das DMF wurde danach im Vakuum abgezogen und der Rückstand zwischen Wasser und Dichlormethan verteilt. Die organische Phase wurde über Natriumsulfat getrocknet und eingeengt. Es wurden 240 mg der rohen Zielverbindung (54% Reinheit, 54% d. Th.) erhalten, welche ohne weitere Aufreinigung in der Folgereaktion eingesetzt wurde.
LC-MS (Methode 5): Rₜ = 1.65 min; MS (ESIpos): m/z = 1102 [M+H]⁺.

### Beispiel 38A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis{2-[(tert.-butoxycarbonyl)amino]-3-methylbutanoat}

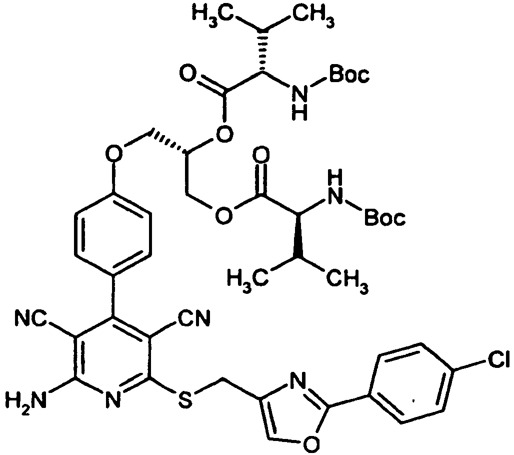

250 mg (0.468 mmol) der Verbindung aus Beispiel 8A wurden in 10 ml Dichlormethan vorgelegt, mit 254 mg (1.170 mmol) *N*-(*tert*.-Butoxycarbonyl)-L-valin, 269 mg (1.405 mmol) 1-(3-Dimethyl-aminopropyl)-3-ethylcarbodiimid-Hydrochlorid sowie 5.7 mg (0.047 mmol) 4-*N*,*N*-Dimethylaminopyridin versetzt und über Nacht bei RT gerührt. Das Reaktionsgemisch wurde danach im Vakuum eingeengt und der Rückstand mittels präparativer HPLC gereinigt (Laufmittel-Gradient: Acetonitril/Wasser 10:90 → 95:5). Es wurden 354 mg (81% d. Th.) der Zielverbindung erhalten.
LC-MS (Methode 10): Rₜ = 3.18 min; MS (ESIpos): m/z = 932 [M+H]⁺.

### Beispiel 39A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-amino-3-methylbutanoat)-Bis(trifluoressigsäure)-Salz

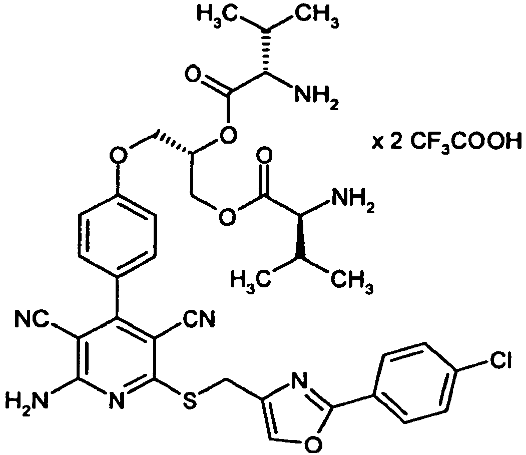

354 mg (0.380 mmol) der Verbindung aus Beispiel 38A wurden in 1 ml Dichlormethan vorgelegt, mit 0.262 ml (3.796 mmol) Trifluoressigsäure versetzt und 3 h bei RT gerührt. Es wurde dann die gleiche Menge an Trifluoressigsäure nachgegeben und weiter über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 279 mg (77% d. Th.) der Zielverbindung erhalten.
LC-MS (Methode 10): Rₜ = 1.76 min; MS (ESIpos): m/z = 732 [M+H]⁺.

### Beispiel 40A

### (10S,14S)-15-{4-[2-Amino-6-({[2-(4-chlorphenyl)-1,3-oxazol-4-yl]methyl}sulfanyl)-3,5-dicyano-pyridin-4-yl]phenoxy}-2,2-dimethyl-4,8,11-trioxo-10-(propan-2-yl)-3,12-dioxa-5,9-diazapenta-decan-14-yl-(2S)-2-({3-[(tert.-butoxycarbonyl)amino]propanoyl}amino)-3-methylbutanoat

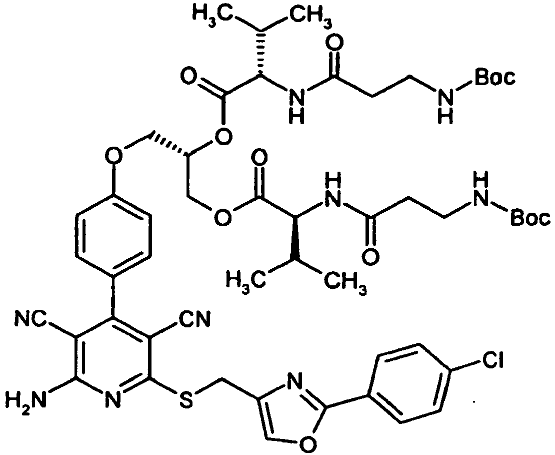

62 mg (0.325 mmol) *N*-(*tert*.-Butoxycarbonyl)-β-alanin wurden in 1 ml DMF vorgelegt, mit 30 mg (0.156 mmol) 1-(3-Dimethylaminopropyl)-3-ethylcarbodiimid-Hydrochlorid sowie 26 mg (0.195 mmol) 1-Hydroxy-1*H*-benzotriazol-Hydrat versetzt und 10 min gerührt. Anschließend wurden 217 mg (0.221 mmol) der Verbindung aus Beispiel 39A sowie 0.91 ml (0.521 mmol) *N*,*N*-Diisopropylethylamin zugegeben und die Mischung über Nacht bei RT gerührt. Danach wurde erneut jeweils die Hälfte der Ausgangsmengen an *N*-(*tert*.-Butoxycarbonyl)-β-alanin, 1-(3-Dimethyl-aminopropyl)-3-ethylcarbodiimid-Hydrochlorid, 1-Hydroxy-1*H*-benzotriazol-Hydrat sowie *N,N-*Diisopropylethylamin hinzugefügt und das Gemisch weitere 2 h bei RT gerührt. Das DMF wurde danach im Vakuum abgezogen und der Rückstand zwischen Wasser und Dichlormethan verteilt. Die organische Phase wurde über Natriumsulfat getrocknet und eingeengt. Es wurden 216 mg der Zielverbindung erhalten, welche als Rohprodukt ohne weitere Aufreinigung in der Folgereaktion eingesetzt wurde.
LC-MS (Methode 7): Rₜ = 3.07 min; MS (ESIpos): m/z = 1074 [M+H]⁺.

### Beispiel 41A

### (6R,9S,12S)-12-({4-[2-Amino-6-({[2-(4-chlorphenyl)-1,3-oxazol-4-yl]methyl}sulfanyl)-3,5-dicyanopyridin-4-yl]phenoxy}methyl)-2,2,6,9-tetramethyl-4,7,10-trioxo-3,11-dioxa-5,8-diazatridecan-13-yl-(2S)-2-({(2R)-2-[(tert.-butoxycarbonyl)amino]propanoyl}amino)propanoat

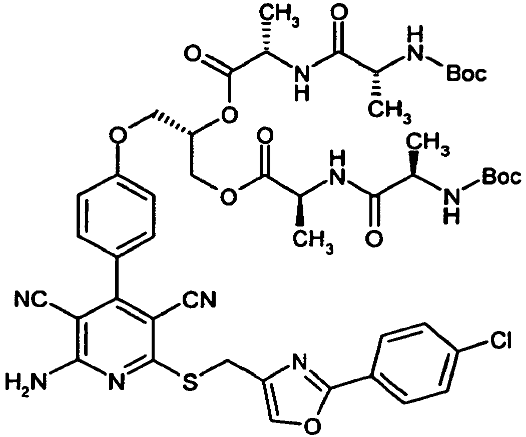

265 mg (1.402 mmol) *N*-(*tert*.-Butoxycarbonyl)-D-alanin wurden in 5 ml DMF vorgelegt, mit 269 mg (1.402 mmol) 1-(3-Dimethylaminopropyl)-3-ethylcarbodiimid-Hydrochlorid sowie 307 mg (2.002 mmol) 1-Hydroxy-1*H*-benzotriazol-Hydrat versetzt und 10 min gerührt. Anschließend wurden 500 mg (0.667 mmol) der Verbindung aus Beispiel 11A sowie 0.581 ml (3.337 mmol) *N*,*N*-Di-isopropylethylamin zugegeben und die Mischung 2 h bei RT gerührt. Der Reaktionsansatz wurde danach durch zweimalige präparative HPLC aufgereinigt (Laufmittel-Gradient: Acetonitril/Wasser 10:90 → 95:5). Es wurden 318 mg (47% d. Th.) der Zielverbindung erhalten.
LC-MS (Methode 8): Rₜ = 1.33 min; MS (ESIpos): m/z= 1018 [M+H]⁺.

### Beispiel 42A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2R,2'R)-bis{2-[(tert.-butoxycarbonyl)amino]propanoat}

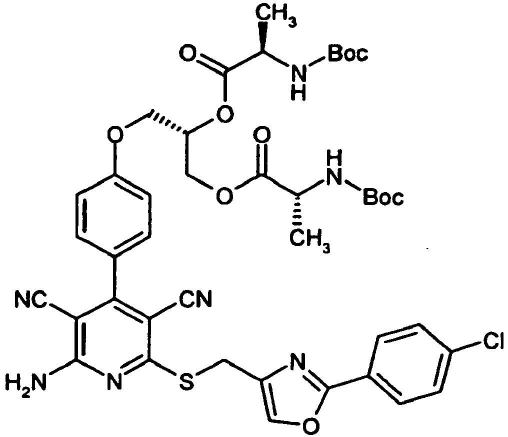

1.063 g (5.618 mmol) *N*-(*tert*.-Butoxycarbonyl)-D-alanin wurden in 10 ml DMF vorgelegt und mit 448 mg (2.341 mmol) 1-(3-Dimethylaminopropyl)-3-ethylcarbodiimid-Hydrochlorid sowie 114 mg (0.936 mmol) 4-*N*,*N*-Dimethylaminopyridin versetzt. Nach 5 min Rühren wurden 500 mg (0.936 mmol) der Verbindung aus Beispiel 8A hinzugefügt und die Mischung 2 h bei RT gerührt. Das Produkt wurde danach mittels präparativer HPLC isoliert (Acetonitril/Wasser-Gradient 10:90 → 95:5). Es wurden 676 mg (82% d. Th.) der Zielverbindung erhalten.
LC-MS (Methode 8): Rₜ = 1.42 min; MS (ESIneg): m/z = 874 [M-H]⁻.

### Beispiel 43A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2R,2'R)-bis(2-aminopropanoat)-Dihydrochlorid

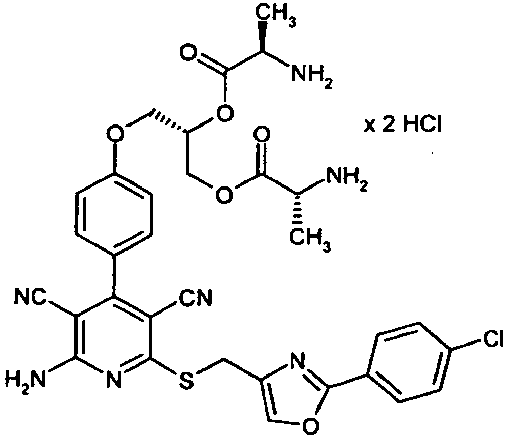

675 mg (0.770 mmol) der Verbindung aus Beispiel 42A wurden in 8 ml Dichlormethan vorgelegt und mit 15.4 ml einer 1 M Lösung von Chlorwasserstoff-Gas in Diethylether versetzt. Nach 4 h Rühren wurde der ausgefallene Feststoff abgesaugt, mit Dichlormethan und Diethylether gewaschen und im Vakuum getrocknet. Es wurden 577 mg (quant.) der Zielverbindung erhalten.
¹H-NMR (400 MHz, DMSO-d₆): δ = 8.55 (br. m, 6H), 8.38 (s, 1H), 8.33-8.02 (br. s, 2H), 7.97 (d, 2H), 7.61 (d, 2H), 7.50 (d, 2H), 7.13 (d, 2H), 5.51 (m, 1H), 4.56-4.44 (m, 2H), 4.42 (s, 2H), 4.39-4.33 (m, 2H), 4.16 (m, 2H), 1.44 (d, 3H), 1.39 (d, 3H).
LC-MS (Methode 8): Rₜ = 0.87 min; MS (ESIpos): m/z = 676 [M+H]⁺.

### Beispiel 44A

### (6R,9R,12S)-12-({4-[2-Amino-6-({[2-(4-chlorphenyl)-1,3-oxazol-4-yl]methyl}sulfanyl)-3,5-di-cyanopyridin-4-yl]phenoxy}methyl)-2,2,6,9-tetramethyl-4,7,10-trioxo-3,11-dioxa-5,8-diazatridecan-13-yl-(2R)-2-({(2R)-2-[(tert.-butoxycarbonyl)amino]propanoyl}amino)propanoat

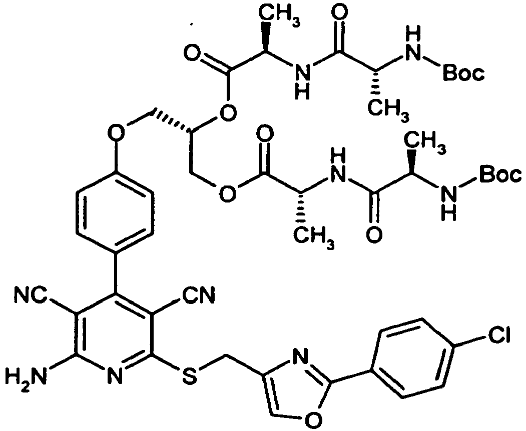

239 mg (1.262 mmol) *N*-(*tert*.-Butoxycarbonyl)-D-alanin wurden in 4.5 ml DMF vorgelegt, mit 242 mg (1.262 mmol) 1-(3-Dimethylaminopropyl)-3-ethylcarbodiimid-Hydrochlorid sowie 276 mg (1.802 mmol) 1-Hydroxy-1*H*-benzotriazol-Hydrat versetzt und 10 min gerührt. Anschließend wurden 450 mg (0.601 mmol) der Verbindung aus Beispiel 43A sowie 0.523 ml (3.004 mmol) *N*,*N*-Diisopropylethylamin zugegeben und die Mischung 4 h bei RT gerührt. Der Reaktionsansatz wurde danach direkt mittels präparativer HPLC aufgereinigt (Laufmittel-Gradient: Acetonitril/Wasser 10:90 → 95:5). Es wurden 438 mg (72% d. Th.) der Zielverbindung erhalten.
LC-MS (Methode 8): Rₜ = 1.34 min; MS (ESIpos): m/z = 1018 [M+H]⁺.

### Beispiel 45A

### (6S,9S,12S)-12-({4-[2-Amino-6-({[2-(4-chlorphenyl)-1,3-oxazol-4-yl]methyl}sulfanyl)-3,5-dicyanopyridin-4-yl]phenoxy}methyl)-2,2,9-trimethyl-4,7,10-trioxo-6-(propan-2-yl)-3,11-dioxa-5,8-diazatridecan-13-yl-(2S)-2-({(2S)-2-[(tert.-butoxycarbonyl)amino]-3-methylbutanoyl}amino)-propanoat

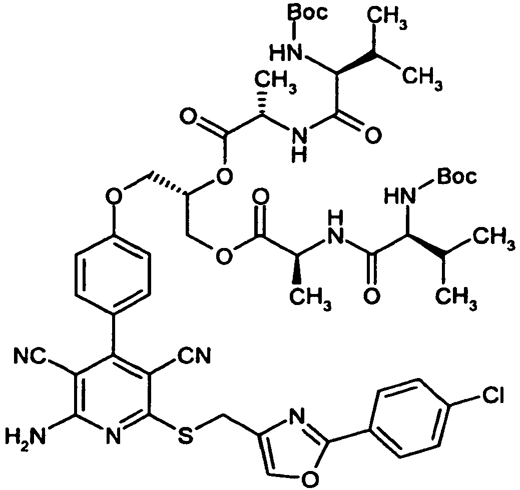

122 mg (0.561 mmol) *N*-(*tert*.-Butoxycarbonyl)-L-valin wurden in 10 ml DMF vorgelegt und nacheinander mit 107.5 mg (0.561 mmol) 1-(3-Dimethylaminopropyl)-3-ethylcarbodiimid-Hydrochlorid, 108 mg (0.801 mmol) 1-Hydroxy-1*H*-benzotriazol-Hydrat, 200 mg (0.267 mmol) der Verbindung aus Beispiel 11A sowie 0.233 ml (1.33 mmol) *N*,*N*-Diisopropylethylamin versetzt. Anschließend wurde das Reaktionsgemisch über Nacht bei RT gerührt. Danach wurde der Ansatz im Vakuum eingeengt und der Rückstand in 500 ml Dichlormethan aufgenommen. Es wurde jeweils zweimal mit 10%-iger Zitronensäure-Lösung und 10%-iger Natriumhydrogencarbonat-Lösung gewaschen und die organische Phase anschließend eingeengt. Der Rückstand wurde in 25 ml Ethylacetat aufgenommen und unter Rühren mit einer Mischung aus 10 ml Diethylether und 10 ml Pentan versetzt. Nach einer Stunde Rühren bei RT wurde der Niederschlag abgesaugt und im Hochvakuum getrocknet. Es wurden 240 mg (84% d. Th.) der Zielverbindung erhalten.
LC-MS (Methode 5): Rₜ = 1.63 min; MS (ESIpos): m/z = 1074 [M+H]⁺.

### Beispiel 46A

### (6S,13S)-13-({4-[2-Amino-6-({[2-(4-chlorphenyl)-1,3-oxazol-4-yl]methyl}sulfanyl)-3,5-dicyanopyridin-4-yl]phenoxy}methyl)-2,2,6-trimethyl-4,7,11-trioxo-3,12-dioxa-5,8-diazatetradecan-14-yl-3-({(2S)2-[(tert.-butoxycarbonyl)amino]propanoyl}amino)propanoat

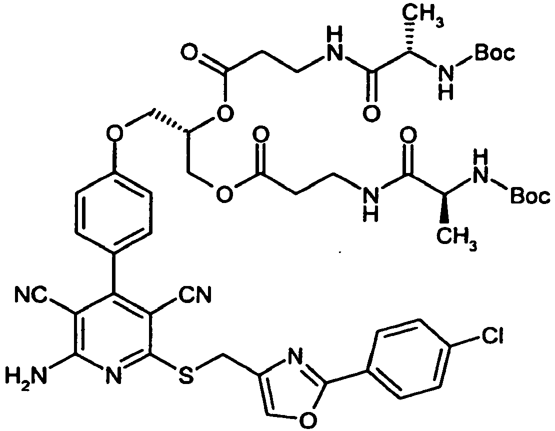

Die Herstellung der Titelverbindung erfolgte analog zur Herstellung von Beispiel 45A ausgehend von der Verbindung aus Beispiel 30A und käuflichem *N*-(*tert*.-Butoxycarbonyl)-L-alanin.
Ausbeute: 73% d. Th.
LC-MS (Methode 6): Rₜ = 2.39 min; MS (ESIpos): m/z = 1018 [M+H]⁺.

### Beispiel 47A

### (10S,13S)-13-({4-[2-Amino-6-({[2-(4-chlorphenyl)-1,3-oxazol-4-yl]methyl}sulfanyl)3,5-dicyanopyridin-4-yl]phenoxy}methyl)-2,2,10-trimethyl-4,8,11-trioxo-3,12-dioxa-5,9-diazatetradecan-14-yl-(2S)-2-({3-[(tert.-butoxycarbonyl)amino]propanoyl}amino)propanoat

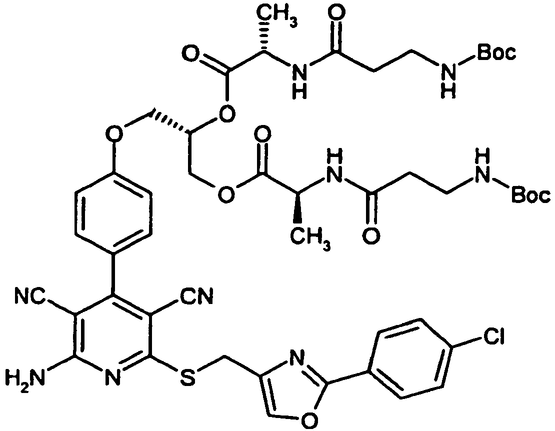

Die Herstellung der Titelverbindung erfolgte analog zur Herstellung von Beispiel 45A ausgehend von der Verbindung aus Beispiel 11A und käuflichem *N*-(*tert*.-Butoxycarbonyl)-β-alanin.
Ausbeute: 78% d. Th.
LC-MS (Methode 5): Rₜ = 1.47 min; MS (ESIpos): m/z = 1018 [M+H]⁺.

### Beispiel 48A

### (6S,9S,12S)-12-({4-[2-Amino-6-({[2-(4-chlorphenyl)-1,3-oxazol-4-yl]methyl}sulfanyl)-3,5-dicyanopyridin-4-yl]phenoxy}methyl)-2,2,6,9-tetramethyl-4,7,10-trioxo-3,11-dioxa-5,8-diazatridecan-13-yl-(2S)-2-({(2S)-2-[(tert.-butoxycarbonyl)amino]propanoyl}amino)propanoat

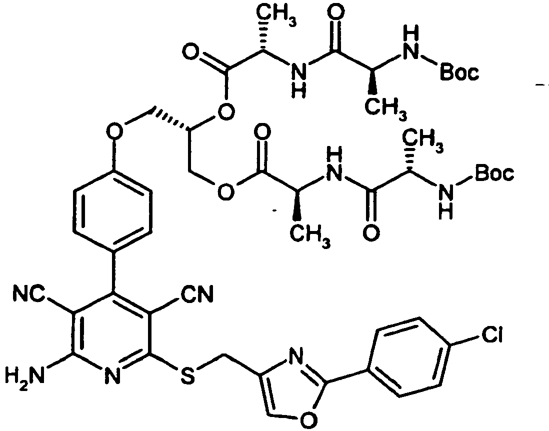

Die Herstellung der Titelverbindung erfolgte analog zur Herstellung von Beispiel 45A ausgehend von der Verbindung aus Beispiel 11A und käuflichem *N*-(*tert*.-Butoxycarbonyl)-L-alanin.
Ausbeute: 77% d. Th.
LC-MS (Methode 5): Rₜ = 1.47 min; MS (ESIpos): m/z = 1018 [M+H]⁺.

### Beispiel 49A

### (2R)-3-{4-[2-Amino-6-({[2-(4-chlorphenyl)-1,3-oxazol-4-yl]methyl}sulfanyl)3,5-dicyanopyridin-4-yl]phenoxy}propan-1,2-diyl-(2S,2'S)-bis{2-[(tert.-butoxycarbonyl)amino]propanoat}

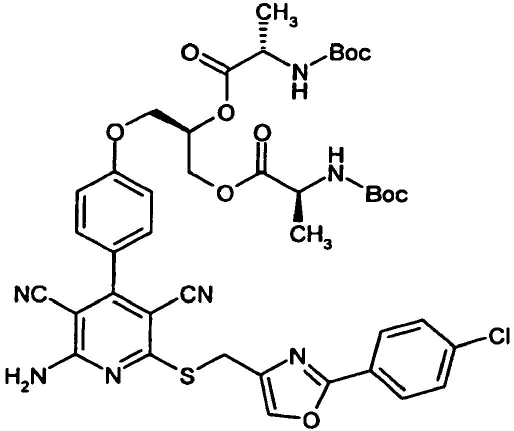

213 mg (1.12 mmol) *N*-Boc-L-Alanin wurden in 2 ml DMF vorgelegt und nacheinander mit 93 mg (0.49 mmol) 1-(3-Dimethylaminopropyl)-3-ethylcarbodiimid-Hydrochlorid, 23 mg (0.19 mmol) 4-*N,N*-Dimethylaminopyridin sowie 200 mg (0.375 mmol) der Verbindung aus Beispiel 9A versetzt. Die Reaktionsmischung wurde anschließend 2 h bei RT gerührt. Danach wurden nochmals die gleichen Mengen an *N*-Boc-L-Alanin, 1-(3-Dimethylaminopropyl)-3-ethylcarbodiimid-Hydrochlorid und 4-*N,N*-Dimethylaminopyridin hinzugefügt und die Mischung weiter über Nacht bei RT gerührt. Der Reaktionsansatz wurde dann direkt mittels präparativer HPLC aufgereinigt (Eluent: Acetonitril/Wasser-Gradient). Es wurden 293 mg (86% d. Th.) der Zielverbindung erhalten.
LC-MS (Methode 7): Rₜ = 3.17 min; MS (ESIpos): m/z = 876 [M+H]⁺.

### Beispiel 50A

### (2R)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-aminopropanoat)-Dihydrochlorid

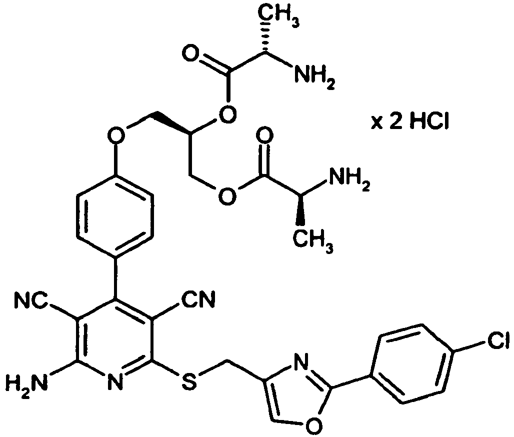

282 g (0.32 mmol) der Verbindung aus Beispiel 49A wurden in 3 ml Dichlormethan vorgelegt und tropfenweise mit 1.6 ml (3.2 mmol) einer 2 M Lösung von Chlorwasserstoff in Diethylether versetzt. Es wurde drei Stunden bei Raumtemperatur gerührt und die Mischung dann im Vakuum eingeengt. Der Rückstand wurde mit 5 ml Acetonitril versetzt und das Gemisch anschließend erneut eingeengt. Diese Prozedur wurde noch einmal wiederholt. Nach Trocknen des Rückstands im Vakuum wurden 215 mg (89% d. Th.) der Zielverbindung erhalten.
LC-MS (Methode 5): Rₜ = 0.99 min; MS (ESIpos): m/z = 676 [M+H]⁺.

### Beispiel 51A

### (10S,13S,17R)-18-{4-[2-Amino-6-({[2-(4-chlorphenyl)-1,3-oxazol-4-yl]methyl}sulfanyl)-3,5-dicyanopyridin-4-yl]phenoxy}-10-[(tert.-butoxycarbonyl)amino]-2,2,13-trimethyl-4,11,14-trioxo-3,15-dioxa-5,12-diazaoctadecan-17-yl-(2S)-2-({(2S)-2,6-bis[(tert.-butoxycarbonyl)amino]-hexanoyl}amino)propanoat

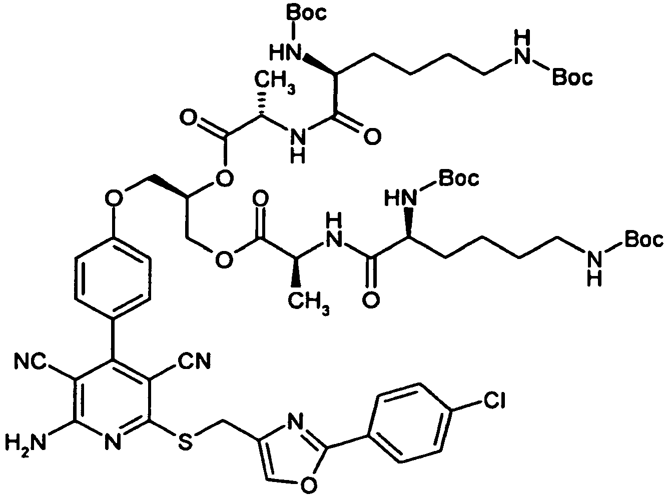

333 mg (0.63 mmol) *N²*,*N⁶*-Bis(*tert*.-butoxycarbonyl)-L-lysin-Dicyclohexylamin-Salz wurden in 2.9 ml DMF vorgelegt und nacheinander mit 116 mg (0.86 mmol) 1-Hydroxy-1*H*-benzouiazol-Hydrat, 132 mg (0.69 mmol) 1-(3-Dimethylaminopropyl)-3-ethylcarbodiimid-Hydrochlorid, 0.25 ml (1.44 mmol) *N,N*-Diisopropylethylamin und 215 mg (0.29 mmol) der Verbindung aus Beispiel 50A versetzt. Die Reaktionsmischung wurde anschließend über Nacht bei RT gerührt. Danach wurde mit Wasser versetzt und der entstandene Niederschlag abfiltriert. Der Feststoff wurde in ca. 5 ml Acetonitril gelöst und mittels präparativer HPLC gereinigt (Eluent: Acetonitril/Wasser-Gradient). Es wurden 297 mg (35% d. Th.) der Zielverbindung erhalten.
LC-MS (Methode 6): Rₜ = 2.82 min; MS (ESIpos): m/z = 1334 [M+H]⁺.

### Beispiel 52A

### (10S,13S,17S)-18-{4-[2-Amino-6-({[2-(4-chlorphenyl)-1,3-oxazol-4-yl]methyl}sulfanyl)-3,5-dicyanopyridin-4-yl]phenoxy}-10-[(tert.-butoxycarbonyl)amino]-2,2,13-trimethyl-4,11,14-trioxo-3,15-dioxa-5,12-diazaoctadecan-17-yl-(2S)-2-({(2S)-2,6-bis[(tert.-butoxycarbonyl)amino]-hexanoyl}amino)propanoat

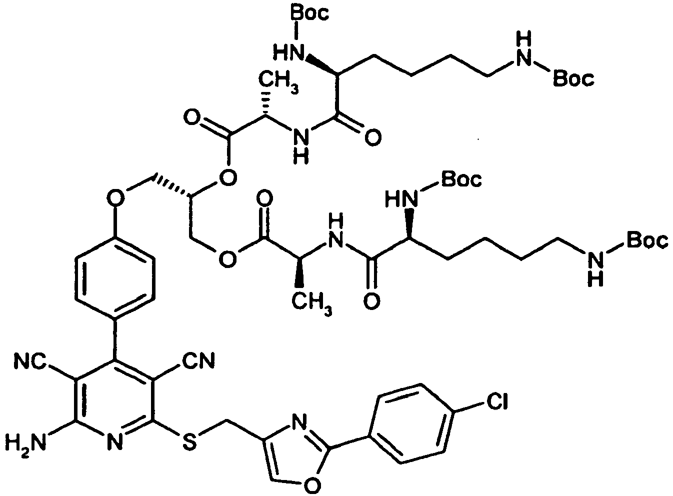

372 mg (0.71 mmol) *N²,N⁶*-Bis(*tert*.-butoxycarbonyl)-L-lysin-Dicyclohexylamin-Salz wurden in 3.2 ml DMF vorgelegt und nacheinander mit 130 mg (0.96 mmol) 1-Hydroxy-1*H*-benzotriazol-Hydrat, 147 mg (0.77 mmol) 1-(3-Dimethylaminopropyl)-3-ethylcarbodümid-Hydrochlorid, 0.28 ml (1.60 mmol) *N,N*-Diisopropylethylamin und 240 mg (0.32 mmol) der Verbindung aus Beispiel 11A versetzt. Die Reaktionsmischung wurde anschließend über Nacht bei RT gerührt. Danach wurde mit Wasser versetzt und der entstandene Niederschlag abfiltriert. Der Feststoff wurde in ca. 5 ml Acetonitril gelöst und mittels präparativer HPLC gereinigt (Eluent: Acetonitril/Wasser-Gradient). Es wurden 326 mg (35% d. Th.) der Zielverbindung erhalten.
LC-MS (Methode 7): Rₜ = 3.24 min; MS (ESIpos): m/z = 1334 [M+H]⁺.

### Beispiel 53A

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,3R,2'S,3'R)-bis{3-tert.-butoxy-2-[(tert.-butoxycarbonyl)amino]-butanoat

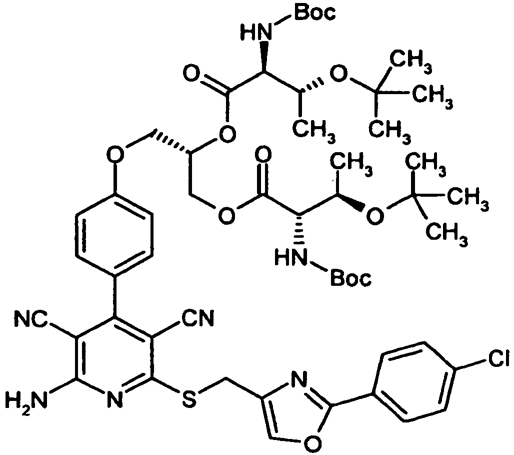

800 mg (1.50 mmol) der Verbindung aus Beispiel 8A wurden in 16 ml DMF/Dichlormethan (1:1) vorgelegt und nacheinander mit 1.237 g (4.49 mmol) *N*-(*tert*.-Butoxycarbonyl)-*O*-*tert*.-butyl-L-threonin, 1.005 g (5.24 mmol) 1-(3-Dimethylaminopropyl)-3-ethylcarbodiimid-Hydrochlorid und 36 mg (0.30 mmol) 4-*N,N*-Dimethylaminopyridin versetzt. Die Reaktionsmischung wurde anschließend über Nacht bei RT gerührt und dann im Vakuum eingeengt. Der Rückstand wurde mit etwas Acetonitril versetzt und mittels präparativer HPLC gereinigt (Eluent: Acetonitril/Wasser + 0.1% TFA). Es wurden 916 mg (58% d. Th.) der Zielverbindung erhalten.
LC-MS (Methode 5): Rₜ = 1.92 min; MS (ESIpos): m/z = 1048 [M+H]⁺.

### Beispiel 54A

### (2S)3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,3R,2'S,3'R)-bis(2-amino-3-hydroxybutanoat)-Bis(trifluoressigsäure)-Salz

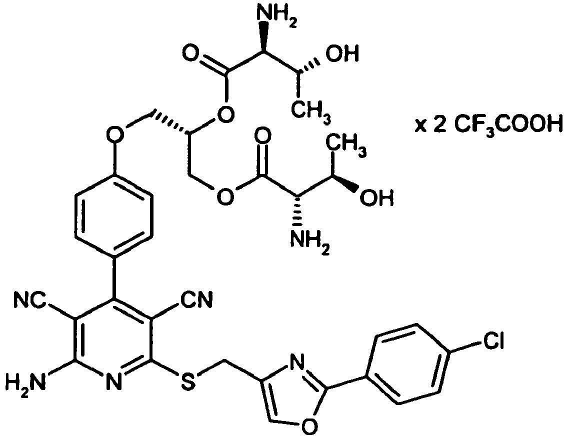

916 mg (0.873 mmol) der Verbindung aus Beispiel 53A wurden in 10 ml Dichlormethan vorgelegt, tropfenweise mit 1.35 ml (17.47 mmol) Trifluoressigsäure versetzt und über Nacht bei RT gerührt. Anschließend wurde das Reaktionsgemisch im Vakuum eingeengt und der Rückstand mittels präparativer HPLC gereinigt (Eluent: Acetonitril/Wasser + 0.1% TFA). Es wurden 574 mg (68% d. Th.) der Zielverbindung erhalten.
¹H-NMR (400 MHz, DMSO-d₆): 8 = 8.42-8.25 (m, 5H), 8.15 (br. s, 2H), 7.98 (d, 2H), 7.61 (d, 2H), 7.51 (d, 2H), 7.12 (d, 2H), 5.68 (br. s, 1H), 5.50 (quint, 1H), 4.50 (d, 2H), 4.42 (s, 2H), 4.38-4.32 (m, 2H), 4.24-3.95 (m, 5H), 1.24-1.18 (m, 6H).
LC-MS (Methode 7): Rₜ = 1.45 min; MS (ESIpos): m/z = 736 [M+H]⁺.

### Beispiel 55A

### (9S,13S)-14-{4-[2-Amino-6-({[2-(4-chlorphenyl)-1,3-oxazol-4-yl]methyl}sulfanyl)-3,5-dicyanopyridin-4-yl]phenoxy}-9-[(1R)-1-hydroxyethyl]-2,2-dimethyl-4,7,10-trioxo-3,11-dioxa-5,8-diazatetradecan-13-yl-(2S,3R)2-({[(tert.-butoxycarbonyl)amino]acetyl}amino)-3-hydroxybutanoat

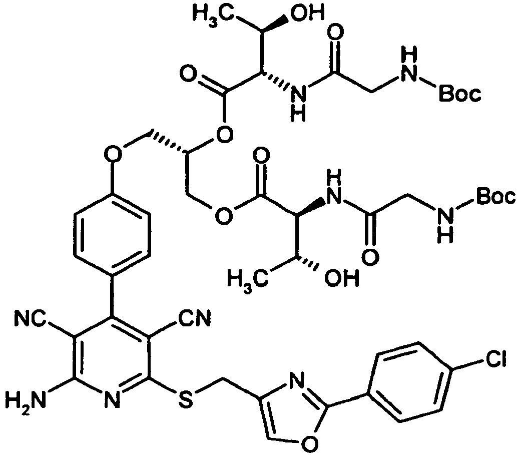

94 mg (0.54 mmol) *N*-(*tert*.-Butoxycarbonyl)glycin wurden in 0.95 ml DMF bei 0°C vorgelegt und mit 154 mg (0.40 mmol) *N*-[(Dimethylamino)(3*H*-[1,2,3]triazolo[4,5-b]pyridin-3-yloxy)-methyliden]-*N*-methylmethanaminium-Hexafluorophosphat (HATU) versetzt. Nach 20 min Rühren wurden bei 0°C 130 mg (0.14 mmol) der Verbindung aus Beispiel 54A und 0.07 ml (0.40 mmol) *N,N*-Diisopropylethylamin hinzugegeben. Die Reaktionsmischung wurde anschließend über Nacht bei 60°C gerührt. Danach wurde mit etwas Wasser/THF versetzt und das Gemisch direkt mittels präparativer HPLC aufgereinigt (Eluent: Acetonitril/Wasser + 0.1% TFA). Es wurden 100 mg (61% d. Th.) der Zielverbindung erhalten.
LC-MS (Methode 7): Rₜ = 2.72 min; MS (ESIpos): m/z = 1051 [M+H]⁺.

### Beispiel 56A

### 6S,9S,13S)-14-{4-[2-Amino-6-({[2-(4-chlorphenyl)-1,3-oxazol-4-yl]methyl}sulfanyl)-3,5-dicyanopyridin-4-yl]phenoxy}-9-[(1R)-1-hydroxyethyl]-2,2,6-trimethyl-4,7,10-trioxo-3,11-dioxa-5,8-diazatetradecan-13-yl-(2S,3R)2-({(2S)-2-[(tert.-butoxycarbonyl)amino]propanoyl}amino)-3-hydroxybutanoat

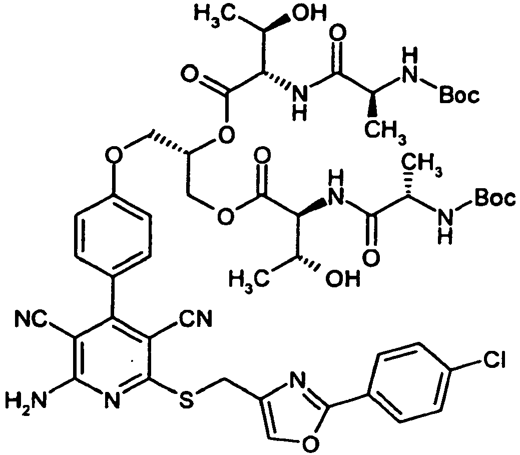

212 mg (1.12 mmol) *N*-(*tert*.-Butoxycarbonyl)-L-alanin wurden in 1.97 ml DMF bei 0°C vorgelegt und mit 319 mg (0.84 mmol) *N*-[(Dimethylamino)(3*H*-[1,2,3]triazolo[4,5-b]pyridin-3-yloxy)-methyliden]-*N*-methylmethanaminium-Hexafluorophosphat (HATU) versetzt. Nach 20 min Rühren wurden bei 0°C 270 mg (0.28 mmol) der Verbindung aus Beispiel 54A und 0.15 ml (0.84 mmol) *N,N*-Diisopropylethylamin hinzugegeben. Die Reaktionsmischung wurde anschließend über Nacht bei 60°C gerührt. Danach wurde mit etwas Wasser/THF versetzt und das Gemisch direkt mittels präparativer HPLC aufgereinigt (Eluent: Acetonitril/Wasser + 0.1% TFA). Es wurden 188 mg (62% d. Th.) der Zielverbindung erhalten.
LC-MS (Methode 5): Rₜ = 1.44 min; MS (ESIpos): m/z = 1079 [M+H]⁺.

### Beispiel 57A

### (6S,14S)15-{4-[2-Amino-6-({[2-(4-chlorphenyl)-1,3-oxazol-4-yl]methyl}sulfanyl)-3,5-dicyanopyridin-4-yl]phenoxy}-6-[(1R)-1-tert.-butoxyethyl]-2,2-dimethyl-4,7,11-trioxo-3,12-dioxa-5,8-diazapentadecan-14-yl-3-({(2S,3R)-3-tert.-butoxy-2-[(tert.-butoxycarbonyl)amino]butanoyl}-amino)propanoat

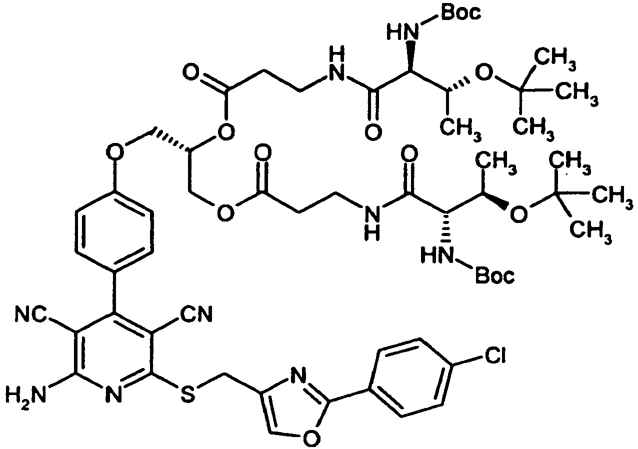

134 mg (0.49 mmol) *N*-(*tert*.-Butoxycarbonyl)-*O*-*tert*.-butyl-L-threonin wurden in 3 ml DMF vorgelegt und mit 102 mg (0.53 mmol) 1-(3-Dimethylaminopropyl)-3-ethylcarbodiimid-Hydrochlorid, 90 mg (0.66 mmol) 1-Hydroxy-1*H*-benzotriazol-Hydrat sowie 0.29 ml (1.66 mmol) *N,N-*Diisopropylethylamin versetzt. Nach 15 min Rühren bei RT wurden 200 mg (0.22 mmol) der Verbindung aus Beispiel 30A hinzugegeben. Die Reaktionsmischung wurde anschließend über Nacht bei RT gerührt. Danach wurde mit etwas Wasser/THF versetzt und das Gemisch direkt mittels präparativer HPLC aufgereinigt (Eluent: Acetonitril/Wasser + 0.1% TFA). Es wurden 106 mg (40% d. Th.) der Zielverbindung erhalten.
LC-MS (Methode 5): Rₜ = 1.76 min; MS (ESIpos): m/z = 1190 [M+H]⁺.

### Ausführungsbeispiele:

### Beispiel 1

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2-amino-4-methylpentanoyl]amino}-propanoat)-Bis(trifluoressigsäure)-Salz

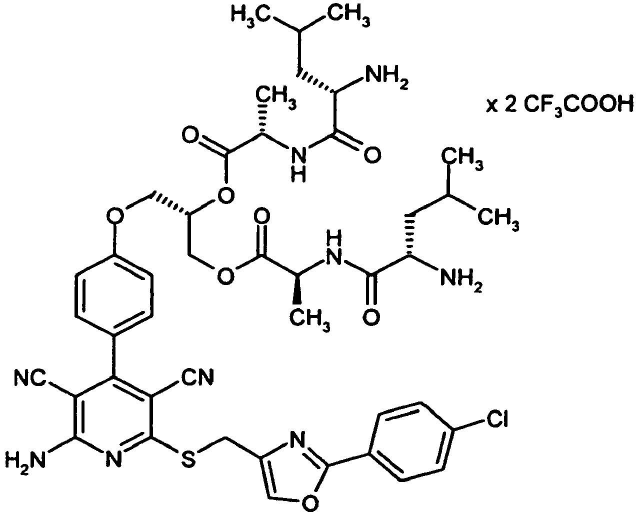

185 mg (0.168 mmol) der Verbindung aus Beispiel 13A wurden in 2 ml Dichlormethan vorgelegt und tropfenweise mit 1.68 ml (3.36 mmol) einer 2 M Lösung von Chlorwasserstoff in Diethylether versetzt. Nach einstündigem Rühren bei RT wurde der ausgefallene Feststoff abgesaugt, mit Diethylether gewaschen und im Hochvakuum getrocknet. Dieses Rohprodukt wurde anschließend mittels präparativer HPLC gereinigt (Laufmittel-Gradient: Acetonitril/Wasser 10:90 → 95:5 unter Zusatz von 0.1% TFA). Es wurden 40 mg (21% d. Th.) der Zielverbindung erhalten.
¹H-NMR (400 MHz, DMSO-d₆): δ = 8.92 (d, 2H), 8.37 (s, 1H), 8.30-8.04 (m, 8H), 7.96 (d, 2H), 7.61 (d, 2H), 7.50 (d, 2H), 7.11 (d, 2H), 5.37 (m, 1H), 4.42-4.21 (m, 8H), 3.75 (br. s, 2H), 1.70 (br. m, 2H), 1.62-1.46 (br. m, 4H), 1.35 (q, 6H), 0.92-0.86 (m, 12H).
LC-MS (Methode 7): Rₜ = 1.55 min; MS (ESIpos): m/z = 902 [M+H]⁺.

### Beispiel 2

### (2S)-3-{4-[2-Amino-6-({[2-(4-chlorphenyl}-1,3-oxazol-4-yl]methyl}sulfanyl)-3,5-dicyanopyridin-4-yl]phenoxy}propan-1,2-diyl-(2S,2'S)-bis(2-{[(2S,3S)-2-amino-3-methylpentanoyl]amino}-propanoat)-Dihydrochlorid

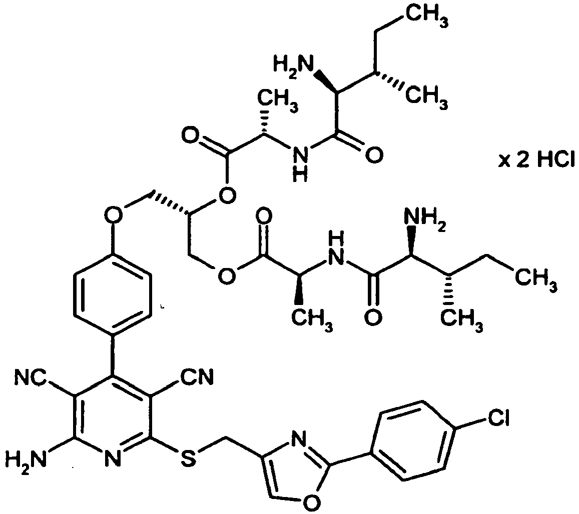

177 mg (0.161 mmol) der Verbindung aus Beispiel 14A wurden in 2 ml Dichlormethan vorgelegt und tropfenweise mit 1.61 ml (3.21 mmol) einer 2 M Lösung von Chlorwasserstoff in Diethylether versetzt. Nach einstündigem Rühren bei RT wurde der ausgefallene Feststoff abgesaugt, mit Diethylether gewaschen und im Hochvakuum getrocknet. Es wurden 128 mg (78% d. Th.) der Zielverbindung erhalten.
¹H-NMR (400 MHz, DMSO-d₆): δ = 8.90 (d, 2H), 8.41 (s, 1H), 8.17 (m, 6H), 7.97 (d, 2H), 7.61 (d, 2H), 7.49 (d, 2H), 7.10 (d, 2H), 5.40 (m, 1H), 4.42-4.21 (m, 8H), 3.64 (m, 2H), 1.84 (m, 2H), 1.56-1.51 (m, 4H), 1.34 (q, 6H), 1.16 (m, 2H), 0.92 (d, 6H), 0.88-0.83 (m, 6H).
LC-MS (Methode 5): Rₜ = 1.04 min; MS (ESIpos): m/z = 902 [M+H]⁺.

### Beispiel 3

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2,4-diaminobutanoyl]amino}propanoat Tetrahydrochlorid

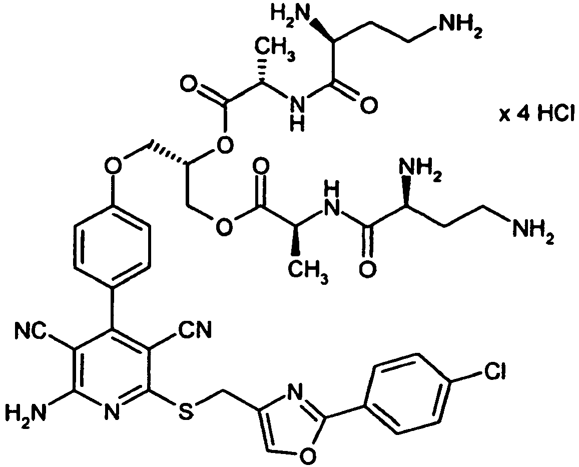

163 mg (0.128 mmol) der Verbindung aus Beispiel 15A wurden in 2 ml Dichlormethan vorgelegt und tropfenweise mit 1.28 ml (2.55 mmol) einer 2 M Lösung von Chlorwasserstoff in Diethylether versetzt. Nach einstündigem Rühren bei RT wurde der ausgefallene Feststoff abgesaugt, mit Diethylether gewaschen und im Hochvakuum getrocknet. Es wurden 117 mg (90% d. Th.) der Zielverbindung erhalten.
¹H-NMR (400 MHz, DMSO-d₆): δ = 9.44-9.39 (m, 2H), 8.55-8.39 (m, 7H), 8.38 (s, 1H), 8.29-8.13 (m, 7H), 7.97 (d, 2H), 7.60 (d, 2H), 7.50 (d, 2H), 7.14 (d, 2H), 5.40 (m, 1H), 4.49-4.26 (m, 8H), 4.15-4.02 (m, 2H), 3.09-2.95 (m, 4H), 2.15-2.02 (m, 4H), 1.37 (m, 6H).
LC-MS (Methode 8): Rₜ = 0.70 min; MS (ESIneg): m/z = 874 [M-H]⁻.

### Beispiel 4

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)2,5-diaminopentanoyl]amino}propanoat)-Tetrahydrochlorid

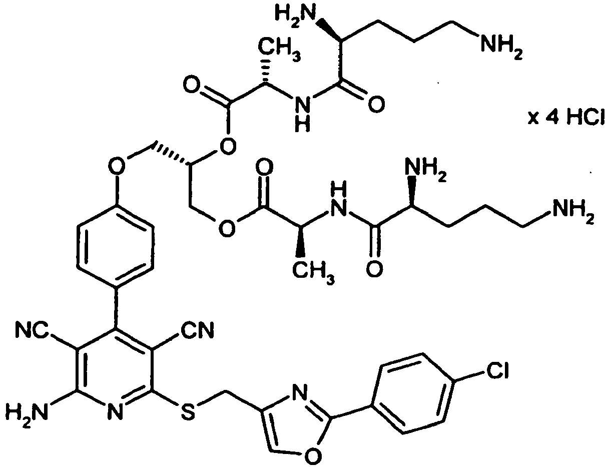

184 mg (0.141 mmol) der Verbindung aus Beispiel 16A wurden in 2 ml Dichlormethan vorgelegt und tropfenweise mit 1.41 ml (2.82 mmol) einer 2 M Lösung von Chlorwasserstoff in Diethylether versetzt. Nach einstündigem Rühren bei RT wurde der ausgefallene Feststoff abgesaugt, mit Diethylether gewaschen und bei 40°C 2 Tage lang im Vakuum getrocknet. Es wurden 98 mg (66% d. Th.) der Zielverbindung erhalten.
¹H-NMR (400 MHz, DMSO-d₆): δ = 9.25 (m, 2H), 8.46-8.37 (m, 8H), 8.20-8.05 (m, 7H), 7.96 (d, 2H), 7.61 (d, 2H), 7.50 (d, 2H), 7.14 (d, 2H), 5.38 (m, 1H), 4.51-4.25 (m, 8H), 3.90 (m, 2H), 2.92 (m, 4H), 1.91-1.70 (m, 8H), 1.37 (m, 6H).
LC-MS (Methode 6): Rₜ = 0.86 min; MS (ESIpos): m/z = 904 [M+H]⁺.

### Beispiel 5

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2-amino-3-hydroxypropanoyl]amino}-propanoat)-Bis(trifluoressigsäure)-Salz

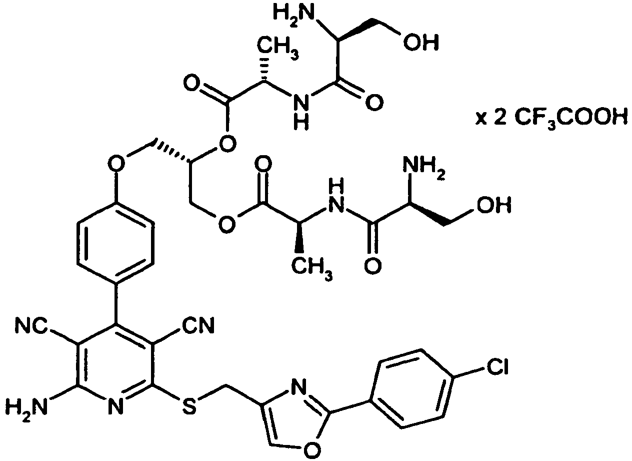

158 mg (0.136 mmol) der Verbindung aus Beispiel 17A wurden in 2 ml Dichlormethan vorgelegt und tropfenweise mit 1.36 ml (2.72 mmol) einer 2 M Lösung von Chlorwasserstoff in Diethylether versetzt. Nach zweistündigem Rühren bei RT wurde der ausgefallene Feststoff abgesaugt, mit Dichlormethan gewaschen und im Hochvakuum getrocknet. Von dem so erhaltenen Rohprodukt (Ausbeute: 100 mg) wurde eine Teilmenge von 50 mg mittels präparativer HPLC gereinigt (Laufmittel-Gradient: Acetonitril/Wasser 10:90 → 95:5 unter Zusatz von 0.1% TFA). Es wurden 33 mg (22% d. Th.) der Zielverbindung erhalten.
¹H-NMR (400 MHz, DMSO-d₆): δ = 8.87 (m, 2H), 8.37 (s, 1H), 8.30-8.02 (m, 8H), 7.97 (d, 2H), 7.61 (d, 2H), 7.49 (d, 2H), 7.14 (d, 2H), 5.52 (br. m, 2H), 5.36 (m, 1 H), 4.42-4.23 (m, 8H), 3.84-3.79 (m, 4H), 3.61 (m, 2H), 1.33 (m, 6H).
LC-MS (Methode 7): Rₜ = 1.41 min; MS (ESIpos): m/z = 850 [M+H]⁺.

### Beispiel 6

### (2S)-3-{4-[2-Amino-6-({[2-(4-chlorphenyl)-1,3-oxazol-4-yl]methyl}sulfanyl)3,5-dicyanopyridin-4-yl]phenoxy}propan-1,2-diyl-(2S,2'S)-bis(2-{[(2S)-2-amino-3-phenylpropanoyl]amino}-propanoat)-Bis(trifluoressigsäure)-Salz

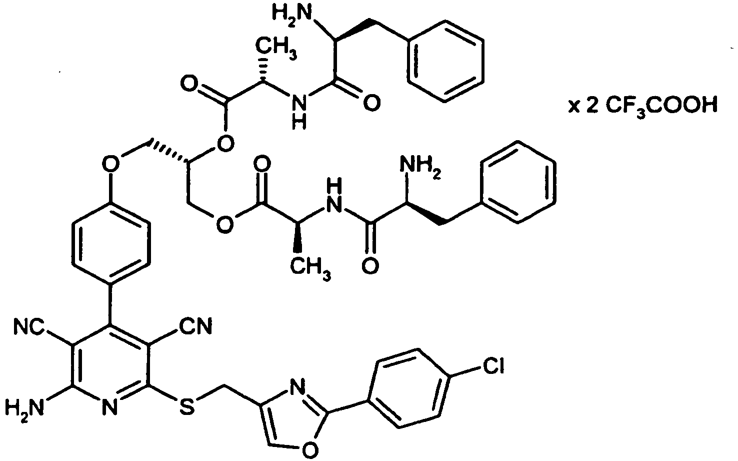

160 mg (0.137 mmol) der Verbindung aus Beispiel 18A wurden in 2 ml Dichlormethan vorgelegt und tropfenweise mit 1.37 ml (2.73 mmol) einer 2 M Lösung von Chlorwasserstoff in Diethylether versetzt. Nach einstündigem Rühren bei RT wurde der ausgefallene Feststoff abgesaugt, mit Diethylether gewaschen und im Vakuum getrocknet. Von dem so erhaltenen Rohprodukt (Ausbeute: 102 mg) wurde eine Teilmenge von 50 mg mittels präparativer HPLC gereinigt (Laufmittel-Gradient: Acetonitril/Wasser 10:90 → 95:5 unter Zusatz von 0.1% TFA). Es wurden 41 mg (25% d. Th.) der Zielverbindung erhalten.
¹H-NMR (400 MHz, DMSO-d₆): δ = 8.97 (m, 2H), 8.37 (s, 1H), 8.27-8.05 (m, 8H), 7.97 (d, 2H), 7.60 (d, 2H), 7.45 (d, 2H), 7.35-7.12 (m, 10H), 7.07 (d, 2H), 5.40 (m, 1H), 4.46-4.37 (m, 6H), 4.28 (m, 2H), 4.02 (br. s, 2H), 3.15 (m, 2H), 2.92 (m, 2H), 1.34 (m, 6H).
LC-MS (Methode 7): Rₜ = 1.59 min; MS (ESIpos): m/z = 970 [M+H]⁺.

### Beispiel 7

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis{2-[(aminoacetyl)amino]propanoat}-Dihydrochlorid

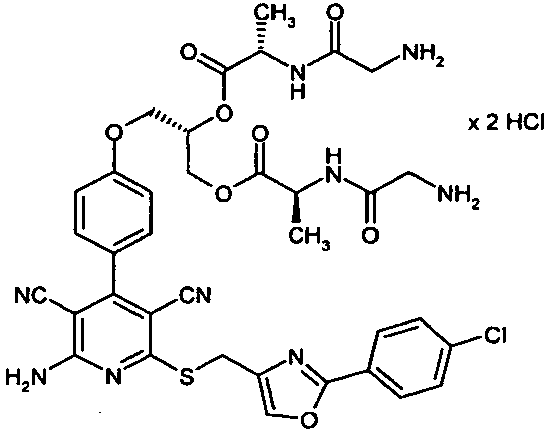

2.37 g (2.393 mmol) der Verbindung aus Beispiel 19A wurden in 200 ml Dichlormethan gelöst. Anschließend wurde unter Rühren 1 h lang bei 15°C Chlorwasserstoff-Gas durch die Lösung geleitet. Der ausgefallene Feststoff wurde abfiltriert, mit Dichlormethan gewaschen und im Vakuum getrocknet. Es wurden 1.89 g (88% d. Th.) der Zielverbindung erhalten.
¹H-NMR (400 MHz, DMSO-d₆): δ = 8.94 (m, 2H), 8.39 (s, 1H), 8.32-8.00 (m, 8H), 7.96 (d, 2H), 7.61 (d, 2H), 7.49 (d, 2H), 7.14 (d, 2H), 5.40 (m, 1H), 4.42-4.24 (m, 8H), 3.64-3.51 (m, 4H), 1.33 (m, 6H).
LC-MS (Methode 7): Rₜ = 1.46 min; MS (ESIpos): m/z = 790 [M+H]⁺.

### Beispiel 8

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-pyrrolidin-2-ylcarbonyl]amino}propanoat)-Bis(trifluoressigsäure)-Salz

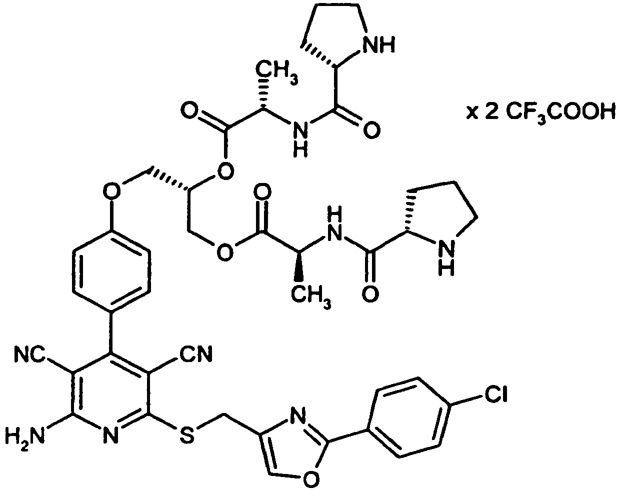

180 mg (0.168 mmol) der Verbindung aus Beispiel 20A wurden in 1 ml Dichlormethan vorgelegt, mit 0.26 ml (3.362 mmol) Trifluoressigsäure versetzt und 2 h bei RT gerührt. Anschließend wurde das Reaktionsgemisch eingeengt und der Rückstand mittels präparativer HPLC gereinigt (Laufmittel-Gradient: Acetonitril/Wasser 10:90 → 95:5 unter Zusatz von 0.1% TFA). Es wurden 150 mg (81% d. Th.) der Zielverbindung erhalten.
¹H-NMR (400 MHz, DMSO-d₆): δ = 9.31 (m, 2H), 8.97 (m, 2H), 8.65-8.49 (m, 2H), 8.37 (s, 1H), 8.28-8.03 (br. s, 2H), 7.97 (d, 2H), 7.61 (d, 2H), 7.50 (d, 2H), 7.11 (d, 2H), 5.38 (m, 1H), 4.42-4.37 (m, 6H), 4.31-4.19 (m, 4H), 3.23 (m, 2H), 2.29 (m, 2H), 1.91-1.76 (m, 8H), 1.36-1.33 (d, 6H).
LC-MS (Methode 5): Rₜ = 1.04 min; MS (ESIpos): m/z = 870 [M+H]⁺.

### Beispiel 9

### (2S)3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2,4-diamino-4-oxobutanoyl]amino}-propanoat)-Bis(trifluoressigsäure)-Salz

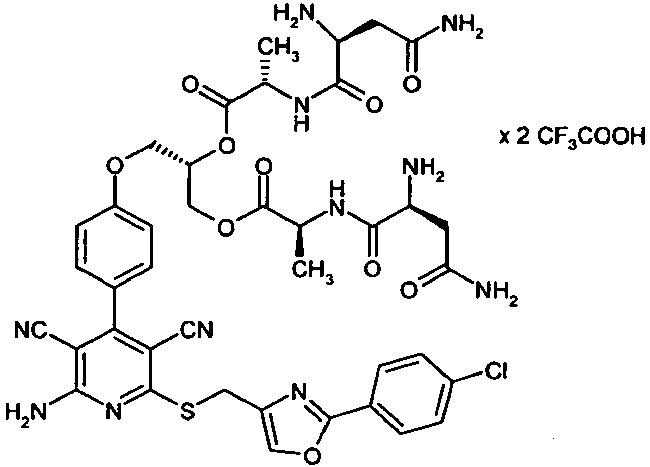

64 mg (0.042 mmol) der Verbindung aus Beispiel 23A wurden in 1 ml Dichlormethan vorgelegt, mit 0.033 ml (0.423 mmol) Trifluoressigsäure versetzt und über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 18 mg (37% d. Th.) der Zielverbindung erhalten.
¹H-NMR (400 MHz, DMSO-d₆): δ = 8.86 (m, 2H), 8.36 (s, 1H), 8.28-7.99 (m, 8H), 7.97 (d, 2H), 7.71 (br. s, 2H), 7.61 (d, 2H), 7.50 (d, 2H), 7.30 (br. s, 2H), 7.13 (d, 2H), 5.38 (m, 1H), 4.45-4.24 (m, 8H), 4.10 (m, 2H), 2.76-2.67 (m, 2H), 2.61-2.56 (m, 2H), 1.32 (m, 6H).
LC-MS (Methode 6): R, = 1.20 min; MS (ESIpos): m/z = 904 [M+H]⁺.

### Beispiel 10

### (3S,6S,9S,13S,16S)-3,16-Diamino-9-({4-[2-amino-6-({[2-(4-chlorphenyl)-1,3-oxazol-4-yl]methyl}-sulfanyl)-3,5-dicyanopyridin-4-yl]phenoxy}methyl)-6,13-dimethyl-4,7,12,15-tetraoxo-8,11-dioxa-5,14-diazaoctadecan-1,18-disäure-Bis(trifluoressigsäure)-Salz

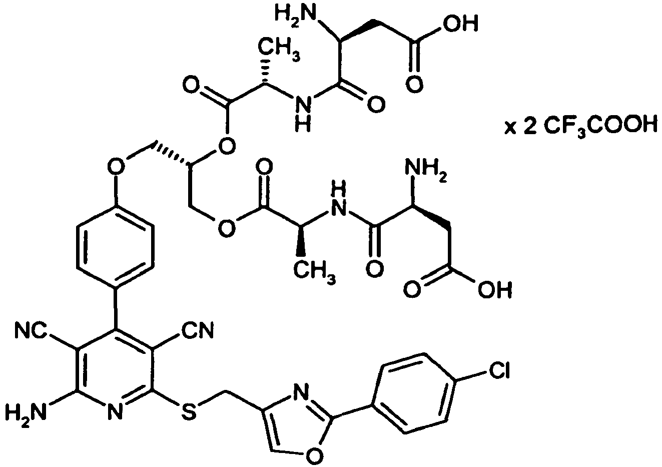

410 mg (0.336 mmol) der Verbindung aus Beispiel 24A wurden in 2 ml Dichlormethan vorgelegt, mit 0.259 ml (3.364 mmol) Trifluoressigsäure versetzt und über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 191 mg (50% d. Th.) der Zielverbindung erhalten.
¹H-NMR (400 MHz, DMSO-d₆): δ = 13.40-12.77 (br. s, 2H), 8.90-8.88 (m, 2H), 8.37 (s, 1H), 8.21 (m, 6H), 7.97 (d, 2H), 7.61 (d, 2H), 7.49 (d, 2H), 7.13 (d, 2H), 5.38 (m, 1H), 4.42-4.23 (m, 8H), 4.12 (m, 2H), 2.89-2.82 (m, 2H), 2.79-2.66 (m, 2H), 1.33 (m, 6H).
LC-MS (Methode 7): Rₜ = 1.80 min; MS (ESIpos): m/z = 906 [M+H]⁺.

### Beispiel 11

### (4S,7S,10S,14S,17S)-4,17-Diamino-10-({4-[2-amino-6-({[2-(4-chlorphenyl)-1,3-oxazol-4-yl]-methyl}sulfanyl)-3,5-dicyanopyridin-4-yl]phenoxy}methyl)-7,14-dimethyl-5,8,13,16-tetraoxo-9,12-dioxa-6,15-diazaicosan-1,20-disäure-Bis(trifluoressigsäure)-Salz

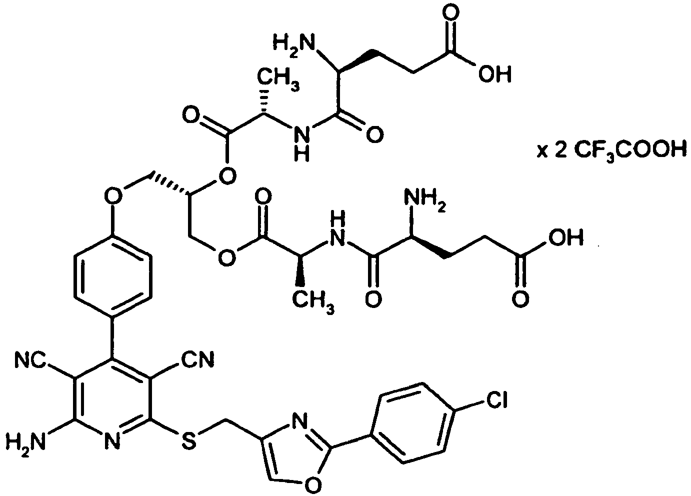

410 mg (0.336 mmol) der Verbindung aus Beispiel 25A wurden in 2 ml Dichlormethan vorgelegt, mit 0.222 ml (2.887 mmol) Trifluoressigsäure versetzt und über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 234 mg (68% d. Th.) der Zielverbindung erhalten.
¹H-NMR (400 MHz, DMSO-d₆): δ = 12.95-11.82 (m, 2H), 8.95-8.89 (m, 2H), 8.37 (s, 1H), 8.23-8.19 (m, 6H), 7.97 (d, 2H), 7.61 (d, 2H), 7.48 (d, 2H), 7.13 (d, 2H), 5.41 (m, 1H), 4.46-4.24 (m, 8H), 3.84 (br. s, 2H), 2.45-2.33 (m, 4H), 2.08-1.90 (m, 4H), 1.33 (m, 6H).
LC-MS (Methode 7): Rₜ = 1.59 min; MS (ESIpos): m/z = 934 [M+H]⁺.

### Beispiel 12

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2-amino-3-(1H-imidazol-4-yl)propanoyl]-amino}propanoat)-Bis(trifluoressigsäure)-Salz

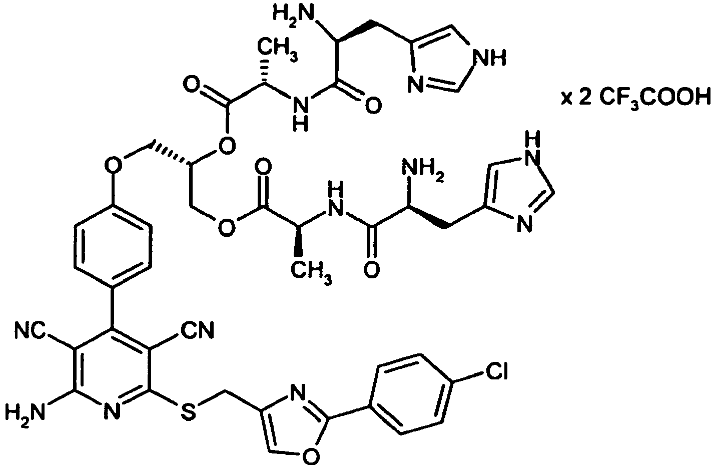

275 mg (0.239 mmol) der Verbindung aus Beispiel 26A wurden in 2 ml Dichlormethan vorgelegt, mit 0.184 ml (2.390 mmol) Trifluoressigsäure versetzt und 3 h bei RT gerührt. Anschließend wurde das Reaktionsgemisch eingeengt und der Rückstand mittels präparativer HPLC gereinigt (Laufmittel-Gradient: Acetonitril/Wasser 10:90 → 95:5 unter Zusatz von 0.1% TFA). Es wurden 190 mg (66% d. Th.) der Zielverbindung erhalten.
¹H-NMR (400 MHz, DMSO-d₆): δ = 14.61-13.61 (m, 2H), 9.01-8.90 (m, 4H), 8.81-8.04 (m, 4H), 8.37 (s, 1H), 8.01 (d, 2H), 7.61 (d, 2H), 7.56 (d, 2H), 7.43 (s, 2H), 7.11 (d, 2H), 5.38 (m, 1H), 4.46-4.33 (m, 6H), 4.28 (m, 2H), 4.15 (m, 2H), 3.34-3.05 (m, 4H), 1.34 (m, 6H).
LC-MS (Methode 7): Rₜ = 1.16 min; MS (ESIpos): m/z = 950 [M+H]⁺.

### Beispiel 13

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S',2'S)-bis(2-{[(methylamino)acetyl]amino}propanoat)-Bis(trifluoressigsäure)-Salz

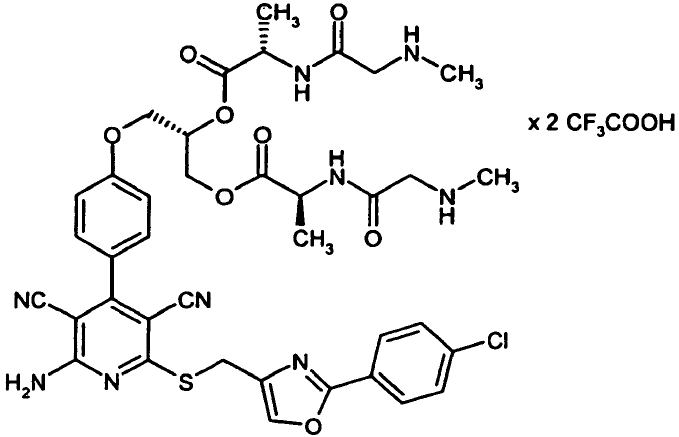

134 mg (0.132 mmol) der Verbindung aus Beispiel 21A wurden in 2 ml Dichlormethan vorgelegt, mit 0.101 ml (1.316 mmol) Trifluoressigsäure versetzt und 2 h bei RT gerührt. Anschließend wurde das Reaktionsgemisch eingeengt und der Rückstand mittels präparativer HPLC gereinigt (Laufmittel-Gradient: Acetonitril/Wasser 10:90 → 95:5 unter Zusatz von 0.1% TFA). Es wurden 103 mg (72% d. Th.) der Zielverbindung erhalten.
¹H-NMR (400 MHz, DMSO-d₆): δ = 8.95 (d, 2H), 8.89-8.69 (m, 4H), 8.37 (s, 1H), 8.26-8.02 (br. s, 2H), 7.97 (d, 2H), 7.61 (d, 2H), 7.50 (d, 2H), 7.13 (d, 2H), 5.40 (m, 1H), 4.42-4.24 (m, 8H), 3.75 (s, 6H), 2.56 (m, 4H), 1.33 (m, 6H).
LC-MS (Methode 5): Rₜ = 1.00 min; MS (ESIpos): m/z = 818 [M+H]⁺.

### Beispiel 14

### (2S,6S,9S,13S,17S)-2,17-Diamino-9-({4-[2-amino-6-({[2-(4-chlorphenyl)-1,3-oxazol-4-yl]methyl}-sulfanyl)-3,5-dicyanopyridin-4-yl]phenoxy}methyl)-6,13-dimethyl-4,7,12,15-tetraoxo-8,11-dioxa-5,14-diazaoctadecan-1,18-disäure-Bis(trifluoressigsäure)-Salz

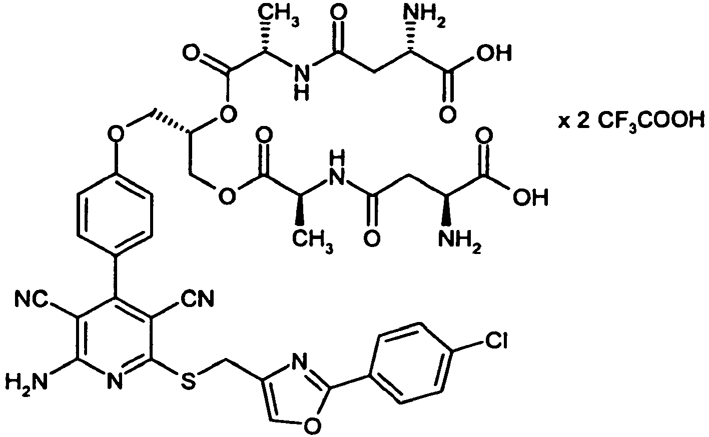

190 mg (0.156 mmol) der Verbindung aus Beispiel 22A wurden in 1 ml Dichlormethan vorgelegt, mit 0.120 ml (1.559 mmol) Trifluoressigsäure versetzt und über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 76 mg (43% d. Th.) der Zielverbindung erhalten.
¹H-NMR (400 MHz, DMSO-d₆): δ = 14.20-13.53 (m, 2H), 8.69 (d, 2H), 8.37 (s, 1H), 8.26-8.02 (m, 6H), 7.97 (d, 2H), 7.61 (d, 2H), 7.49 (d, 2H), 7.12 (d, 2H), 5.36 (m, 1H), 4.45-4.18 (m, 10H), 2.81-2.72 (m, 4H), 1.30 (m, 6H).
LC-MS (Methode 6): Rₜ = 1.00 min; MS (ESIpos): m/z = 906 [M+H]⁺.

### Beispiel 15

### (2S,7S,10S,14S,19S)-2,19-Diamino-10-({4-[2-amino-6-({[2-{4-chlorphenyl)-1,3-oxazol-4-yl]-methyl}sulfanyl)-3,5-dicyanopyridin-4-yl]phenoxy}methyl)-7,14-dimethyl-5,8,13,16-tetraoxo-9,12-dioxa-6,15-diazaicosan-1,20-disäure-Bis(trifluoressigsäure)-Salz

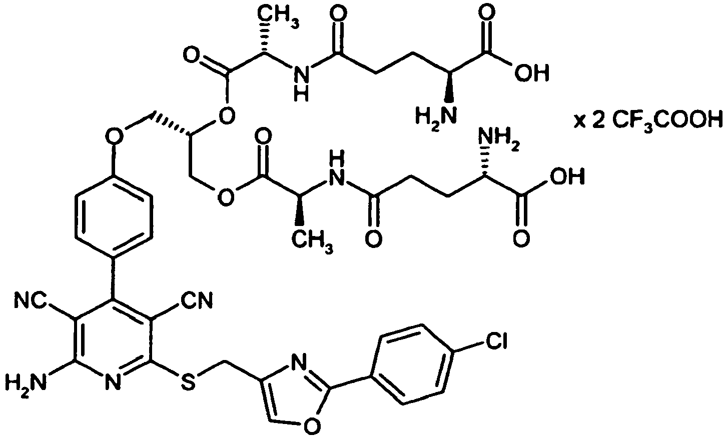

120 mg (0.096 mmol) der Verbindung aus Beispiel 27A wurden in 1 ml Dichlormethan vorgelegt, mit 0.074 ml (0962 mmol) Trifluoressigsäure versetzt und über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 20 mg (18% d. Th.) der Zielverbindung erhalten.
¹H-NMR (400 MHz, DMSO-d₆): δ = 14.11-13.62 (m, 2H), 8.44 (d, 2H), 8.37 (s, 1H), 8.35-8.06 (m, 8H), 7.97 (d, 2H), 7.61 (d, 2H), 7.49 (d, 2H), 7.12 (d, 2H), 5.35 (m, 1H), 4.39-4.20 (m, 8H), 3.93 (br. s, 2H), 2.38-2.24 (m, 4H), 2.06-1.90 (m, 4H), 1.28 (m, 6H).
LC-MS (Methode 7): Rₜ = 1.00 min; MS (ESIpos): m/z = 934 [M+H]⁺.

### Beispiel 16

### (2S)-3-{4-[2-Amino-6-({[2-(4-chlorphenyl)-1,3-xazol-4-yl]methyl}sulfanyl)-3,5-dicyanopyridin-4-yl]phenoxy}propan-1,2-diyl-(2S,2'S)-bis(2-{[(2S)-pyrrolidin-2-ylcarbonyl]amino}propanoat)-Dihydrochlorid

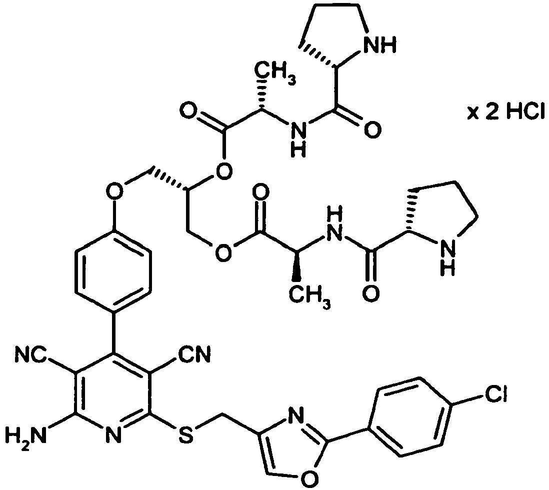

339 mg (0.317 mmol) der Verbindung aus Beispiel 20A wurden in 4 ml Dichlormethan vorgelegt und tropfenweise mit 3.166 ml (6.333 mmol) einer 2 M Lösung von Chlorwasserstoff in Diethylether versetzt. Anschließend wurde über Nacht bei RT gerührt. Der ausgefallene Feststoff wurde abfiltriert, mit Diethylether gewaschen und im Vakuum getrocknet. Es wurden 277 mg (91% d. Th.) der Zielverbindung erhalten.
¹H-NMR (400 MHz, DMSO-d₆): δ = 9.73 (m, 2H), 9.04 (m, 2H), 8.54 (m, 2H), 8.38 (s, 1H), 8.31-8.03 (br. s, 2H), 7.96 (d, 2H), 7.60 (d, 2H), 7.49 (d, 2H), 7.11 (d, 2H), 5.37 (m, 1H), 4.41-4.17 (m, 10H), 3.20-3.16 (m, 4H), 2.29 (m, 2H), 1.86-1.80 (m, 6H), 1.34 (m, 6H).
LC-MS (Methode 5): Rₜ = 0.96 min; MS (ESIpos): m/z = 870 [M+H]⁺.

### Beispiel 17

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2-aminopropanoyl]amino}propanoat)-Bis(4-methylbenzolsulfonsäure)-Salz

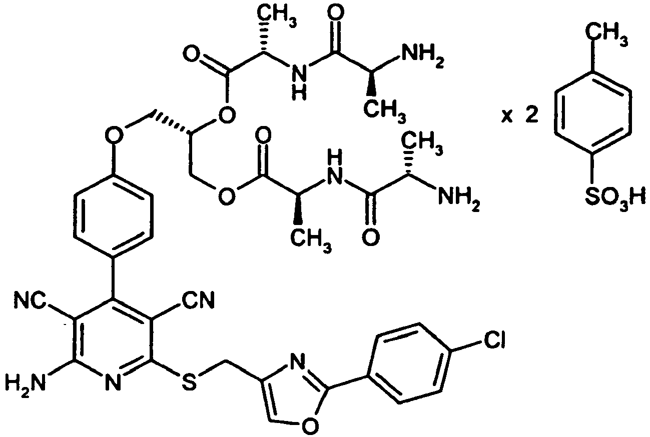

220 mg (0.216 mmol) der Verbindung aus Beispiel 48A wurden in 20 ml Dichlormethan vorgelegt, mit 82 mg (0.475 mmol) 4-Methylbenzolsulfonsäure versetzt und 24 h bei 60°C gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% 4-Methylbenzolsulfonsäure). Es wurden 158 mg (57% d. Th.) der Zielverbindung erhalten.
¹H-NMR (400 MHz, DMSO-d₆): δ = 8.79-8.75 (m, 2H), 8.37 (s, 1H), 8.31-8.01 (m, 8H), 7.97 (d, 2H), 7.61 (d, 2H), 7.53-7.43 (m, 6H), 7.11 (d, 6H), 5.40-5.32 (m, 1H), 4.45-4.34 (m, 4H), 4.32-3.98 (m, 4H), 3.89-3.77 (m, 2H), 2.29 (s, 6H), 1.37-1.29 (m, 12H).
LC-MS (Methode 6): Rₜ = 1.25 min; MS (ESIpos): m/z = 818 [M+H]⁺.

### Beispiel 18

### (2S)-3-{4-[2-Amino-6-({[2-(4-chlorphenyl)-1,3-oxazol-4-yl]methyl}sulfanyl}-3,5-dicyanopyridin-4-yl]phenoxy}propan-1,2-diyl-bis(3-{[(2S)-2,6-diaminohexanoyl]amino}propanoat)-Tetrakis-(trifluoressigsäure)-Salz

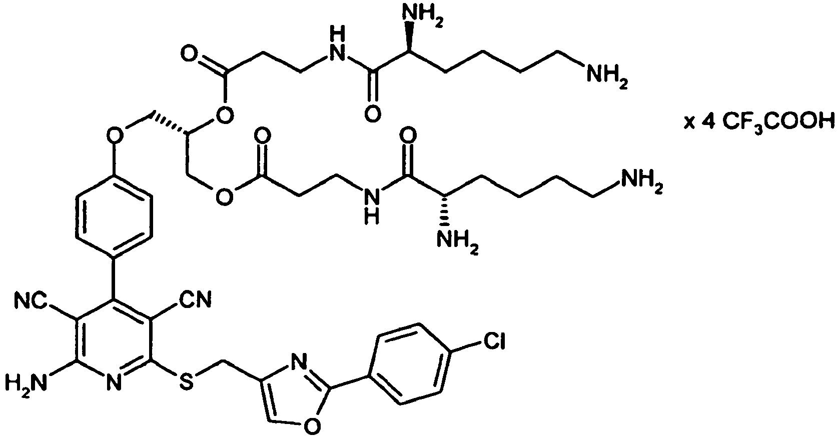

70 mg (0.058 mmol) der Verbindung aus Beispiel 31A wurden in 0.5 ml Dichlormethan vorgelegt, mit 0.045 ml (0.578 mmol) Trifluoressigsäure versetzt und bei RT gerührt. Nach 3 h wurde nochmals die gleiche Menge an Trifluoressigsäure hinzugefügt und die Mischung über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 45 mg (55% d. Th.) der Zielverbindung erhalten.
¹H-NMR (400 MHz, DMSO-d₆): δ = 8.61-8.57 (m, 2H), 8.37 (s, 1H), 8.33-8.01 (m, 8H), 7.98 (d, 2H), 7.86-7.37 (m, 6H), 7.61 (d, 2H), 7.50 (d, 2H), 7.12 (d, 2H), 5.36 (m, 1H), 4.42 (s, 2H), 4.41-4.24 (m, 4H), 3.67 (m, 2H), 3.38 (m, 4H), 2.75 (m, 4H), 2.61-2.57 (m, 4H), 1.67 (m, 4H), 1.51 (m, 4H), 1.29 (m, 4H).
LC-MS (Methode 6): Rₜ = 0.90 min; MS (ESIpos): m/z = 932 [M+H]⁺.

### Beispiel 19

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-bis{3-[(aminoacetyl)amino]propanoat}-Bis(trifluoressigsäure)-Salz

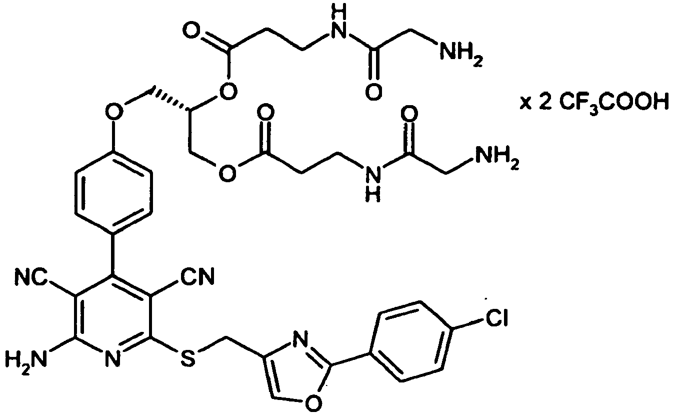

79 mg (0.080 mmol) der Verbindung aus Beispiel 32A wurden in 0.5 ml Dichlormethan vorgelegt, mit 0.061 ml (0.798 mmol) Trifluoressigsäure versetzt und bei RT gerührt. Nach 3 h wurde nochmals die gleiche Menge an Trifluoressigsäure hinzugefügt und die Mischung über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 54 mg (65% d. Th.) der Zielverbindung erhalten.
¹H-NMR (400 MHz, DMSO-d₆): δ = 8.44 (m, 2H), 8.37 (s, 1H), 8.32-7.66 (br. m, 8H), 7.97 (d, 2H), 7.61 (d, 2H), 7.49 (d, 2H), 7.13 (d, 2H), 5.37 (m, 1H), 4.42 (s, 2H), 4.41-4.24 (m, 4H), 3.52 (m, 4H), 3.38 (m, 4H), 2.58-2.53 (m, 4H).
LC-MS (Methode 6): Rₜ = 1.19 min; MS (ESIpos): m/z = 790 [M+H]⁺.

### Beispiel 20

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-bis(3-{[(2S)2-amino-4-methylpentanoyl]amino}propanoat)-Bis(trifluoressigsäure)-Salz

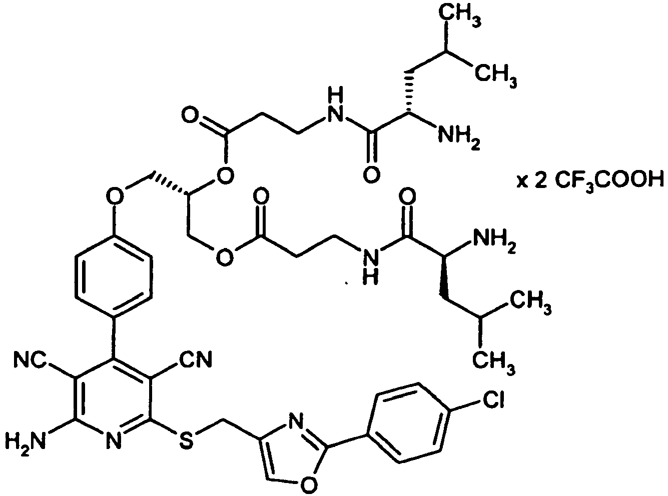

85 mg (0.077 mmol) der Verbindung aus Beispiel 33A wurden in 0.5 ml Dichlormethan vorgelegt, mit 0.059 ml (0.771 mmol) Trifluoressigsäure versetzt und über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 47 mg (51% d. Th.) der Zielverbindung erhalten.
¹H-NMR (400 MHz, DMSO-d₆): δ = 8.60 (m, 2H), 8.37 (s, 1H), 8.18-8.04 (m, 6H), 7.97 (d, 2H), 7.61 (d, 2H), 7.49 (d, 2H), 7.12 (d, 2H), 5.35 (m, 1H), 4.42 (s, 2H), 4.41-4.24 (m, 4H), 3.67 (m, 2H), 3.45 (m, 2H), 3.29 (m, 2H), 2.59-2.54 (m, 4H), 1.64-1.45 (m, 6H), 0.89-0.84 (m, 12H).
LC-MS (Methode 6): Rₜ = 1.31 min; MS (ESIpos): m/z = 902 [M+H]⁺.

### Bespiel 21

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-bis(3-{[(2S)-2-amino-3-methylbutanoyl]amino}propanoat)-Bis(trifluoressigsäure)-Salz

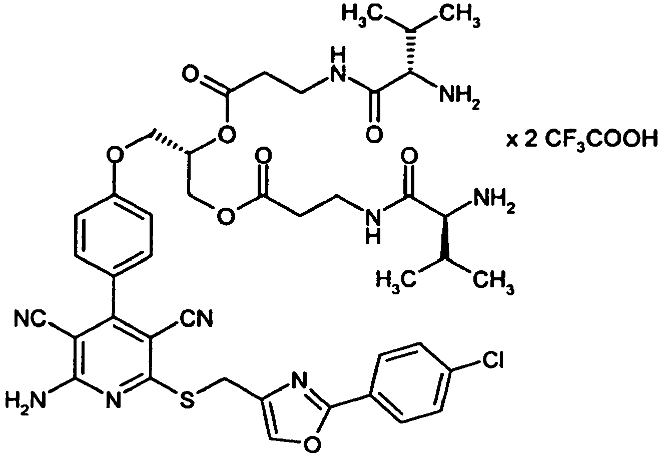

74 mg (0.070 mmol) der Verbindung aus Beispiel 34A wurden in 0.5 ml Dichlormethan vorgelegt, mit 0.054 ml (0.698 mmol) Trifluoressigsäure versetzt und über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 20 mg (26% d. Th.) der Zielverbindung erhalten.
¹H-NMR (400 MHz, DMSO-d₆): δ = 8.51 (dd, 2H), 8.36 (s, 1H), 8.33-8.01 (m, 6H), 7.97 (d, 2H), 7.61 (d, 2H), 7.49 (d, 2H), 7.11 (d, 2H), 5.35 (m, 1H), 4.42 (s, 2H), 4.39-4.23 (m, 4H), 3.50-3.39 (m, 4H), 3.26 (m, 2H), 2.59-2.54 (m, 4H), 1.99 (m, 2H), 0.90-0.84 (m, 12H).
LC-MS (Methode 6): Rₜ = 1.31 min; MS (ESIpos): m/z = 874 [M+H]⁺.

### Beispiel 22

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis{2-[(3-aminopropanoyl)amino]-4-methylpentanoat}-Bis(trifluoressigsäure)-Salz

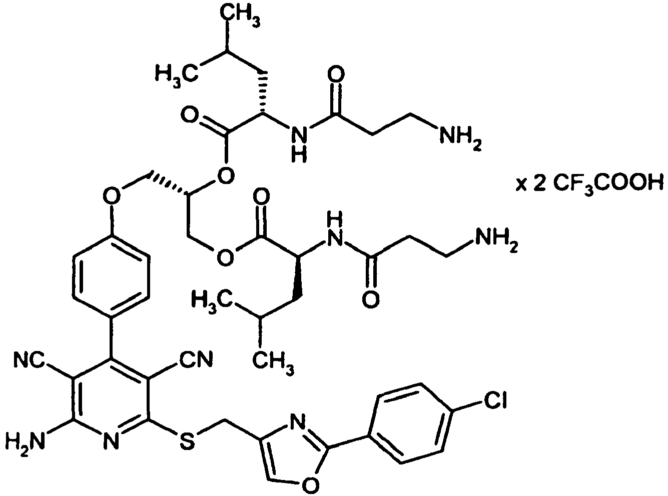

240 mg (0.118 mmol) der Verbindung aus Beispiel 37A wurden in 1 ml Dichlormethan vorgelegt, mit 0.091 ml (1.175 mmol) Trifluoressigsäure versetzt und bei RT gerührt. Nach 3 h wurde nochmals die gleiche Menge an Trifluoressigsäure hinzugefügt und die Mischung über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 119 mg (88% d. Th.) der Zielverbindung erhalten.
¹H-NMR (400 MHz, DMSO-d₆): 5 = 8.53 (m, 2H), 8.37 (s, 1H), 8.26-8.03 (m, 2H), 7.97 (d, 2H), 7.70 (br. m, 4H), 7.61 (d, 2H), 7.50 (d, 2H), 7.11 (d, 2H), 5.35 (m, 1H), 4.42 (s, 2H), 4.39-4.20 (m, 6H), 4.00-3.50 (m, 4H), 2.97 (m, 4H), 1.71-1.35 (m, 6H), 0.95-0.83 (m, 12H).
LC-MS (Methode 7): Rₜ = 1.67 min; MS (ESIpos): m/z = 902 [M+H]⁺.

### Beispiel 23

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis{2-[(3-aminopropanoyl)amino]-3-methylbutanoat}-Bis(trifluoressigsäure)-Salz

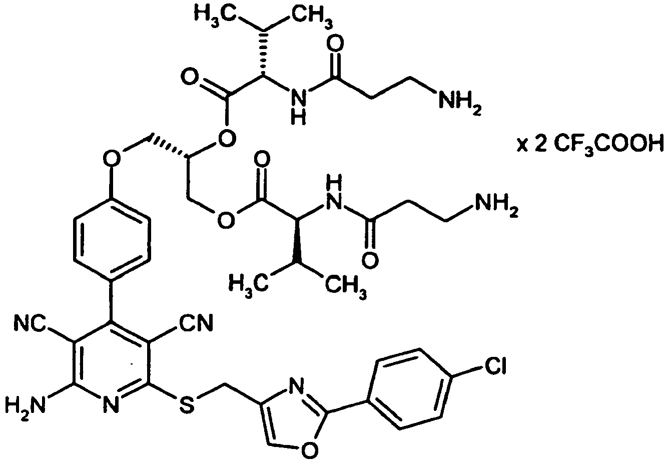

216 mg (0.201 mmol) der Verbindung aus Beispiel 40A wurden in 1 ml Dichlormethan vorgelegt, mit 0.155 ml (2.010 mmol) Trifluoressigsäure versetzt und bei RT gerührt. Nach 3 h wurde nochmals die gleiche Menge an Trifluoressigsäure hinzugefügt und die Mischung über Nacht bei RT gerührt. Das Reaktionsgemisch wurde danach eingeengt und der Rückstand mittels präparativer HPLC gereinigt (Laufmittel-Gradient: Acetonitril/Wasser 10:90 → 95:5 unter Zusatz von 0.1% TFA). Es wurden 92 mg (42% d. Th.) der Zielverbindung erhalten.
¹H-NMR (400 MHz, DMSO-d₆): δ = 8.44 (br. d, 2H), 8.37 (s, 1H), 8.34-8.07 (m, 2H), 7.97 (d, 2H), 7.86-7.66 (m, 6H), 7.61 (d, 2H), 7.50 (d, 2H), 7.12 (d, 2H), 5.40-5.35 (m, 1H), 4.42 (s, 2H), 4.41-4.23 (m, 6H), 3.05-2.96 (m, 4H), 2.61-2.56 (m, 4H), 2.13-2.00 (m, 2H), 0.93-0.86 (m, 12H).
LC-MS (Methode 5): Rₜ = 1.04 min; MS (ESIpos): m/z = 874 [M+H]⁺.

### Beispiel 24

### (2S)-3-{4-[2-Amino-6-({[2-(4-chlorphenyl)-1,3-oxazol-4-yl]methyl}sulfanyl)~3,5-dicyanopyridin-4-yl]phenoxy}propan-1,2-diyl-(2S,2'S)-bis(2-{[(2R)-2-aminopropanoyl]amino}propanoat)-Dihydrochlorid

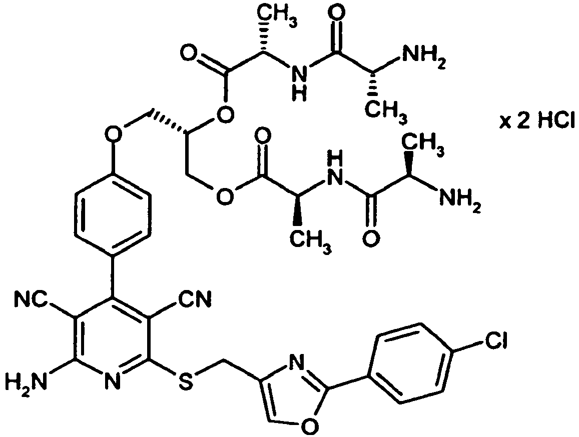

315 mg (0.309 mmol) der Verbindung aus Beispiel 41A wurden in 5 ml Dichlormethan vorgelegt und unter Rühren tropfenweise mit 6.185 ml (6.185 mmol) einer 1 M Lösung von Chlorwasserstoff in Diethylether versetzt. Anschließend wurde über Nacht bei RT gerührt. Der ausgefallene Feststoff wurde abfiltriert, zunächst mit Dichlormethan, dann mit Diethylether gewaschen und schließlich im Vakuum getrocknet. Es wurden 256 mg (93% d. Th.) der Zielverbindung erhalten.
¹H-NMR (400 MHz, DMSO-d₆): δ = 8.95 (m, 2H), 8.38 (s, 1H), 8.20 (br. m, 8H), 7.97 (d, 2H), 7.61 (d, 2H), 7.50 (d, 2H), 7.13 (d, 2H), 5.39 (m, 1H), 4.42 (s, 2H), 4.41-4.24 (m, 6H), 3.87 (m, 2H), 1.39-1.30 (m, 12H).
LC-MS (Methode 8): Rₜ = 0.85 min; MS (ESIpos): m/z = 818 [M+H]⁺.

### Beispiel 25

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2R,2'R)-bis(2-{[(2R)-2-aminopropanoyl]amino}propanoat)-Dihydrochlorid

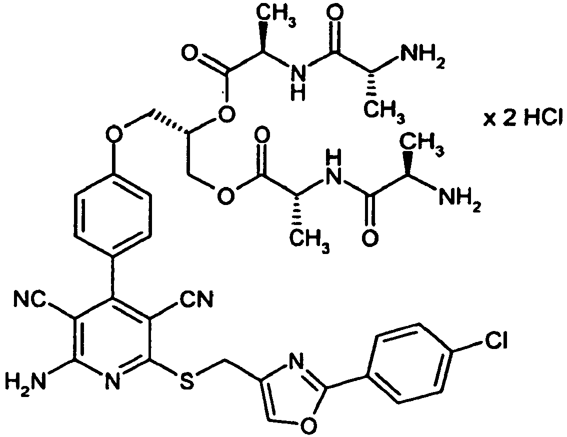

435 mg (0.427 mmol) der Verbindung aus Beispiel 44A wurden in 8 ml Dichlormethan vorgelegt und unter Rühren tropfenweise mit 8.542 ml (8.542 mmol) einer 1 M Lösung von Chlorwasserstoff in Diethylether versetzt. Anschließend wurde 6 h bei RT gerührt. Der ausgefallene Feststoff wurde abfiltriert, zunächst mit Dichlormethan, dann mit Diethylether gewaschen und schließlich im Vakuum getrocknet. Es wurden 348 mg (92% d. Th.) der Zielverbindung erhalten.
¹H-NMR (400 MHz, DMSO-d₆): δ = 8.95 (d, 2H), 8.38 (s, 1H), 8.34-8.02 (br. m, 8H), 7.97 (d, 2H), 7.61 (d, 2H), 7.49 (d, 2H), 7.11 (d, 2H), 5.38 (m, 1H), 4.47-4.22 (m, 8H), 3.86 (m, 2H), 1.39-1.30 (m, 12H).
LC-MS (Methode 8): R, = 0.88 min; MS (ESIpos): m/z = 818 [M+H]⁺.

### Beispiel 26

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2-amino-3-methylbutanoyl]amino}propanoat)-Dihydrochlorid

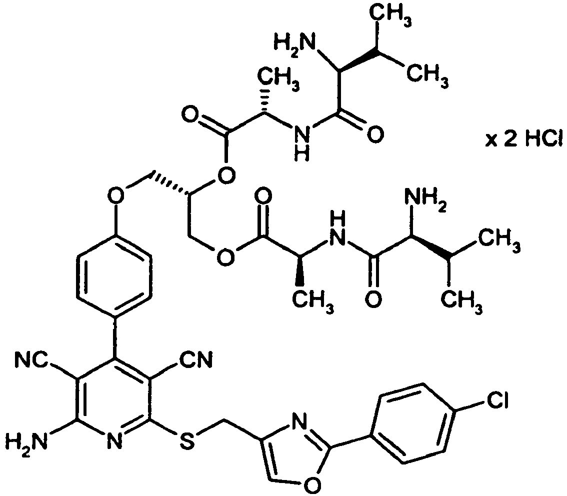

240 mg (0.223 mmol) der Verbindung aus Beispiel 45A wurden in 250 ml Dichlormethan gelöst. In diese Lösung wurde Chlorwasserstoff-Gas eingeleitet. Nach einstündigem Rühren bei RT wurde die Lösung im Vakuum auf ein Volumen von ca. 50 ml aufkonzentriert und mit 200 ml Ethylacetat versetzt. Der ausgefallene Feststoff wurde abgesaugt, mit Ethylacetat gewaschen und im Hochvakuum getrocknet. Es wurden 175 mg (83% d. Th.) der Zielverbindung erhalten..
¹H-NMR (400 MHz, DMSO-d₆): δ = 8.95 (d, 2H), 8.4 (s, 1H), 8.3-8.1 (m, 6H), 7.97 (d, 2H), 7.61 (d, 2H), 7.49 (d, 2H), 7.10 (d, 2H), 5.40 (m, 1H), 4.4-4.2 (m, 8H), 3.64 (m, 2H), 2.1 (m, 2H), 1.4-1.3 (2d, 6H), 0.95 (d, 12H).
LC-MS (Methode 6): R, = 1.22 min; MS (ESIpos): m/z = 874 [M+H]⁺.

### Beispiel 27

### (2S)-3-{4-[2-Amino-6-({[2-(4-chlorphenyl)-1,3-oxazot-4-yl]methyl}sulfanyl)-3,5-dicyanopyridin-4-yl]phenoxy}propan-1,2-diyl-(2S,2'S)-bis{2-[(aminoacetyl)amino]-3-methylbutanoat}-Dihydrochlorid

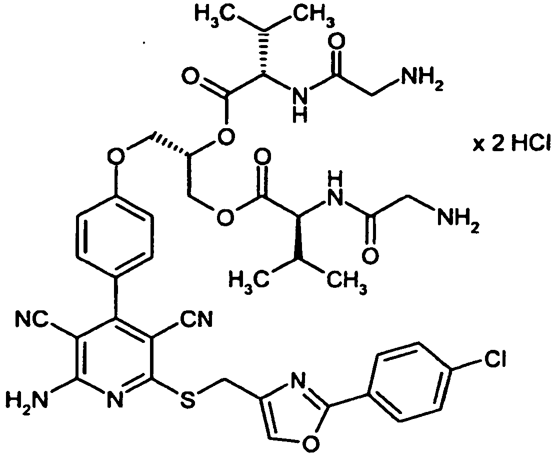

55 mg (0.312 mmol) *N*-(*tert*.-Butoxycarbonyl)glycin wurden in 2 ml DMF vorgelegt und mit 57.5 mg (0.3 mmol) 1-(3-Dimethylaminopropyl)-3-ethylcarbodiimid-Hydrochlorid sowie 51 mg (0.375 mmol) 1-Hydroxy-1*H*-benzotriazol-Hydrat versetzt. Nach 30 min Rühren bei RT wurden 120 mg (0.125 mmol) der Verbindung aus Beispiel 39A sowie 0.044 ml (0.25 mmol) *N,N*-Diisopropylethylamin hinzugegeben. Anschließend wurde das Gemisch über Nacht bei RT gerührt. Danach wurde der Ansatz im Vakuum eingeengt und der Rückstand in 50 ml Dichlormethan aufgenommen. Es wurde jeweils viermal mit 10 ml 0.5 M Zitronensäure-Lösung und 10 ml 10%-iger Natriumhydrogencarbonat-Lösung gewaschen und die organische Phase über Magnesiumsulfat getrocknet und anschließend eingeengt. Der Rückstand wurde durch präparative HPLC gereinigt. Nach Trocknen der Produktfraktion im Hochvakuum verblieben 90 mg (69% d. Th.) des Bocgeschützten Intermediats, das sofort weiter umgesetzt wurde.

90 mg (0.086 mmol) des erhaltenen Intermediats wurden in 50 ml Dichlormethan aufgenommen, und in diese Lösung wurde Chlorwasserstoff-Gas eingeleitet. Nach einstündigem Rühren bei RT wurde die Lösung im Vakuum auf ein Volumen von ca. 25 ml aufkonzentriert und mit Diethylether versetzt. Der ausgefallene Feststoff wurde abgesaugt, mit Dichlormethan und Diethylether gewaschen und im Hochvakuum getrocknet. Es wurden 51 mg (65% d. Th.) der Zielverbindung erhalten.
¹H-NMR (400 MHz, DMSO-d₆): δ = 8.75 (d, 2H), 8.39 (s, 1H), 8.25-8.10 (m, 6H), 7.97 (d, 2H), 7.61 (d, 2H), 7.49 (d, 2H), 7.12 (d, 2H), 5.40-5.35 (m, 1H), 4.40-4.25 (m, 8H), 3.75-3.55 (m, 4H), 2.15-2.05 (m, 2H), 0.93 (d, 6H), 0.88 (d, 3H), 0.86 (d, 3H).
LC-MS (Methode 10): Rₜ = 1.86 min; MS (ESIpos): m/z = 846 [M+H]⁺.

### Beispiel 28

### (2S)-3-{4-[2-Amino-6-({[2-(4-chlorphenyl)-1,3-oxazol-4-yl]methyl}-sulfanyl)-3,5-dicyanopyridin-4-yl]phenoxy)propan-1,2-diyl-(2S,2'S)-bis(2-{[(2S)-2-aminopropanoyl]amino}-3-methylbutanoat)-Dihydrochlorid

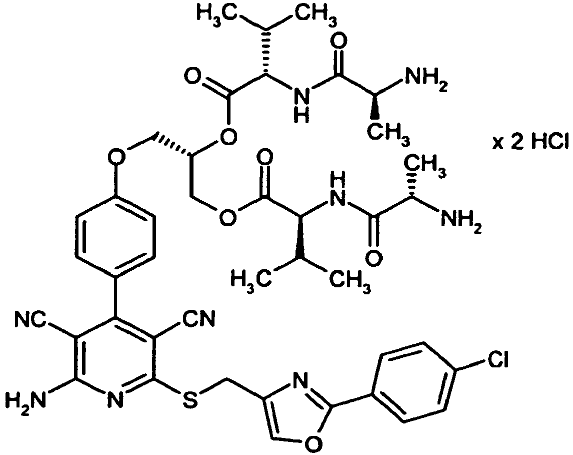

Die Herstellung der Titelverbindung erfolgte analog zur Herstellung von Beispiel 27 ausgehend von der Verbindung aus Beispiel 39A und käuflichem *N*-(*tert*.-Butoxycarbonyl)-L-alanin.
Ausbeute: 37% d. Th.
¹H-NMR (400 MHz, DMSO-d₆): δ = 8.7 (d, 2H), 8.4 (s, 1H), 8.30-8.15 (m, 6H), 7.97 (d, 2H), 7.63 (d, 2H), 7.5 (d, 2H), 7.1 (d, 2H), 5.45-5.35 (m, 1H), 4.45-4.20 (m, 8H), 4.05-3.90 (m, 2H), 2.15-2.05 (m, 2H), 1.35 (d, 6H), 0.94 (d, 6H), 0.91 (d, 3H), 0.89 (d, 3H).
LC-MS (Methode 7): Rₜ = 1.56 min; MS (ESIpos): m/z = 874 [M+H]⁺.

### Beispiel 29

### (2S)-3-{4-[2-Amino-6-({[2-(4-chlorphenyl)-1,3-oxazol-4-yl]methyl}sulfanyl)3,5-dicyanopyridin-4-yl]phenoxy}propan-1,2-diyl-(2S,2'S)-bis(2-{[aminoacetyl]amino}-4-methylpentanoat)-Dihydrochlorid

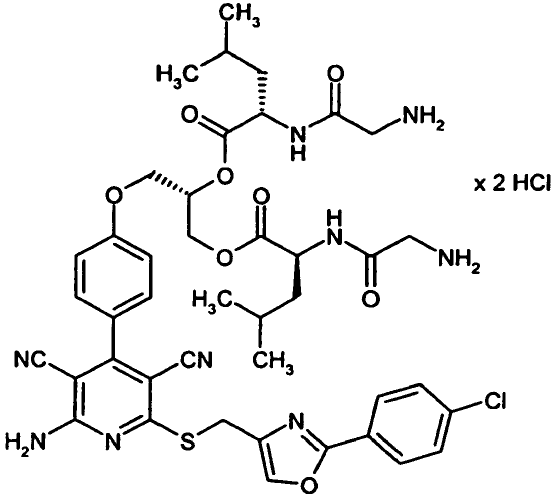

Die Herstellung der Titelverbindung erfolgte analog zur Herstellung von Beispiel 27 ausgehend von der Verbindung aus Beispiel 36A und käuflichem *N*-(*tert*.-Butoxycarbonyl)glycin.
Ausbeute: 54% d. Th.
¹H-NMR (400 MHz, DMSO-d₆): δ = 8.9 (m, 2H), 8.39 (s, 1H), 8.2-8.1 (m, 6H), 7.97 (d, 2H), 7.61 (d, 2H), 7.50 (d, 2H), 7.13 (d, 2H), 5.40-5.35 (m, 1H), 4.45-4.20 (m, 8H), 3.70-3.55 (m, 4H), 1.75-1.45 (m, 6H), 0.92 (d, 3H), 0.90 (d, 3H), 0.87 (d, 3H), 0.85 (d, 3H).
LC-MS (Methode 7): Rₜ = 1.64 min; MS (ESIpos): m/z = 874 [M+H]⁺.

### Beispiel 30

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2-aminopropanoyl]amino}-4-methylpentanoat)-Dihydrochlorid

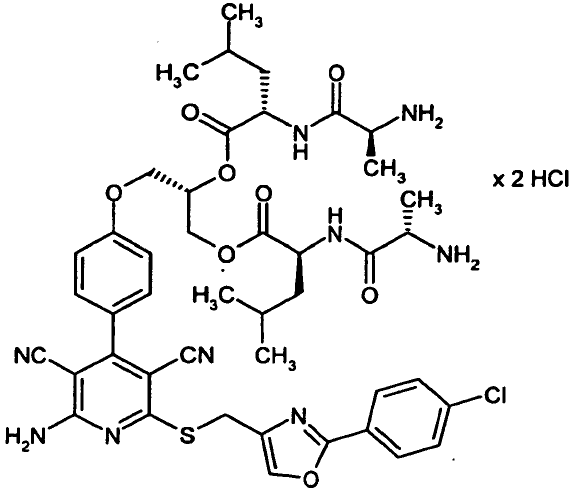

Die Herstellung der Titelverbindung erfolgte analog zur Herstellung von Beispiel 27 ausgehend von der Verbindung aus Beispiel 36A und käuflichem *N*-(*tert*.-Butoxycarbonyl)-L-alanin.
Ausbeute: 54% d. Th.
¹H-NMR (400 MHz, DMSO-d₆): δ = 8.9 (m, 2H), 8.39 (s, 1H), 8.30-8.15 (m, 4H), 7.97 (d, 2H), 7.6 (d, 2H), 7.50 (d, 2H), 7.1 (d, 2H), 5.40-5.30 (m, 1H), 4.45-4.20 (m, 8H), 3.9 (br. m, 2H), 1.75-1.50 (m, 6H), 1.37 (d, 6H), 0.92 (d, 3H), 0.90 (d, 3H), 0.87 (d, 3H), 0.06 (d, 3H).
LC-MS (Methode 6): Rₜ = 1.37 min; MS (ESIpos): m/z = 902 [M+H]⁺.

### Beispiel 31

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-bis(3-{[(2S)-2-aminopropanoyl]amino}propanoat)-Dihydrochlorid

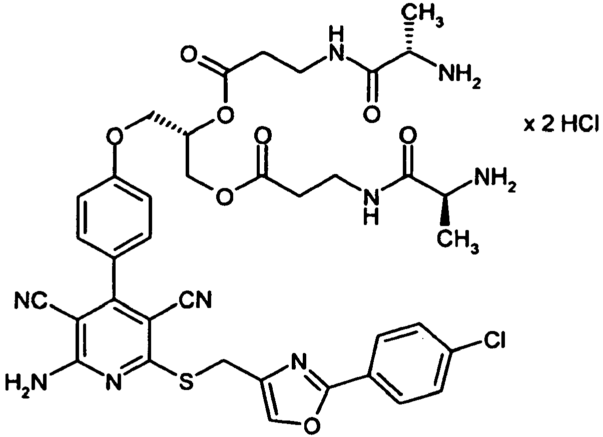

200 mg (0.196 mmol) der Verbindung aus Beispiel 46A wurden in 60 ml Dichlormethan aufgenommen, und in diese Lösung wurde Chlorwasserstoff-Gas eingeleitet. Nach einstündigem Rühren bei RT wurde die Lösung im Vakuum auf ein Volumen von ca. 30 ml aufkonzentriert und mit 50 ml Ethylacetat versetzt. Der ausgefallene Feststoff wurde abgesaugt, mit Ethylacetat gewaschen und 5 h lang im Hochvakuum bei 100°C getrocknet. Es wurden 151 mg (86% d. Th.) der Zielverbindung erhalten.
LC-MS (Methode 6): Rₜ = 1.16 min; MS (ESIpos): m/z = 818 [M+H]⁺.

### Beispiel 32

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2',S)-bis{2-[(3-aminopropanoyl)amino]propanoat}-Dihydrochlorid

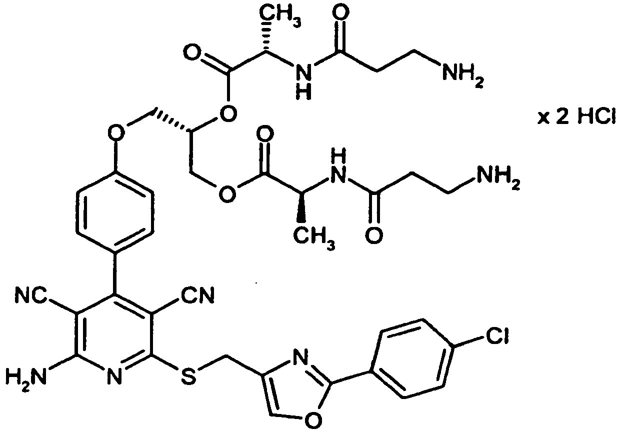

3740 mg (3.672 mmol) der Verbindung aus Beispiel 47A wurden in 800 ml Dichlormethan aufgenommen, und in diese Lösung wurde Chlorwasserstoff-Gas eingeleitet. Nach einstündigem Rühren bei RT wurde die Lösung im Vakuum auf ein Volumen von ca. 100 ml aufkonzentriert, mit 100 ml Ethylacetat versetzt und 10 min bei RT gerührt. Der ausgefallene Feststoff wurde abgesaugt, dreimal mit Diethylether gewaschen und anschließend 20 h lang im Hochvakuum bei 90°C getrocknet. Es wurden 3290 mg (quant.) der Zielverbindung erhalten.
¹H-NMR (400 MHz, DMSO-d₆): δ = 8.64 (d, 2H), 8.4 (s, 1H), 8.05-7.90 (m, 8H), 7.63 (d, 2H), 7.50 (d, 2H), 7.13 (d, 2H), 5.40-5.30 (m, 1H), 4.43 (s, 2H), 4.4-4.2 (m, 6H), 3.0-2.9 (m, 4H), 2.55 (m, 4H), 1.3 (t, 6H).
LC-MS (Methode 6): Rₜ = 1.15 min; MS (ESIpos): m/z = 818 [M+H]⁺.

### Beispiel 33

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2-aminopropanoyl]amino}propanoat)-Dihydrochlorid

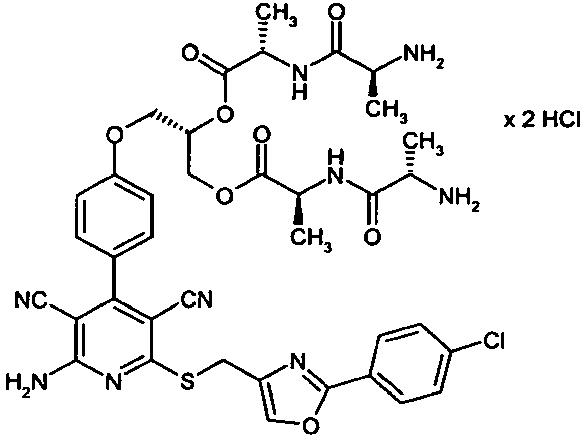

2484 mg (2.439 mmol) der Verbindung aus Beispiel 48A wurden in 300 ml Dichlormethan aufgenommen, und in diese Lösung wurde Chlorwasserstoff-Gas eingeleitet. Nach einstündigem Rühren bei RT wurde die Lösung im Vakuum auf ein Volumen von ca. 250 ml aufkonzentriert, mit 250 ml Ethylacetat versetzt und 10 min bei RT gerührt. Der ausgefallene Feststoff wurde abgesaugt, dreimal mit Ethylacetat gewaschen und anschließend 18 h lang im Hochvakuum bei 100°C getrocknet. Es wurden 2110 mg (97% d. Th.) der Zielverbindung erhalten.
¹H-NMR (400 MHz, DMSO-d₆): δ = 8.95 (m, 2H), 8.4 (s, 1H), 8.25-8.15 (m, 6H), 7.97 (d, 2H), 7.6 (d, 2H), 7.50 (d, 2H), 7.13 (d, 2H), 5.40-5.30 (m, 1H), 4.42 (s, 2H), 4.4-4.2 (m, 6H), 4.3-4.2 (m, 2H), 1.4-1.3 (m, 12H).
LC-MS (Methode 6): Rₜ = 1.18 min; MS (ESIpos): m/z = 818 [M+H]⁺.

### Beispiel 34

### (2R)-3-{4-[2-Amino-6-({[2-(4-chlorphenyl)-1,3-oxazol-4-yl]methyl}sulfanyl}-3,5-dicyanopyridin-4-yl]phenoxy}propan-1,2-diyl-(2S,2'S)-bis(2-{[(2S)-2,6-diaminohexanoyl]amino}propanoat)-Tetrahydrochlorid

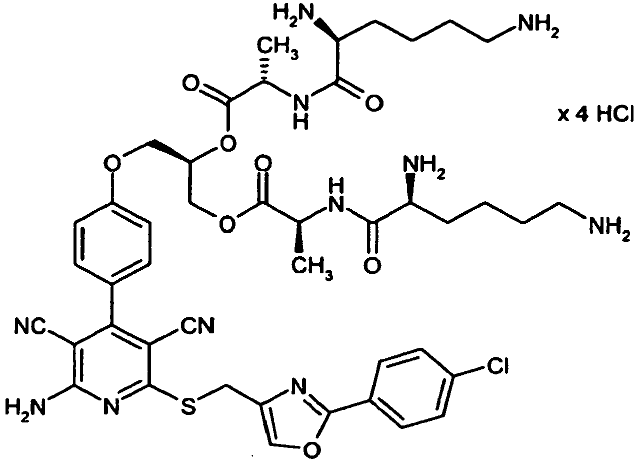

297 mg (0.22 mmol) der Verbindung aus Beispiel 51A wurden in 2.1 ml Dichlormethan vorgelegt und tropfenweise mit 2.2 ml (4.45 mmol) einer 2 M Lösung von Chlorwasserstoff in Diethylether versetzt. Es wurde vier Stunden bei Raumtemperatur gerührt und die Mischung dann im Vakuum eingeengt. Der Rückstand wurde mit 5 ml Acetonitril versetzt und das Gemisch anschließend erneut eingeengt. Diese Prozedur wurde noch einmal wiederholt. Nach Trocknen des Rückstands im Vakuum wurden 230 mg (96% d. Th.) der Zielverbindung erhalten.
¹H-NMR (400 MHz, DMSO-d₆): δ = 9.13 (dd, 2H), 8.41-8.26 (m, 5H), 8.12-7.89 (m, 6H), 7.62 (d, 2H), 7.49 (d, 2H), 7.12 (d, 2H), 5.42-5.36 (m, 1H), 4.49-4.22 (m, 8H), 3.89-3.78 (m, 2H), 2.81-2.69 (m, 4H), 1.82-1.71 (m,4H), 1.65-1.53 (m, 4H), 1.49-1.30 (m, 10H).
LC-MS (Methode 6): Rₜ = 0.91 min; MS (ESIpos): m/z = 932 [M+H]⁺.

### Beispiel 35

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,2'S)-bis(2-{[(2S)-2,6-diaminohexanoyl]amino}propanoat)-Tetrahydrochlorid

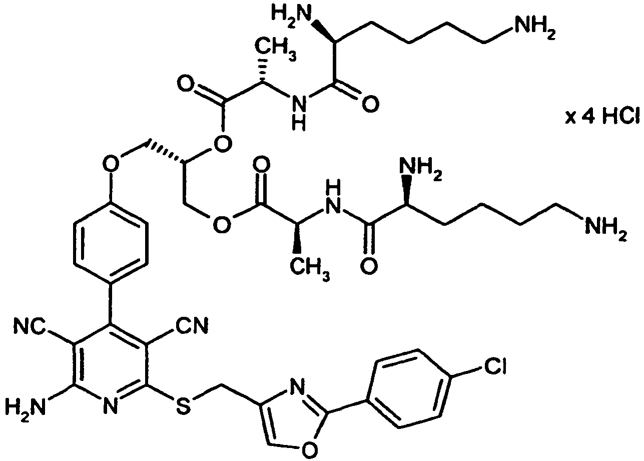

325 mg (0.24 mmol) der Verbindung aus Beispiel 52A wurden in 2.3 ml Dichlormethan vorgelegt und tropfenweise mit 2.4 ml (4.89 mmol) einer 2 M Lösung von Chlorwasserstoff in Diethylether versetzt. Es wurde vier Stunden bei Raumtemperatur gerührt und die Mischung dann im Vakuum eingeengt. Der Rückstand wurde mit 5 ml Acetonitril versetzt und das Gemisch anschließend erneut eingeengt. Diese Prozedur wurde noch einmal wiederholt. Nach Trocknen des Rückstands im Vakuum wurden 250 mg (95% d. Th.) der Zielverbindung erhalten.
¹H-NMR (400 MHz, DMSO-d₆): δ = 9.13 (t, 2H), 8.41-8.26 (m, 5H), 8.12-7.89 (m, 6H), 7.62 (d, 2H), 7.49 (d, 2H), 7.12 (d, 2H), 5.42-5.36 (m, 1H), 4.49-4.22 (m, 8H), 3.89-3.78 (m, 2H), 2.81-2.69 (m, 4H), 1.82-1.71 (m, 4H), 1.65-1.53 (m, 4H), 1.49-1.30 (m, 10H).
LC-MS (Methode 5): Rₜ = 0.79 min; MS (ESIpos): m/z = 932 [M+H]⁺.

### Beispiel 36

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,3R,2'S,3'R)-bis{2-[(aminoacetyl)amino]-3-hydroxybutanoat}-Bis(trifluoressigsäure)-Salz

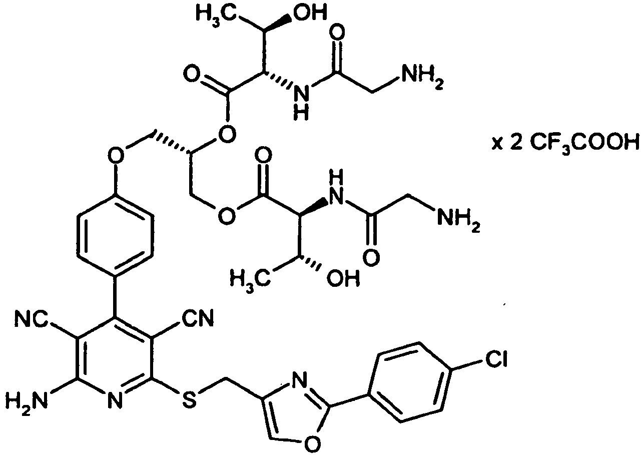

92 mg (0.09 mmol) der Verbindung aus Beispiel 55A wurden in 2.0 ml Dichlormethan vorgelegt und tropfenweise mit 0.27 ml (3.50 mmol) Trifluoressigsäure versetzt. Es wurde über Nacht bei Raumtemperatur gerührt und die Mischung dann im Vakuum eingeengt. Der Rückstand wurde mittels präparativer HPLC gereinigt (Eluent: Acetonitril/Wasser + 0.1% TFA). Es wurden 67 mg (70% d. Th.) der Zielverbindung erhalten.
¹H-NMR (400 MHz, DMSO-d₆): δ = 8.68 (d, 2H), 8.38 (s, 1H), 8.30-7.91 (m, 8H), 7.62 (d, 2H), 7.49 (d, 2H), 7.11 (d, 2H), 5.39 (quint, 1H), 5.32-5.12 (br. s, 1H), 4.50-4.15 (m, 11H), 3.78-3.58 (m,4H), 1.11 (dd, 6H).
LC-MS (Methode 5): Rₜ = 0.96 min; MS (ESIpos): m/z = 850 [M+H]⁺.

### Beispiel 37

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,3R,2'S,3'R)-bis{2-[(aminoacetyl)amino]-3-hydroxybutanoat}-Dihydrochlorid

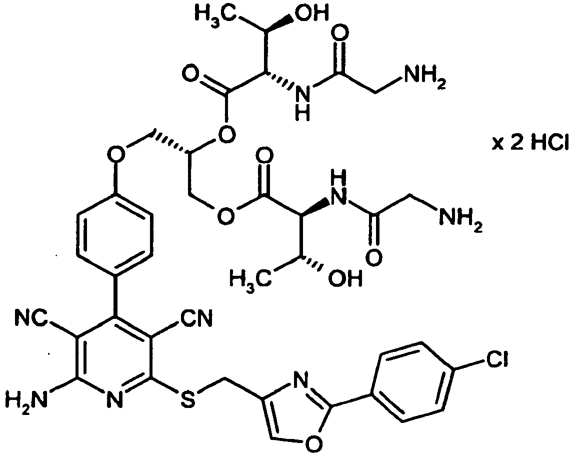

190 mg (0.18 mmol) der Verbindung aus Beispiel 55A wurden in 2.5 ml THF vorgelegt und tropfenweise mit 1.8 ml einer 2 M Lösung von Chlorwasserstoff in Diethylether versetzt. Es wurde über Nacht bei Raumtemperatur gerührt und die Mischung dann im Vakuum eingeengt. Der Rückstand wurde mittels präparativer HPLC gereinigt (Eluent: Acetonitril/Wasser + 0.15% Salzsäure). Es wurden 123 mg (64% d. Th.) der Zielverbindung erhalten.
¹H-NMR (400 MHz, DMSO-d₆): δ = 8.72 (dd, 2H), 8.38 (s, 1H), 8.30-8.00 (m, 6H), 7.97 (d, 2H), 7.62 (d, 2H), 7.49 (d, 2H), 7.11 (d, 2H), 5.39 (quint, 1H), 5.32-5.12 (br. s, 1H), 4.50-4.15 (m, 11H), 3.78-3.58 (m, 4H), 1.11 (dd, 6H).
LC-MS (Methode 5): Rₜ = 0.92 min; MS (ESIpos): m/z = 850 [M+H]⁺.

### Beispiel 38

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-(2S,3R,2'S,3'R)-bis(2-{[(2S)-2-aminopropanoyl]amino}-3-hydroxybutanoat)-Bis(trifluoressigsäure)-Salz

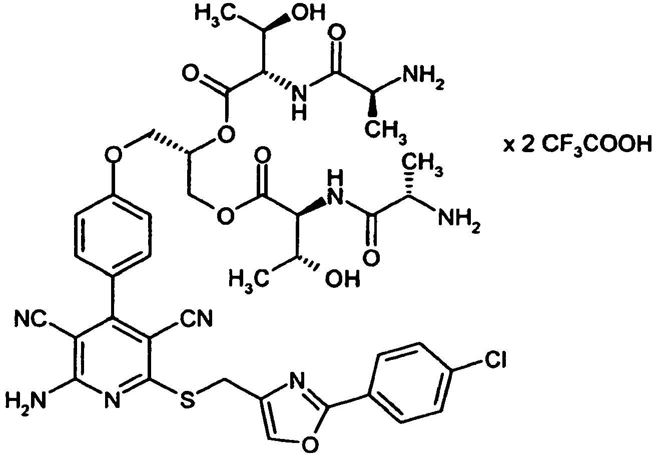

188 mg (0.17 mmol) der Verbindung aus Beispiel 56A wurden in 5 ml Dichlormethan vorgelegt und tropfenweise mit 0.54 ml (6.97 mmol) Trifluoressigsäure versetzt. Es wurde über Nacht bei Raumtemperatur gerührt und die Mischung dann im Vakuum eingeengt. Der Rückstand wurde mittels präparativer HPLC gereinigt (Eluent: Acetonitril/Wasser + 0.1% TFA). Es wurden 162 mg (84% d. Th.) der Zielverbindung erhalten.
¹H-NMR (400 MHz, DMSO-d₄): δ = 8.68 (d, 2H), 8.38 (s, 1H), 8.30-8.00 (m, 6H), 7.97 (d, 2H), 7.62 (d, 2H), 7.49 (d, 2H), 7.11 (d, 2H), 5.39 (quint, 1H), 5.32-5.12 (br. s, 1H), 4.45-4.18 (m, 11H), 4.09-3.98 (m, 2H), 1.39 (t, 6H), 1.12 (dd, 6H).
LC-MS (Methode 6): Rₜ = 1.17 min; MS (ESIpos): m/z = 878 [M+H]⁺.

### Beispiel 39

### (2S)-3-{4-[2-Amino-6-({[2-(4-chlorphenyl)-1,3-oxazol-4-yl]methyl}sulfanyl)-3,5-dicyanopyridin-4-yl]phenoxy}propan-1,2-diyl-bis(3-{[(2S,3R)-2-amino-3-hydroxybutanoyl]amino}propanoat) Bis(trifluoressigsäure)-Salz

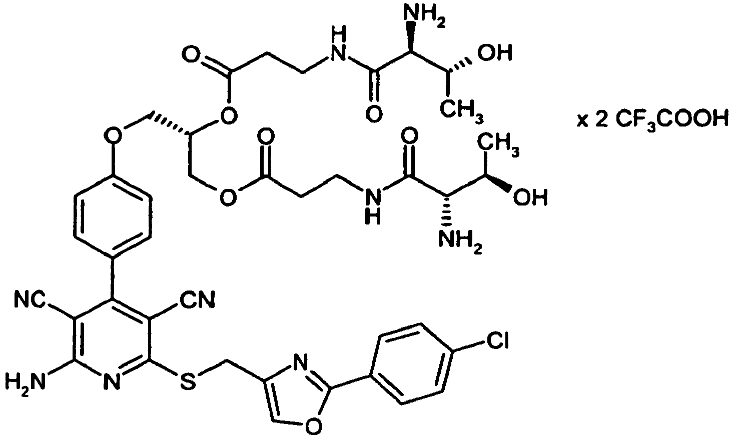

106 mg (0.09 mmol) der Verbindung aus Beispiel 57A wurden in 2.5 ml Dichlormethan vorgelegt und tropfenweise mit 0.27 ml (3.56 mmol) Trifluoressigsäure versetzt. Es wurde über Nacht bei Raumtemperatur gerührt und die Mischung dann im Vakuum eingeengt. Der Rückstand wurde mittels präparativer HPLC aufgereinigt (Eluent: Acetonitril/Wasser + 0.1% TFA). Anschließend erfolgte eine nochmalige Reinigung durch präparative HPLC [Säule: Waters Sunfire C18, 5 µm, 250 x 20 mm; Eluent: Acetonitril/0.2%-ige wässrige TFA (50:50); Fluss: 25 ml/min; Temperatur 30°C]. Das so erhaltene Produkt wurde mit 2.5 ml kaltem Acetonitril verrührt, und der Feststoff wurde abfiltriert und im Hochvakuum getrocknet. Es wurden 20 mg (21% d. Th.) der Zielverbindung erhalten.
¹H-NMR (400 MHz, DMSO-d₆): δ = 8.60-8.51 (m, 2H), 8.38 (s, 1H), 8.30-7.94 (m, 8H), 7.62 (d, 2H), 7.49 (d, 2H), 7.12 (d, 2H), 5.52 (br. s, 1H), 5.37 (quint, 1H), 4.47-4.20 (m, 6H), 3.86-3.77 (m, 2H), 3.52-3.38 (m, 4H), 2.60-2.51 (m, 7H), 1.11 (dd, 6H).
LC-MS (Methode 6): Rₜ = 1.24 min; MS (ESIpos): m/z = 878 [M+H]⁺.

### B. Bestimmung von Löslichkeit, Stabilität und Freisetzungsverbalten

### a) Bestimmung der Löslichkeit:

Die Prüfsubstanz wird in 5%-iger wässriger Dextrose-Lösung suspendiert. Diese Suspension wird 24 h bei Raumtemperatur geschüttelt. Nach Ultra-Zentrifugation bei 224000g für 30 min wird der Überstand mit DMSO verdünnt und per HPLC analysiert. Quantifiziert wird über eine Zwei-Punkt-Kalibrationskurve der Testverbindung in DMSO.

### HPLC-Methode für Säuren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C18, 5 µm, 50 mm x 2 mm; Temperatur: 40°C; Eluent A: Wasser/Phosphorsäure pH 2, Eluent B: Acetonitril; Flussrate: 0.7 ml/min; Gradient: 0-0.5 min 85% A, 15% B; Rampe 0.5-3 min 10% A, 90% B; 3-3.5 min 10% A, 90% B; Rampe 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 3.5 µm, 60 mm x 2.1 mm; Temperatur: 30°C; Eluent A: Wasser + 5 ml Perchlorsäure/Liter, Eluent B: Acetonitril; Flussrate: 0.75 ml/min; Gradient: 0-0.5 min 98% A, 2% B; Rampe 0.5-4.5 min 10% A, 90% B; 4.5-6 min 10% A, 90% B; Rampe 6.5-6.7 min 98% A, 2% B; 6.7-7.5 min 98% A, 2% B.

In Tabelle 1 sind die Löslichkeitswerte repräsentativer Ausführungsbeispiele in 5%-iger wässriger Dextrose-Lösung dargestellt:

**Tabelle 1**

| **Beispiel Nr.** | **Löslichkeit [mg/Liter]** |
|---|---|
| 2 | 670 |
| 3 | 590 |
| 4 | 860 |
| 31 | 220 |
| 33 | 620 |
| 35 | 610 |
| 36 | 700 |
| 38 | 750 |
| 39 | 490 |

Eine Zersetzung der Beispielverbindungen in diesen Lösungen wurde nicht beobachtet.

Die Löslichkeit der Wirksubstanz aus Beispiel 8A wurde in diesem Test mit < 1.2 mg/Liter bestimmt.

### b) Stabilität in Puffer bei verschiedenen pH-Werten:

0.3 mg der Prüfsubstanz werden in einem 2 ml-HPLC-Vial eingewogen und mit 0.5 ml Acetonitril bzw. Acetonitril/DMSO (9:1) versetzt. Zum Lösen der Substanz wird das Probengefäß für ca. 10 Sekunden ins Ultraschallbad gegeben. Anschließend werden 0.5 ml der jeweiligen (Puffer-) Lösung zugefügt und die Probe erneut im Ultraschallbad behandelt.

### Eingesetzte (Puffer-)Lösungen:

| | |
|---|---|
| pH 2: | 0.03 Mol Zitronensäure, 0.061 Mol Natriumchlorid und 0.0082 Mol Salzsäure *ad* 1 Liter Wasser; |
| pH 4: | 1 Liter Millipore-Wasser wird mit 1 N Salzsäure auf pH 4.0 eingestellt; |
| pH 5: | 0.096 Mol Zitronensäure und 0.2 Mol Natriumhydroxid *ad 1* Liter Wasser; |
| pH 6: | 0.06 Mol Zitronensäure und 0.16 Mol Natriumhydroxid *ad* 1 Liter Wasser; |
| pH 7.4: | 90.0 g Natriumchlorid, 13.61 g Kaliumdihydrogenphosphat und 83.35 g 1 N Natronlauge *ad* 1 Liter Wasser; diese Lösung wird dann noch 1:10 mit Millipore-Wasser weiter verdünnt; |
| pH 8: | 0.013 Mol Borax und 0.021 Mol Salzsäure *ad* 1 Liter Wasser. |

Über einen Zeitraum von 24 Stunden bei 37°C werden stündlich jeweils 5 pl der Probenlösung per HPLC auf ihren Gehalt an unveränderter Testsubstanz bzw. an gebildeter Wirksubstanz (A) analysiert. Quantifiziert wird über die Flächenprozente der entsprechenden Peaks.

### HPLC-Methode für Beispiel 2:

Agilent 1100 mit DAD (G1315B), binärer Pumpe (G1312A), Autosampler (G1329A), Säulenofen (G1316A), Thermostat (G1330B); Säule: Kromasil 100 C18, 125 mm x 4 mm, 5 µm; Säulentemperatur: 30°C; Eluent A: Wasser + 5 ml Perchlorsäure/Liter, Eluent B: Acetonitril; Gradient: 0 min 90% A → 6.0 min 61% A →18.0 min 61% A → 20.0 min 10% A → 21.0 min 10% A → 23.0 min 90% A → 26.0 min 90% A; Flussrate: 2.0 ml/min; UV-Detektion: 294 nm.

### HPLC-Methode für Beispiel 7:

Agilent 1100 mit DAD (G1314A), binärer Pumpe (G1312A), Autosampler (G1329A), Säulenofen (G1316A), Thermostat (G1330A); Säule: Kromasil 100 C18, 250 mm x 4 mm, 5 µm; Säulentemperatur: 30°C; Eluent A: Wasser + 5 ml Perchlorsäure/Liter, Eluent B: Acetonitril; Gradient: 0 min 90% A → 5.0 min 55% A → 18.0 min 55% A → 20.0 min 10% A → 21.0 min 10% A → 22.5 min 90% A → 25.0 min 90% A; Flussrate: 2.0 ml/min; UV-Detektion: 288 nm.

### HPLC-Methode für Beispiel 8:

Agilent 1100 mit DAD (G1315B), binärer Pumpe (G1312A), Autosampler (G1329A), Säulenofen (G1316A), Thermostat (G1330B); Säule: Kromasil 100 C18, 250 mm x 4 mm, 5 µm; Säulentemperatur: 30°C; Eluent A: Wasser + 5 ml Perchlorsäure/Liter, Eluent B: Acetonitril; Gradient: 0 min 90% A → 3.0 min 55.5% A → 14.0 min 55.5% A →20.0 min 10% A → 23.0 min 10% A → 26.0 min 90% A; Flussrate: 2.0 ml/min; UV-Detektion: 294 nm.

### HPLC-Methode für Beispiel 14:

Agilent 1100 mit DAD (G1315A), binärer Pumpe (G1312A), Autosampler (G1329A), Säulenofen (G1316A), Thermostat (G1330A); Säule: Kromasil 100 C18, 125 mm x 4 mm, 5 µm; Säulentemperatur: 30°C; Eluent A: Wasser + 5 ml Perchlorsäure/Liter, Eluent B: Acetonitril; Gradient: 0 min 90% A → 4.0 min 56% A → 18.0 min 56% A → 20.0 min 10% A → 21.0 min 10% A → 23.0 min 90% A → 25.0 min 90% A; Flussrate: 2.0 ml/min; UV-Detektion: 294 nm.

### HPLC-Methode für Beispiel 26:

Agilent 1100 mit DAD (G1315A), binärer Pumpe (G1312A), Autosampier (G1329A), Säulenofen (G1316A), Thermostat (G1330A); Säule: Kromasil 100 C18, 125 mm x 4 mm, 5 µm; Säulentemperatur: 30°C; Eluent A: Wasser + 5 ml Perchlorsäure/Liter, Eluent B: Acetonitril; Gradient: 0 min 90% A → 8.0 min 47% A → 18.0 min 47% A → 20.0 min 10% A → 21.0 min 10% A → 22.5 min 98% A → 25.0 min 98% A; Flussrate: 2.0 ml/min; UV-Detektion: 294 nm.

### HPLC-Methode für Beispiel 28 und Beispiel 32:

Agilent 1100 mit DAD (G1314A), binärer Pumpe (G1312A), Autosampler (G1329A), Säulenofen (G1316A), Thermostat (G1330A); Säule: Kromasil 100 C18, 125 mm x 4 mm, 5 µm; Säulentemperatur: 30°C; Eluent A: Wasser + 5 ml Perchlorsäure/Liter, Eluent B: Acetonitril; Gradient: 0 min 90% A → 6.0 min 61% A → 18.0 min 61% A → 20.0 min 10% A → 21.0 min 10% A → 23.0 min 90% A → 26.0 min 90% A; Flussrate: 2.0 ml/min; UV-Detektion: 294 nm.

### HPLC-Methode für Beispiel 33:

Agilent 1100 mit DAD (G1314A), binärer Pumpe (G1312A), Autosampler (G1329A), Säulenofen (G1316A), Thermostat (G1330A); Säule: Kromasil 100 C18, 125 mm x 4 mm, 5 µm; Säulentemperatur: 30°C; Eluent A: Wasser + 5 ml Perchlorsäure/Liter, Eluent B: Acetonitril; Gradient: 0 min 90% A → 4.0 min 56% A → 18.0 min 56% A → 20.0 min 10% A → 21.0 min 10% A → 23.0 min 90% A → 25.0 min 90% A; Flussrate: 2.0 ml/min; UV-Detektion: 294 nm.

### HPLC-Methode für Beispiel 34:

Agilent 1100 mit DAD (G1314A), binärer Pumpe (G1312A), Autosampler (G1329A), Säulenofen (G1316A), Thermostat (G1330A); Säule: Kromasil 100 C18, 60 mm x 2.1 mm, 3.5 µm; Säulen-temperatur: 30°C; Eluent A: Wasser + 5 ml Perchlorsäure/Liter, Eluent B: Acetonitril; Gradient: 0 min 98% A → 1.0 min 98% A → 9.0 min 2% A → 13.0 min 2% A → 13.5 min 98% A → 15.0 min 98% A; Flussrate: 0.75 ml/min; UV-Detektion: 210 nm.

### HPLC-Methode für Beispiel 35:

Agilent 1100 mit DAD (G1314A), binärer Pumpe (G1312A), Autosampler (G1329A), Säulenofen (G1316A), Thermostat (G1330A); Säule: Kromasil 100 C18, 125 mm x 4 mm, 5 µm; Säulentemperatur: 30°C; Eluent A: Wasser + 5 ml Perchlorsäure/Liter, Eluent B: Acetonitril; Gradient: 0 min 90% A → 2.0 min 64% A → 18.0 min 64% A → 20.0 min 10% A → 21.0 min 10% A → 23.0 min 90% A → 26.0 min 90% A; Flussrate: 2.0 ml/min; UV-Detektion: 294 nm.

In Tabelle 2 sind zu repräsentativen Ausführungsbeispielen die Verhältnisse der Peakflächen (F) zu den jeweiligen Zeitpunkten in Relation zu den Peakflächen beim Startzeitpunkt dargestellt:

**Tabelle 2**

| **Beispiel Nr.** | **pH-Wert** | **% Testsubstanz nach 4 b [F(t=4h) x 100 / F(t=0h)]** | **% Testsubstanz nach 24 h (F(t=24h) x 100 / F(t=0h)]** |
|---|---|---|---|
| 2 | 4 | 100 | 100 |
| 2 | 7.4 | 100 | 68 |
| 7 | 4 | 100 | 100 |
| 7 | 5 | 98 | 83 |
| 7 | 7.4 | 0 | 0 |
| 8 | 4 | 100 | 100 |
| 8 | 7.4 | 82 | 34 |
| 14 | 4 | 96 | 95 |
| 14 | 7.4 | 100 | 91 |
| 26 | 4 | 100 | 99 |
| 26 | 5 | 97 | 92 |
| 26 | 7.4 | 84 | 34 |
| 28 | 4 | 100 | 99 |
| 28 | 7.4 | 99 | 88 |
| 32 | 4 | 100 | 100 |
| 32 | 5 | 100 | 100 |
| 32 | 6 | 99 | 98 |
| 32 | 7.4 | 96 | 81 |
| 33 | 4 | 100 | 100 |
| 33 | 5 | 100 | 97 |
| 33 | 6 | 78 | 28 |
| 33 | 7.4 | 29 | 0 |
| 34 | 4 | 100 | 99 |
| 34 | 7.4 | 0 | 0 |
| 35 | 4 | 98 | 94 |
| 35 | 7.4 | 2 | 0 |

In diesem Test wurde gleichzeitig mit einer Abnahme des Gehalts an Testsubstanz eine Zunahme der betreffenden Wirkstoff-Verbindung aus Beispiel 8A bzw. 9A festgestellt.

### c) In vitro-Stabilität in Ratten- und Humanplasma:

1 mg der Prüfsubstanz wird in einem 2 ml-HPLC-Vial eingewogen und mit 1.5 ml DMSO und 1 ml Wasser versetzt. Zum Lösen der Substanz wird das Probengefäß für ca. 10 Sekunden ins Ultraschallbad gestellt. Zu 0.5 ml dieser Lösung werden 0.5 ml 37°C warmes Ratten- bzw. Humanplasma gegeben. Die Probe wird geschüttelt und für eine erste Analyse ca. 10 µl entnommen (Zeitpunkt t₀). Im Zeitraum bis zu 2 Stunden nach Inkubationsbeginn werden 4-6 weitere Aliquote zur Quantifizierung entnommen. Die Probe wird über die Prüfzeit bei 37°C gehalten. Charakterisierung und Quantifizierung erfolgen per HPLC.

### HPLC-Methode:

Agilent 1100 mit DAD (G1314A), binärer Pumpe (G1312A), Autosampler (G1329A), Säulenofen (G1316A), Thermostat (G1330A); Säule: Kromasil 100 C18, 250 mm x 4 mm, 5 µm; Säulentemperatur: 30°C; Eluent A: Wasser + 5 ml Perchlorsäure/Liter, Eluent B: Acetonitril; Gradient: 0-8.0 min 53% A, 47% B; 8.0-18.0 min 53% A, 47% B; 18.0-20.0 min 90% A, 10% B; 20.0-21.0 min 90% A, 10% B; 21.0-22.5 min 98% A, 2% B; 22.5-25.0 min 98% A, 2% B; Flussrate: 2 ml/min; UV-Detektion: 294 nm.

In Tabelle 3 sind für repräsentative Ausführungsbeispiele die jeweiligen Zeiten angegeben, bei denen nach Inkubation mit Rattenplasma 50% (Beispiel 33: 30%) der maximal möglichen Menge an Wirkstoff-Verbindung (Beispiel 8A bzw. 9A) entstanden sind (t_{50%A} bzw. t_{30%A}). Zur Auswertung wurde jeweils das Verhältnis der Peakflächen zu den einzelnen Zeitpunkten gegenüber dem Startzeitpunkt herangezogen.

**Tabelle 3**

| **Beispiel Nr.** | **t_{50%A} [min] in Rattenplasma** |
|---|---|
| 2 | 5 |
| 7 | 5 |
| 8 | 5 |
| 26 | 3 |
| 31 | 3 |
| 32 | 90 |
| 33 | 17* |
| 34 | 10 |
| 35 | 10 |

| | |
|---|---|
| * t_{30%A}-Wert [min] | |

### d) i.v.-Pharmakokinetik in Wistar-Ratten:

Am Tag vor der Substanzgabe wird den Versuchstieren (männliche Wistar-Ratten, Körpergewicht 200-250 g) unter Isofluran^{®}-Narkose ein Katheter für die Blutgewinnung in die *Vena jugularis* implantiert.

Am Versuchstag wird eine definierte Dosis der Prüfsubstanz als Lösung mit einer Hamilton^{®}-Glasspritze in die Schwanzvene appliziert (Bolusgabe, Apptikationsdauer < 10 s). Innerhalb von 24 h nach Substanzgabe werden sequentiell Blutproben (8-12 Zeitpunkte) über den Katheter entnommen. Zur Plasmagewinnung werden die Proben in heparinisierten Röhrchen zentrifugiert. Pro Zeitpunkt wird ein definiertes Plasmavolumen zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Die Berechnung pharmakokinetischer Kenngrößen der Prüfsubstanz bzw. der daraus freigesetzten Wirkstoff-Verbindung (A) wie AUC, Cₘₐₓ, T_{½} (Halbwertszeit) und CL (Clearance) erfolgt aus den gemessenen Plasmakonzentrationen.

Nach i.v.-Applikation der Verbindung aus Beispiel 32 bzw. Beispiel 33 konnten diese Substanzen bereits zum ersten Messpunkt nicht mehr im Plasma detektiert werden. Lediglich der Wirkstoff (Beispiel 8A) war auch bis zum Zeitpunkt von 24 Stunden detektierbar.

### e) p.o.-Pharmakokinetik in Wistar-Ratten:

Am Tag vor der Substanzgabe wird den Versuchstieren (männliche Wistar-Ratten, Körpergewicht 200-250 g) unter Isofluran^{®}-Narkose ein Katheter für die Blutgewinnung in die *Vena jugularis* implantiert.

Am Versuchstag wird eine definierte Dosis der Prüfsubstanz als Lösung mit einer Schlundsonde in den Magen appliziert. Innerhalb von 24 h nach Substanzgabe werden sequentiell Blutproben (8-12 Zeitpunkte) über den Katheter entnommen. Zur Plasmagewinnung werden die Proben in heparinisierten Röhrchen zentrifugiert. Pro Zeitpunkt wird ein definiertes Plasmavolumen zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Die Berechnung pharmakokinetischer Kenngrößen der Prüfsubstanz bzw. der daraus freigesetzten Wirkstoff-Verbindung (A) wie AUC, Cₘₐₓ und T_{½} (Halbwertszeit) erfolgt aus den gemessenen Plasmakonzentrationen.

Nach p.o.-Applikation der Verbindung aus Beispiel 2, Beispiel 7, Beispiel 8 bzw. Beispiel 33 konnten diese Substanzen bereits zum ersten Messpunkt nicht mehr im Plasma detektiert werden. Lediglich der Wirkstoff (Beispiel 8A) war auch bis zum Zeitpunkt von 24 Stunden detektierbar.

### f) Hämodynamische Messungen an narkotisierten Ratten:

Wistar-Ratten (250-300 g Körpergewicht; Fa. Harlan-Winkelmann) werden mit 5% Isofluran^{®} narkotisiert. Die Narkose wird mit 2% Isofluran^{®} und Druckluft in einer Narkosemaske aufrecht erhalten. Die *A. carotis* wird frei präpariert, und ein Tip-Katheter (Millar Micro-Tip-Transducer, 2 French; Fa. HSE) wird eingeführt und bis in den linken Ventrikel vorgeschoben. Anschließend wird ein zweiter Katheter in die *V. jugularis* eingeführt. Über diesen Katheter werden Placebo-Lösung und Testsubstanz-Lösungen in aufsteigender Konzentration in die Tiere infundiert. Gleichzeitig erfolgt die Messung der Herzfunktion (wie Herzfrequenz, linksventrikulärer Druck, Kontraktilität (dp/dt), linksventrikulärer enddiastolischer Druck) durch den linksventrikulären Katheter. Durch Zurückziehen des Katheters aus dem linken Ventrikel in die Aorta kann auch noch der systemische Blutdruck gemessen werden.

### g) Blutdruck- und Herzfrequenz-Messungen an wachen Ratten:

Wachen spontan-hypertensiven Ratten (SH-Ratten), die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral verabreicht. Anschließend werden über 24 Stunden Blutdruck und Herzfrequenz und deren Veränderungen aufgezeichnet.

In Tabelle 4 ist die maximale Herzfrequenz-Senkung nach einer p.o.-Gabe von 3 mg/kg der Verbindung aus Beispiel 32 bzw. Beispiel 33 im Vergleich zur Wirksubstanz (Beispiel 8A) wiedergegeben:

**Tabelle 4**

| **Beispiel Nr.** | **Dosierung** | **Herzfrequenz-Senkung** |
|---|---|---|
| 8A | 3 mg/kg | 10% |
| 32 | 3 mg/kg | 10% |
| 33 | 3 mg/kg | 10% |

### C. Ausführungsbeispiele für pharmazeutische Zusammensetzungen

Die erfindungsgemäßen Verbindungen können beispielsweise folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%, die jeweils auf einen pH-Wert von 3-5 eingestellt sind) gelöst. Die Lösung wird gegebenenfalls steril filtriert und/oder in sterile und pyrogenfreie Injektionsbehältnisse abgefüllt.

## Patentansprüche

1. Verbindung der Formel (I) 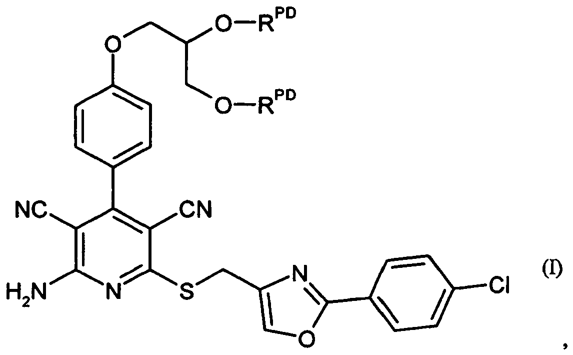 in welcher
R^{PD} für eine Gruppe der Formel 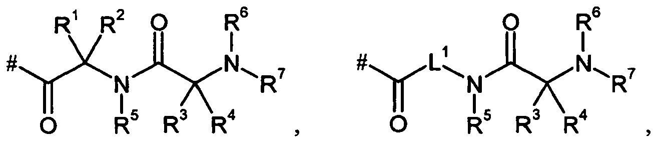 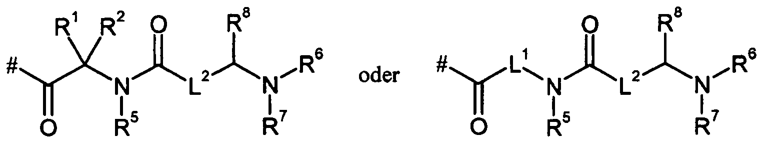 steht, worin
# die Verknüpfungsstelle mit dem jeweiligen O-Atom bedeutet,
L¹ geradkettiges (C₂-C₄)-Alkandiyl bedeutet,
L² geradkettiges (C₁-C₃)-Alkandiyl bedeutet,
R¹ und R³ gleich oder verschieden sind und unabhängig voneinander Wasserstoff oder Methyl (Alanin), Propan-2-yl (Valin), Propan-1-yl (Norvalin), 2-Methylpropan-l-yl (Leucin), 1-Methylpropan-1-yl (Isoleucin), Butan-1-yl (Norleucin), *tert*.-Butyl (2-*tert*.-Butylglycin), Phenyl (2-Phenylglycin), Benzyl (Phenylalanin), *p*-Hydroxybenzyl (Tyrosin), Indol-3-yl-methyl (Tryptophan), Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (*S*-Methylcystein), 2-Mercaptoethyl (Homocystein), 2-Methylthioethyl (Methionin), Carbamoylmethyl (Asparagin), 2-Carbamoylethyl (Glutamin), Carboxymethyl (Asparaginsäure), 2-Carboxyethyl (Glutaminsäure), 4-Aminobutan-1-yl (Lysin), 4-Amino-3-hydroxybutan-1-yl (Hydroxylysin), 3-Aminopropan-1-yl (Ornithin), 2-Aminoethyl (2,4-Diaminobuttersäure), Aminomethyl (2,3-Diaminopropionsäure), 3-Guanidinopropan-1-yl (Arginin), 3-Ureidopropan-1-yl (Citrullin) bedeuten,
R² und R⁴ unabhängig voneinander Wasserstoff oder Methyl bedeuten
oder
R¹ und R² oder R³ und R⁴ jeweils miteinander verknüpft sind und zusammen mit dem Kohlenstoffatom, an das sie gemeinsam gebunden sind, einen 3- bis 6-gliedrigen gesättigten Carbocyclus bilden,
R⁵ Wasserstoff oder (C₁-C₄)-Alkyl bedeutet
oder
R⁵ mit R¹ verknüpft ist und beide zusammen mit den Atomen, an die sie gebunden sind, einen Pyrrolidin- oder Piperidin-Ring bilden,
R⁶ und R⁷ gleich oder verschieden sind und unabhängig voneinander Wasserstoff oder (C₁-C₄)-Alkyl, das mit Hydroxy, (C₁-C₄)-Alkoxy, Amino, Mono-(C₁-C₄)-alkylamino oder Di-(C₁-C₄)-alkylamino substituiert sein kann, bedeuten
oder
R⁶ und R⁷ miteinander verknüpft sind und zusammen mit dem Stickstoffatom, an das sie gebunden sind, einen 5- oder 6-gliedrigen gesättigten Heterocyclus bilden, der ein weiteres Ring-Heteroatom aus der Reihe N und O enthalten und ein- oder zweifach, gleich oder verschieden, mit (C₁-C₄)-Alkyl, Amino, Hydroxy und/oder (C₁-C₄)-Alkoxy substituiert sein kann,
oder
R⁶ mit R³ verknüpft ist und beide zusammen mit den Atomen, an die sie gebunden sind, einen Pyrrolidin- oder Piperidin-Ring bilden
und
R⁸ Wasserstoff oder Carboxyl bedeutet,
sowie ihre Salze, Solvate und Solvate der Salze.

2. Verbindung der Formel (I) nach Anspruch 1, in welcher
R^{PD} für eine Gruppe der Formel 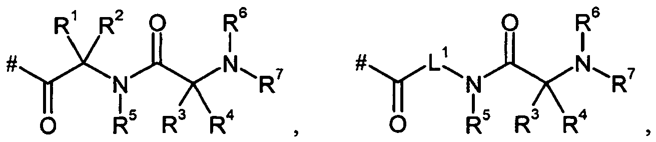 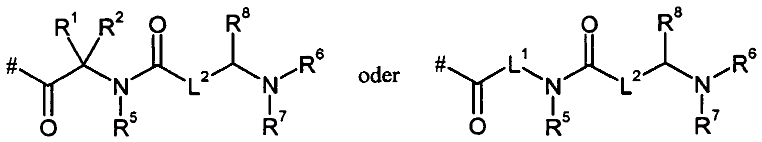 steht, worin
# die Verknüpfungsstelle mit dem jeweiligen O-Atom bedeutet,
L¹ Ethan-1,2-diyl bedeutet,
L² Methandiyl oder Ethan-1,2-diyl bedeutet,
R¹ Wasserstoff, Methyl, Propan-2-yl, 1-Methylpropan-1-yl, 2-Methylpropan-1-yl, Benzyl, *p*-Hydroxybenzyl, Hydroxymethyl oder 1-Hydroxyethyl bedeutet,
R² Wasserstoff bedeutet,
R³ Wasserstoff, Methyl, Propan-2-yl, 1-Methylpropan-1-yl, 2-Methylpropan-1-yl, Benzyl, Imidazol-4-ylmethyl, Hydroxymethyl, 1-Hydroxyethyl, Carbamoylmethyl, 2-Carbamoyl-ethyl, Carboxymethyl, 2-Carboxyethyl, 4-Aminobutan-1-yl, 3-Aminopropan-1-yl, 2-Aminoethyl, Aminomethyl oder 3-Guanidinopropan-1-yl bedeutet,
R⁴ Wasserstoff bedeutet,
R⁵ Wasserstoff oder Methyl bedeutet
oder
R⁵ mit R¹ verknüpft ist und beide zusammen mit den Atomen, an die sie gebunden sind, einen Pyrrolidin-Ring bilden,
R⁶ Wasserstoff oder Methyl bedeutet
oder
R⁶ mit R³ verknüpft ist und beide zusammen mit den Atomen, an die sie gebunden sind, einen Pyrrolidin-Ring bilden,
R⁷ Wasserstoff oder Methyl bedeutet und
R⁸ Wasserstoff oder Carboxyl bedeutet,
sowie ihre Salze, Solvate und Solvate der Salze.

3. Verbindung der Formel (I) nach Anspruch 1 oder 2, in welcher
R^{PD} für eine Gruppe der Formel 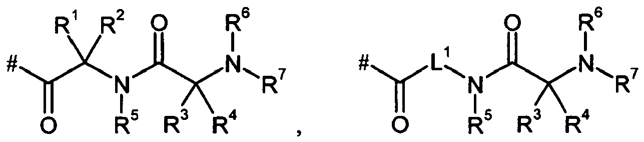 oder 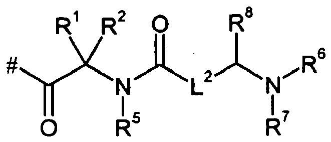 steht, worin
# die Verknüpfungsstelle mit dem jeweiligen O-Atom bedeutet,
L¹ Ethan-1,2-diyl bedeutet,
L² Methandiyl bedeutet,
R¹ Wasserstoff, Methyl, Propan-2-yl, 1-Methylpropan-1-yl, 2-Methylpropan-1-yl, Hydroxymethyl oder 1-Hydroxyethyl bedeutet,
R² Wasserstoff bedeutet,
R³ Wasserstoff, Methyl, Propan-2-yl, 1-Methylpropan-1-yl, 2-Methylpropan-1-yl, Imidazol-4-ylmethyl, Hydroxymethyl, 1-Hydroxyethyl, 2-Carboxyethyl, 4-Aminobutan-1-yl, 3-Amino-propan-1-yl oder 2-Aminoethyl bedeutet,
R⁴ Wasserstoff bedeutet,
R⁵ Wasserstoff bedeutet,
R⁶ Wasserstoff oder Methyl bedeutet oder
R⁶ mit R³ verknüpft ist und beide zusammen mit den Atomen, an die sie gebunden sind, einen Pyrrolidin-Ring bilden,
R⁷ Wasserstoff bedeutet
und
R⁸ Wasserstoff bedeutet,
sowie ihre Salze, Solvate und Solvate der Salze.

4. Verbindung der Formel (I) nach Anspruch 1, 2 oder 3, in welcher die beiden Gruppen R^{PD} identisch sind, sowie ihre Salze, Solvate und Solvate der Salze.

5. Verbindung gemäß Anspruch 1, 2, 3 oder 4 mit der Formel (I-A) 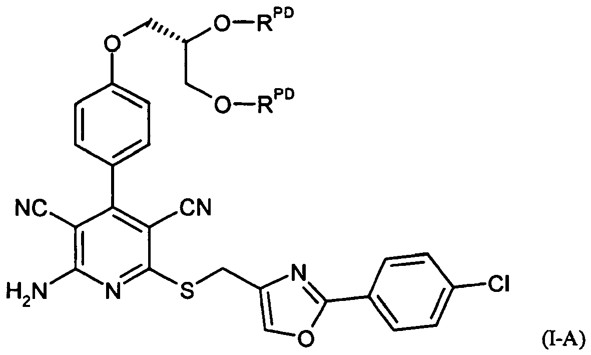 sowie ihre Salze, Solvate und Solvate der Salze.

6. Verbindung (2S)-3-{4-[2-Amino-6-({[2-(4-chlorphenyl)-1,3-oxazol-4-yl]methyl}sulfanyl)-3,5-dicyano-pyridin-4-yl]phenoxy}propan-1,2-diyl-(2S,2'S)-bis{2-[(3-aminopropanoyl)amino]propanoat}-Dihydrochlorid

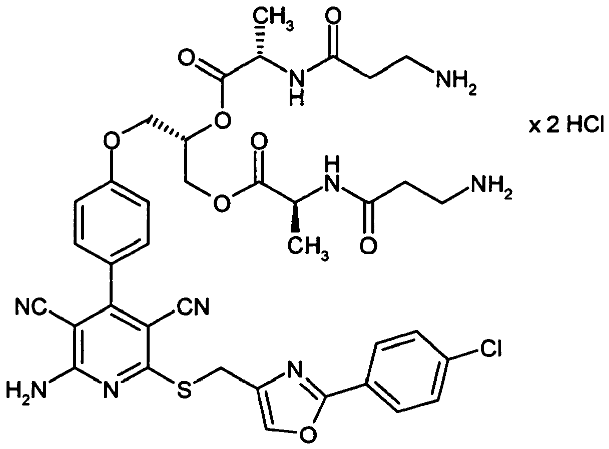

7. Verbindung (2S)-3-{4-[2-Amino-6-({[2-(4-chlorphenyl)-1,3-oxazol-4-yl]methyl}sulfanyl)-3,5-dicyano-pyridin-4-yl]phenoxy}propan-1,2-diyl-(2S,2'S)-bis(2-{[(2S)-2-aminopropanoyl]amino}propanoat)-Dihydrochlorid

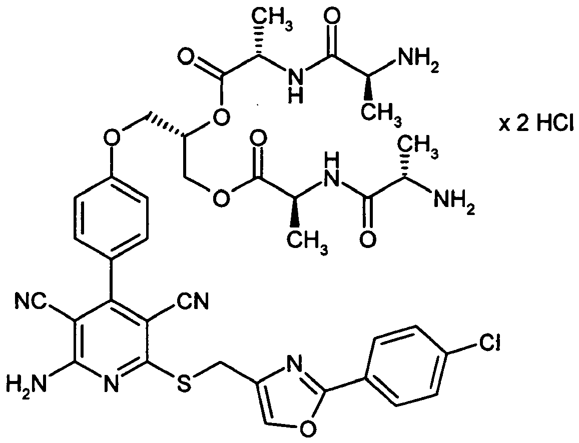

8. Verbindung (2*S*)-3-{4-[2-Amino-6-({[2-(4-chlorphenyl)-1,3-oxazol-4-yl]methyl}sutfanyl)-3,5-dicyano-pyridin-4-yl]phenoxy}propan-1,2-diyl-(2*S*,2'*S*)-bis(2-{[(2*S*)-2-aminopropanoyl]amino}propanoat)-Bis(4-methylbenzolsulfonsäure)-Salz

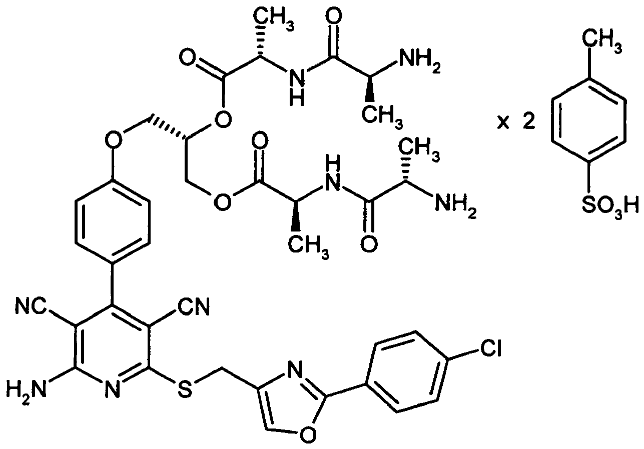

9. Verbindung (2*S*)-3-{4-[2-Amino-6-({[2-(4-chlorphenyl)-1,3-oxazol-4-yl]methyl}sulfanyl)-3,5-dicyano-pyridin-4-yl]phenoxy}propan-1,2-diyl-(2*S*,2'*S*)-bis(2-{[(2*R*)-2-aminopropanoyl]amino}propanoat)-Dihydrochlorid

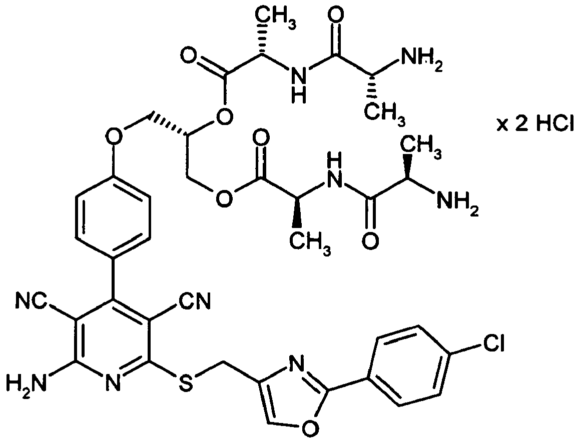

10. Verbindung (2*S*)-3-{4-[2-Amino-6-({[2-(4-chlorphenyl)-1,3-oxazol-4-yl]methyl)sulfanyl)-3,5-dicyano-pyridin-4-yl]phenoxy}propan-1,2-diyl-{2*R*,2'*R*)-bis(2-{[(2*R*)-2-aminopropanoyl]amino}propanoat)-Dihydrochlorid

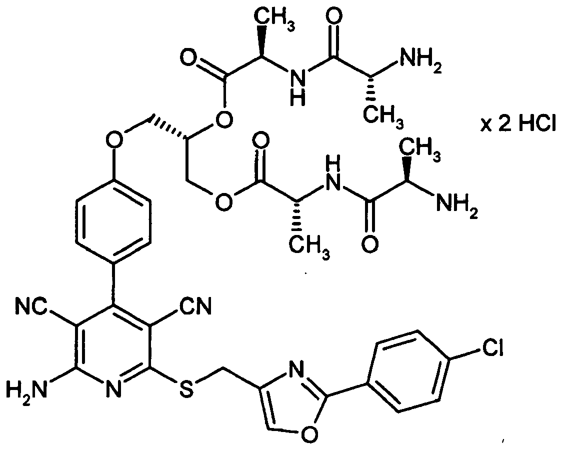

11. Verfahren zur Herstellung von Verbindungen der Formel (I), wie in den Ansprüchen 1 bis 10 definiert, in welchen die beiden Gruppen R^{PD} jeweils identisch sind, **dadurch gekennzeichnet, dass** man die Verbindung (A) 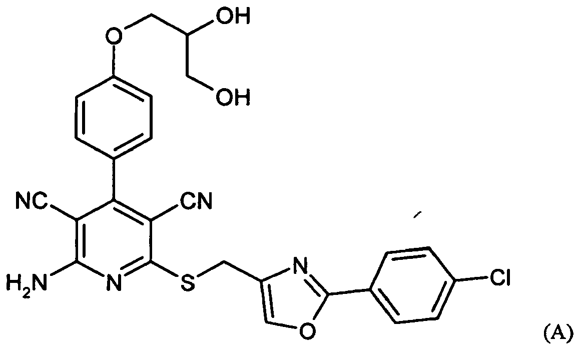 entweder
[A] in einem inerten Lösungsmittel in Gegenwart eines Kondensationsmittels zunächst mit zwei oder mehr Äquivalenten einer Aminosäure der Formel (II) oder (III) 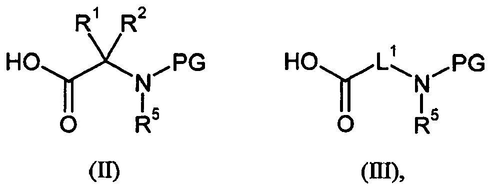 in welchen L¹, R¹, R² und R⁵ die in den Ansprüchen 1 bis 5 angegebenen Bedeutungen haben und
PG für eine temporäre Amino-Schutzgruppe wie beispielsweise *tert*.-Butoxycarbonyl steht, zu einer Verbindung der Formel (IV) bzw: (V) 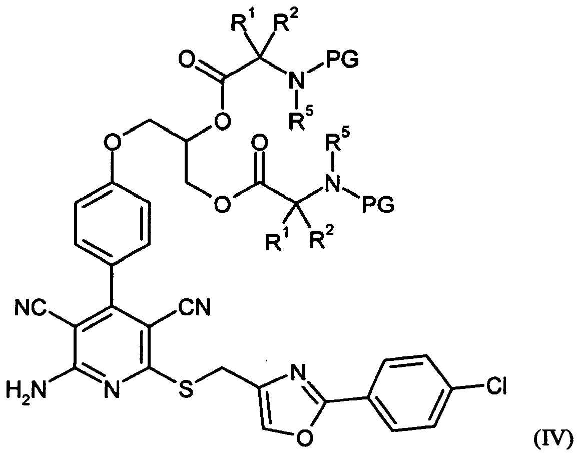 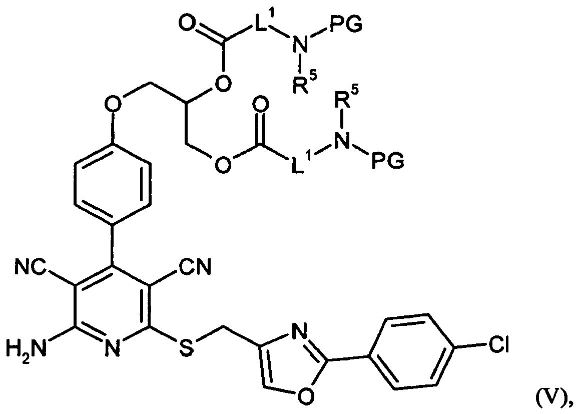 in welchen L¹, PG, R¹R² und R⁵ die oben angegebenen Bedeutungen haben, verestert, diese nach Abspaltung der Schutzgruppen PG dann in einem inerten Lösungsmittel in Gegenwart eines Kondensationsmittels mit zwei oder mehr Äquivalenten einer Aminosäure der Formel (VI) oder (VII) 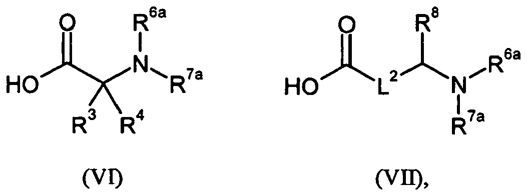 in welchen L², R³, R⁴ und R⁸ die in den Ansprüchen 1 bis 5 angegebenen Bedeutungen haben und
R^{6a} und R^{7a} gleich oder verschieden sind und die in den Ansprüchen 1 bis 5 angegebenen Bedeutungen von R⁶ bzw. R⁷ haben oder für eine temporäre Amino-Schutzgruppe stehen,
zu einer Verbindung der Formel (VIII), (IX), (X) bzw. (XI) 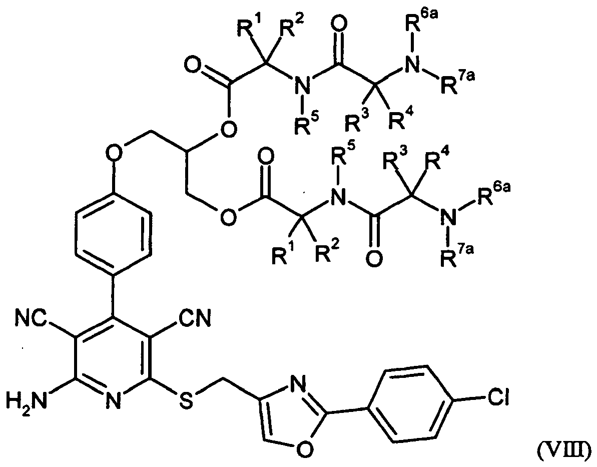 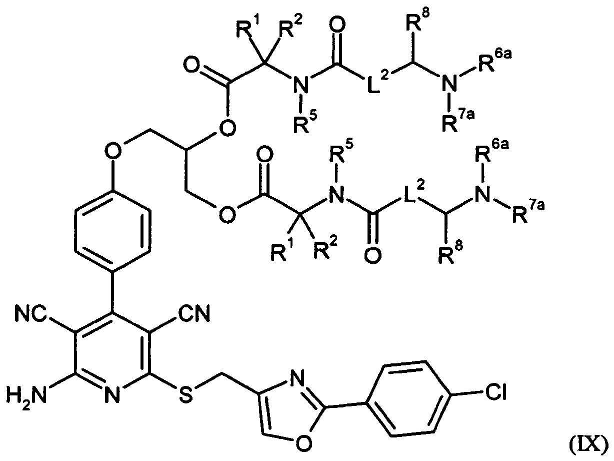 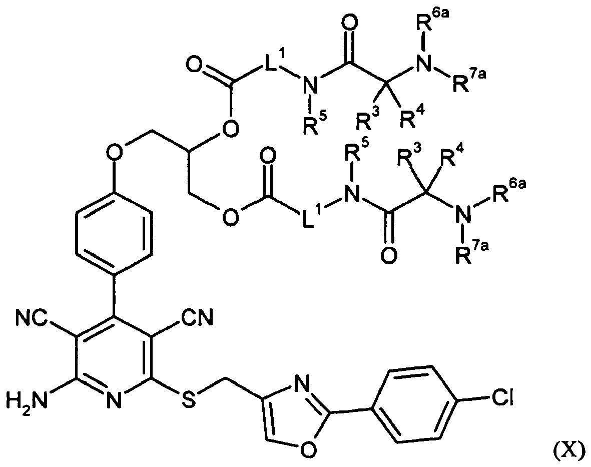 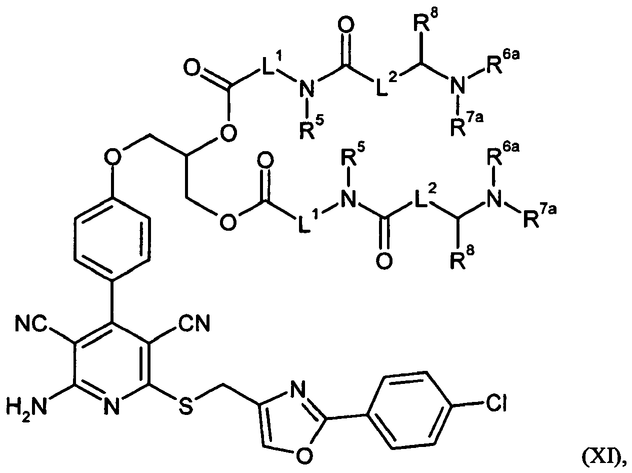 in welchen L¹, L², R¹, R², R³, R⁴, R⁵, R^{6a}, R^{7a} und R⁸ jeweils die oben angegebenen Bedeutungen haben,
kuppelt und anschließend gegebenenfalls vorhandene Schutzgruppen wieder entfernt
oder
[B] in einem inerten Lösungsmittel in Gegenwart eines Kondensationsmittels mit zwei oder mehr Äquivalenten einer Carbonsäure der Formel (XII), (XIII), (XIV) oder (XV) 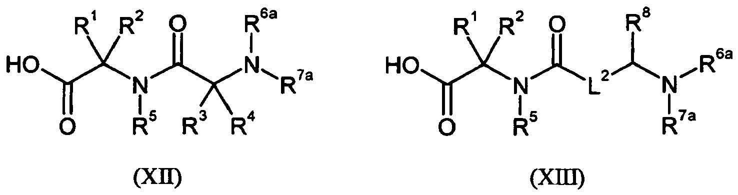 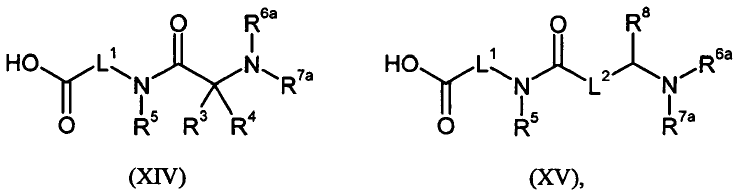 in welchen L¹, L², R¹, R², R³, R⁴, R⁵ und R⁸ die in den Ansprüchen 1 bis 5 angegebenen Bedeutungen haben
und
R^{6a} und R^{7a} gleich oder verschieden sind und die in den Ansprüchen 1 bis 5 angegebenen Bedeutungen von R⁶ bzw. R⁷ haben oder für eine temporäre Amino-Schutzgruppe stehen,
zu einer der in Variante [A] aufgeführten Verbindungen (VIII), (IX), (X) bzw. (XI) kuppelt und anschließend gegebenenfalls vorhandene Schutzgruppen wieder entfernt und die jeweils resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

12. Verbindung, wie in einem der Ansprüche 1 bis 10 definiert, zur Behandlung und/oder Prophylaxe von Krankheiten.

13. Verbindung, wie in einem der Ansprüche 1 bis 10 definiert, zur Verwendung in einem Verfahren zur Behandlung und/oder Prophylaxe von kardiovaskulären Erkrankungen.

14. Verwendung einer Verbindung, wie in einem der Ansprüche 1 bis 10 definiert, zur Herstellung eines Arzneimittels zur Behandlung und/oder Prophylaxe von kardiovaskulären Erkrankungen.

15. Arzneimittel enthaltend eine Verbindung, wie in einem der Ansprüche 1 bis 10 definiert, gegebenenfalls in Kombination mit einem oder mehreren inerten, nicht-toxischen, pharmazeutisch geeigneten Hilfsstoffen.

16. Arzneimittel enthaltend eine Verbindung, wie in einem der Ansprüche 1 bis 10 definiert, in Kombination mit einem oder mehreren weiteren Wirkstoffen.

17. Arzneimittel nach Anspruch 15 oder 16 zur Behandlung und/oder Prophylaxe von kardiovaskulären Erkrankungen.

## Claims

1. Compound of the formula (I) 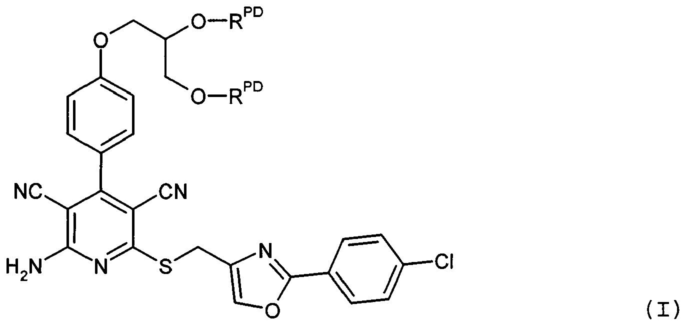 in which
R^{PD} is a group of the formula 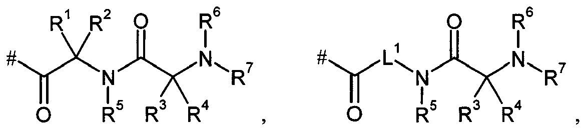 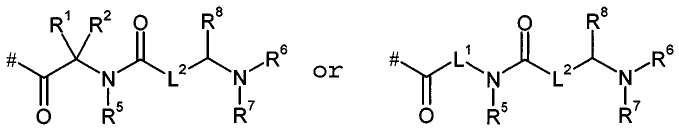 in which
# means the point of linkage to the respective O atom,
L¹ is straight-chain (C₂-C₄)-alkanediyl,
L² is straight-chain (C₁-C₃)-alkanediyl,
R¹ and R³ are identical or different and independently of one another are hydrogen or methyl (alanine), propan-2-yl (valine), propan-1-yl (norvaline), 2-methylpropan-1-yl (leucine), 1-methylpropan-1-yl (isoleucine), butan-1-yl (norleucine), tert-butyl (2-tert-butylglycine), phenyl (2-phenylglycine), benzyl (phenylalanine), p-hydroxybenzyl (tyrosine), indol-3-ylmethyl (tryptophan), imidazol-4-ylmethyl (histidine), hydroxymethyl (serine), 2-hydroxyethyl (homoserine), 1-hydroxyethyl (threonine), mercaptomethyl (cysteine), methylthiomethyl (S-methylcysteine), 2-mercaptoethyl (homocysteine), 2-methylthioethyl (methionine), carbamoylmethyl (asparagine), 2-carbamoylethyl (glutamine), carboxymethyl (aspartic acid), 2-carboxyethyl (glutamic acid), 4-aminobutan-1-yl (lysine), 4-amino-3-hydroxybutan-1-yl (hydroxylysine), 3-aminopropan-1-yl (ornithine), 2-aminoethyl (2,4-diaminobutyric acid), aminomethyl (2,3-diaminopropionic acid), 3-guanidinopropan-1-yl (arginine), 3-ureidopropan-1-yl (citrulline),
R² and R⁴ are independently of one another hydrogen or methyl
or
R¹ and R² or R³ and R⁴ are in each case linked to one another and, together with the carbon atom to which they are jointly attached, form a 3- to 6-membered saturated carbocycle,
R⁵ is hydrogen or (C₁-C₄)-alkyl
or
R⁵ is linked to R¹ and both, together with the atoms to which they are attached, form a pyrrolidine or piperidine ring,
R⁶ and R⁷ are identical or different and independently of one another are hydrogen or (C₁-C₄)-alkyl which may be substituted by hydroxyl, (C₁-C₄)-alkoxy, amino, mono- (C₁-C₄) -alkylamino or di-(C₁-C₄)-alkylamino
or
R⁶ and R⁷ are linked to one another and, together with the nitrogen atom to which they are attached, form a 5- or 6-membered saturated heterocycle which may comprise a further ring heteroatom from the series consisting of N and O, and may be substituted one or two times, identically or differently, by (C₁-C₄)-alkyl, amino, hydroxyl and/or (C₁-C₄)-alkoxy,
or
R⁶ is linked to R³ and both, together with the atoms to which they are attached, form a pyrrolidine or piperidine ring
and
R⁸ is hydrogen or carboxyl,
and the salts, solvates and solvates of the salts thereof.

2. Compound of the formula (I) according to Claim 1, in which
R^{PD} is a group of the formula 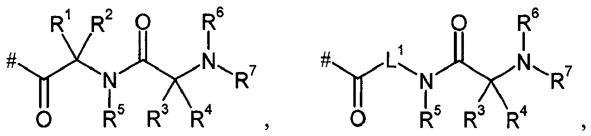 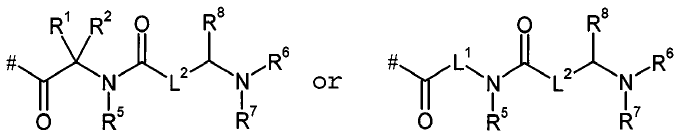 in which
# means the point of linkage to the respective O atom,
L¹ is ethane-1,2-diyl,
L² is methanediyl or ethane-1,2-diyl,
R¹ is hydrogen, methyl, propan-2-yl, 1-methylpropan-1-yl, 2-methylpropan-1-yl, benzyl, p-hydroxybenzyl, hydroxymethyl or 1-hydroxyethyl,
R² is hydrogen,
R³ is hydrogen, methyl, propan-2-yl, 1-methylpropan-1-yl, 2-methylpropan-1-yl, benzyl, imidazol-4-ylmethyl, hydroxymethyl, 1-hydroxyethyl, carbamoylmethyl, 2-carbamoylethyl, carboxymethyl, 2-carboxyethyl, 4-aminobutan-1-yl, 3-aminopropan-1-yl, 2-aminoethyl, aminomethyl or 3-guanidinopropan-1-yl,
R⁴ is hydrogen,
R⁵ is hydrogen or methyl
or
R⁵ is linked to R¹ and both, together with the atoms to which they are attached, form a pyrrolidine ring,
R⁶ is hydrogen or methyl
or
R⁶ is linked to R³ and both, together with the atoms to which they are attached, form a pyrrolidine ring,
R⁷ is hydrogen or methyl
and
R⁸ is hydrogen or carboxyl,
and the salts, solvates and solvates of the salts thereof.

3. Compound of the formula (I) according to Claim 1 or 2, in which
R^{PD} is a group of the formula 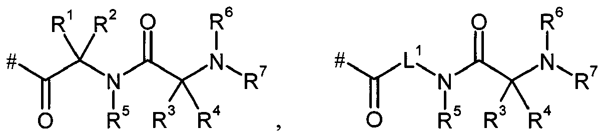 or 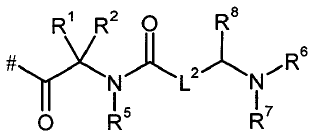 in which
# means the point of linkage to the respective O atom,
L¹ is ethane-1,2-diyl,
L² is methanediyl,
R¹ is hydrogen, methyl, propan-2-yl, 1-methylpropan-1-yl, 2-methylpropan-1-yl, hydroxymethyl or 1-hydroxyethyl,
R² is hydrogen,
R³ is hydrogen, methyl, propan-2-yl, 1-methylpropan-1-yl, 2-methylpropan-1-yl, imidazol-4-ylmethyl, hydroxymethyl, 1-hydroxyethyl, 2-carboxyethyl, 4-aminobutan-1-yl, 3-aminopropan-1-yl or 2-aminoethyl,
R⁴ is hydrogen,
R⁵ is hydrogen
R⁶ is hydrogen or methyl
or
R⁶ is linked to R³ and both, together with the atoms to which they are attached, form a pyrrolidine ring,
R⁷ is hydrogen
and
R⁸ is hydrogen,
and the salts, solvates and solvates of the salts thereof.

4. Compound of the formula (I) according to Claim 1, 2 or 3, in which the two groups R^{PD} are identical, and the salts, solvates and solvates of the salts thereof.

5. Compound according to Claim 1, 2, 3 or 4, with the formula (I-A) 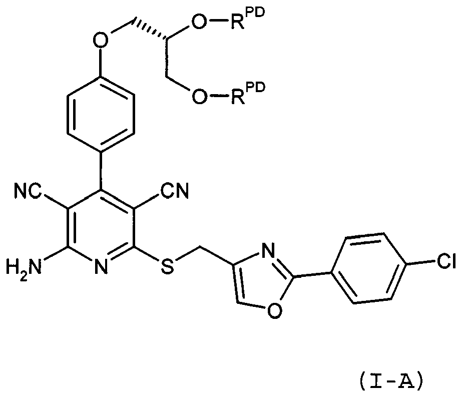 and the salts, solvates and solvates of the salts thereof.

6. Compound (2S)-3-{4-[2-amino-6-({[2-(4-chlorophenyl)-1,3-oxazol-4-yl]methyl}sulfanyl)-3,5-dicyanopyridin-4-yl]phenoxy}propane-1,2-diyl (2S,2'S)-bis{2-[(3-aminopropanoyl)amino]propanoate} dihydrochloride

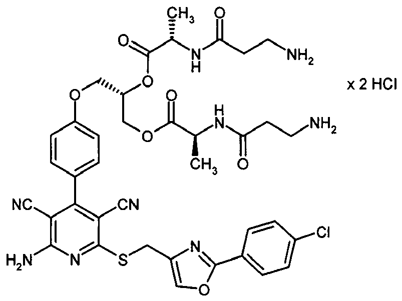

7. Compound (2S)-3-{4-[2-amino-6-({[2-(4-chloro-phenyl)-1,3-oxazol-4-yl]methyl}sulfanyl)-3,5-dicyano-pyridin-4-yl]phenoxy}propane-1,2-diyl (25,2'S)-bis(2-{[(2S)-2-aminopropanoyl]amino}propanoate) dihydrochloride

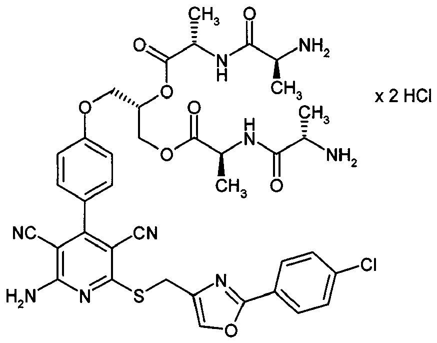

8. Compound (2S)-3-{4-[2-amino-6-({[2-(4-chlorophenyl)-1,3-oxazol-4-yl]methyl}sulfanyl)-3,5-dicyanopyridin-4-yl]phenoxy}propane-1,2-diyl (25,2'S)-bis(2-{[(2S)-2-aminopropanoyl]amino}propanoate) bis(4-methylbenzenesulfonic acid) salt

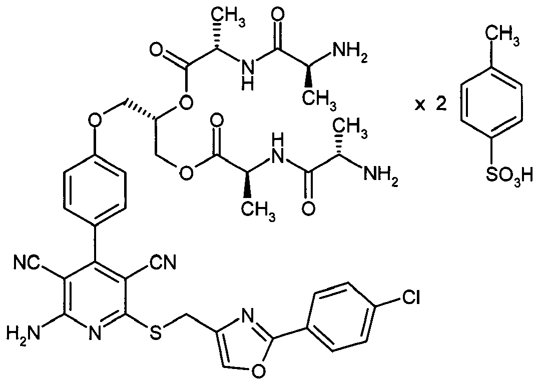

9. Compound (2S)-3-{4-[2-amino-6-({[2-(4-chlorophenyl)-1,3-oxazol-4-yl]methyl}sulfanyl)-3,5-dicyanopyridin-4-yl]phenoxy}propane-1,2-diyl (2S,2'S)-bis(2-{[(2R)-2-aminopropanoyl]amino}propanoate) dihydrochloride

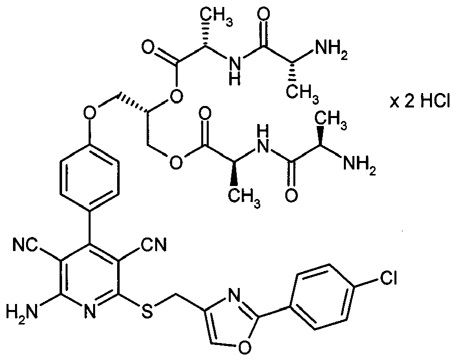

10. Compound (2S)-3-{4-[2-amino-6-({[2-(4-chlorophenyl)-1,3-oxazol-4-yl]methyl}sulfanyl)-3,5-dicyanopyridin-4-yl]phenoxy}propane-1,2-diyl (2R,2'R)-bis(2-{[(2R)-2-aminopropanoyl]amino}propanoate) dihydrochloride

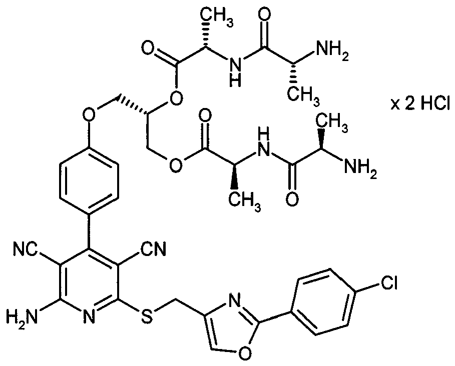

11. Process for preparing compounds of the formula (I) as defined in Claims 1 to 10, in which the two groups R^{PD} are each identical, **characterized in that** the compound (A) 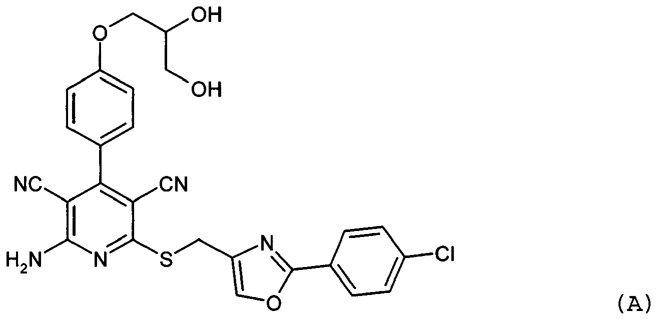 either
[A] is esterified in an inert solvent in the presence of a condensing agent initially with two or more equivalents of an amino acid of the formula (II) or (III) 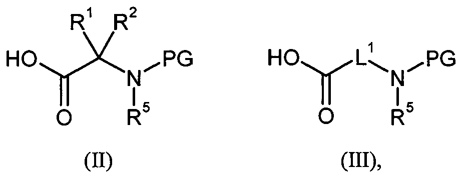 in which L¹, R¹, R² and R⁵ have the meanings indicated in Claims 1 to 5,
and
PG is a temporary amino protective group such as, for example, tert-butoxycarbonyl
to give a compound of the formula (IV) or (V) 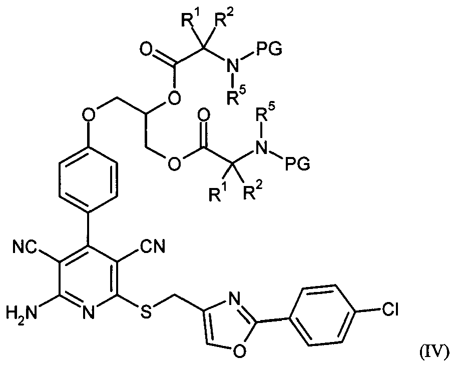 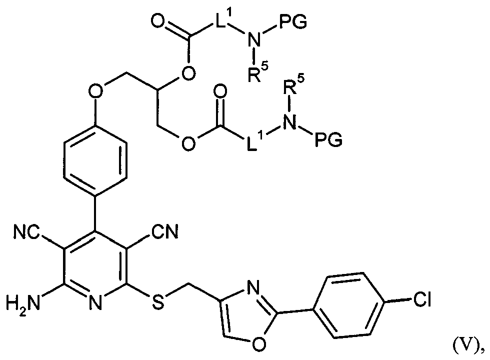 in which L¹, PG, R¹, R² and R⁵ have the meanings indicated above,
then, after elimination of the protective groups PG, said compound (A) is coupled in an inert solvent in the presence of a condensing agent with two or more equivalents of an amino acid of the formula (VI) or (VII) 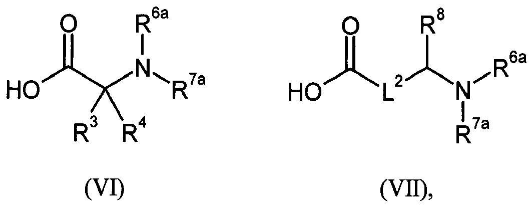 in which L², R³, R⁴ and R⁸ have the meanings indicated in Claims 1 to 5,
and
R^{6a} and R^{7a} are identical or different and have the meanings of respectively R⁶ and R⁷ indicated in Claims 1 to 5, or are a temporary amino protective group,
to give a compound of the formula (VIII), (IX), (X) or (XI) 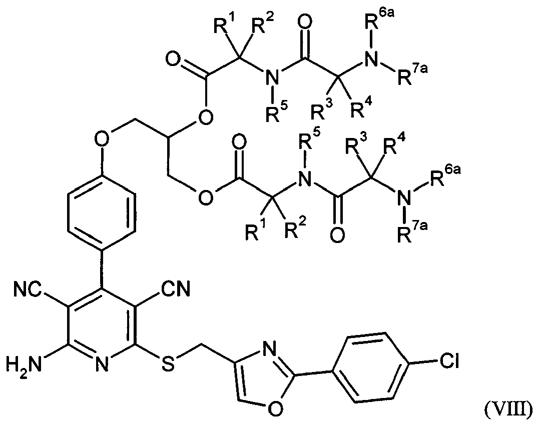 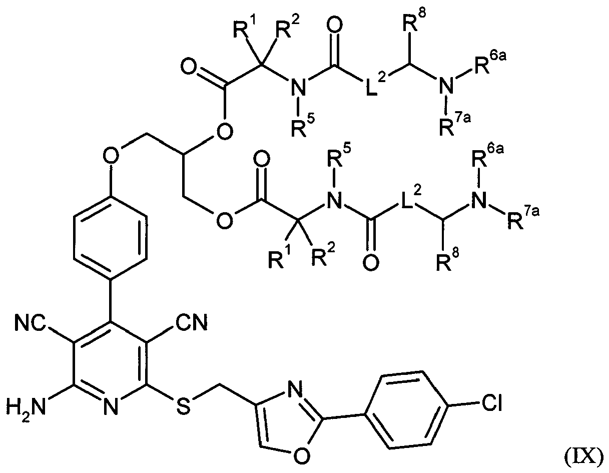 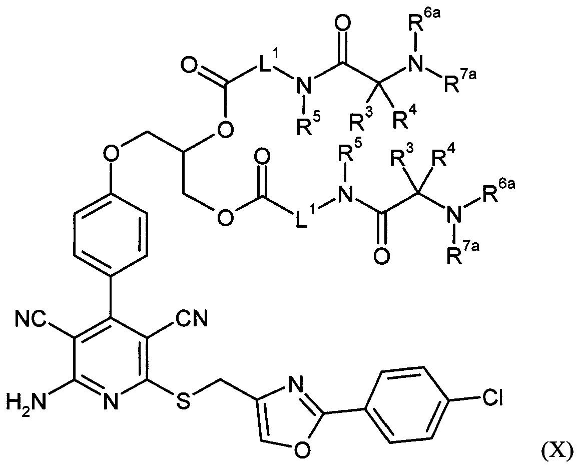 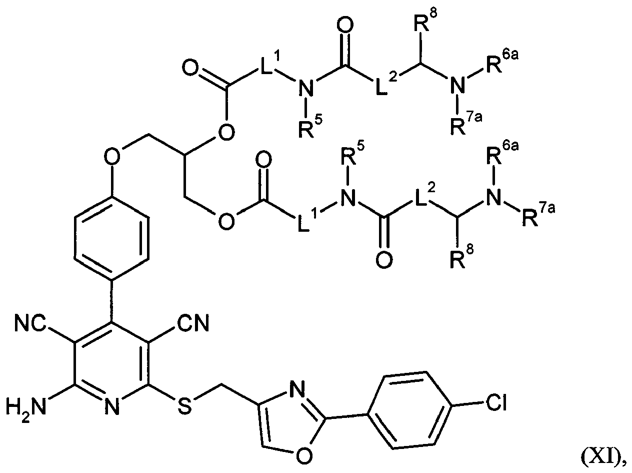 in which L¹, L², R¹, R², R³, R⁴, R⁵, R^{6a}, R^{7a} and R⁸ each have the meanings indicated above, and subsequently protective groups which are present where appropriate are removed again,
or
[B] is coupled in an inert solvent in the presence of a condensing agent with two or more equivalents of a carboxylic acid of the formula (XII), (XIII), (XIV) or (XV) 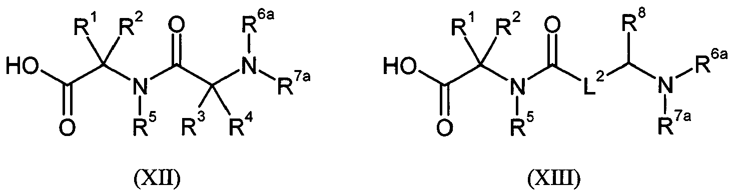 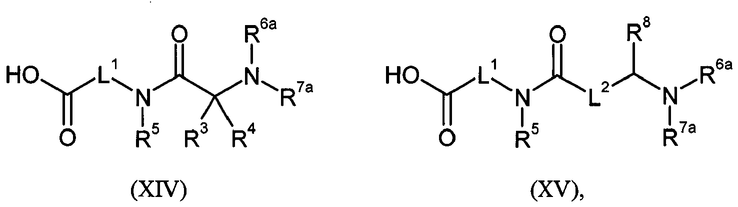 in which L¹, L², R¹, R², R³, R⁴, R⁵ and R⁸ have the meanings indicated in Claims 1 to 5,
and
R^{6a} and R^{7a} are identical or different and have the meanings of respectively R⁶ and R⁷ indicated in Claims 1 to 5, or are a temporary amino protective group,
to give one of the compounds (VIII), (IX), (X) or (XI) indicated in variant [A] and subsequently protective groups which are present where appropriate are removed again,
and the compounds of the formula (I) resulting in each case are converted where appropriate with the appropriate (i) solvents and/or (ii) acids or bases into the solvates, salts and/or solvates of the salts thereof.

12. Compound as defined in any of Claims 1 to 10 for the treatment and/or prophylaxis of diseases.

13. Compound as defined in any of Claims 1 to 10 for use in a method for the treatment and/or prophylaxis of cardiovascular diseases.

14. Use of a compound as defined in any of Claims 1 to 10 for the manufacture of a medicament for the treatment and/or prophylaxis of cardiovascular disorders.

15. Medicament comprising a compound as defined in any of Claims 1 to 10, where appropriate in combination with one or more inert, non-toxic pharmaceutically suitable excipients.

16. Medicament comprising a compound as defined in any of Claims 1 to 10 in combination with one or more further active ingredients.

17. Medicament according to Claim 15 or 16 for the treatment and/or prophylaxis of cardiovascular disorders.

## Revendications

1. Composé de formule (I) 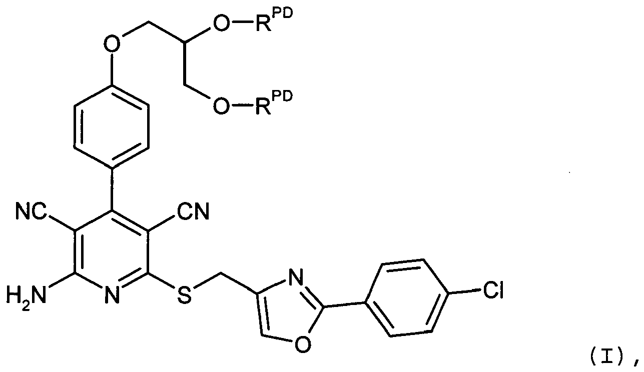 dans laquelle
R^{PD} représente un groupe de formule 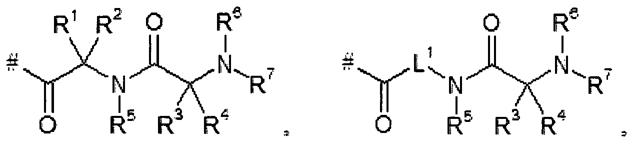 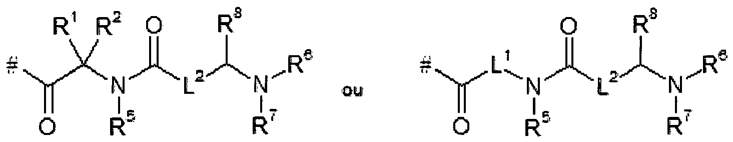 où
# représente la position de liaison à l'atome d'oxygène respectif,
L¹ représente un groupe alcane (C₂-C₄) diyle à chaîne droite,
L² représente un groupe alcane (C₁-C₃) diyle à chaîne droite,
R¹ et R³ sont identiques ou différents et représentent, indépendamment l'un de l'autre, un atome d'hydrogène ou un groupe méthyle (alanine), propan-2-yle (valine), propan-1-yle (norvaline), 2-méthylpropan-l-yle (leucine), 1-méthylpropan-1-yle (isoleucine), butan-1-yle (norleucine), *tert*.-butyle (2-*tert*.-butylglycine), phényle (2-phényl-glycine), benzyle (phénylalanine), *p*-hydroxybenzyle (tyrosine), indol-3-ylméthyle (tryptophane), imidazol-4-ylméthyle (histidine), hydroxyméthyle (sérine), 2-hydroxyéthyle (homosérine), 1-hydroxyéthyle (thréonine), mercaptométhyle (cystéine), méthylthiométhyle (*s*-méthylcystéine), 2-mercaptoéthyle (homocystéine), 2-méthyl-thioéthyle (méthionine), carbamoylméthyle (asparagine), 2-carbamoyléthyle (glutamine), carboxyméthyle (acide aspartique), 2-carboxy-éthyle (acide glutamique), 4-aminobutan-1-yle (lysine), 4-amino-3-hydroxybutan-1-yle (hydroxylysine), 3-aminopropan-1-yle (ornithine), 2-aminoéthyle (acide 2,4-diaminobutyrique), aminométhyle (acide 2,3-diaminopropionique), 3-guanidinopropan-1-yle (arginine), 3-uréidopropan-1-yle (citrulline),
R² et R⁴ représentent indépendamment l'un de l'autre un atome d'hydrogène ou le groupe méthyle ou
R¹ et R² ou R³ et R⁴ sont respectivement liés l'un à l'autre et forment ensemble avec l'atome de carbone, auquel ils sont liés ensemble, un carbocycle saturé à 3-6 chaînons,
R⁵ représente un atome d'hydrogène ou un groupe alkyle en (C₁-C₄) ou
R⁵ est lié à R¹ et les deux forment ensemble avec les atomes, auxquels ils sont liés, un cycle pyrrolidine ou pipéridine,
R⁶ et R⁷ sont identiques ou différents et représentent, indépendamment l'un de l'autre, un atome d'hydrogène ou un groupe alkyle en C₁-C₄ qui peut être substitué par hydroxy, alcoxy en C₁-C₄, amino, monoalkyl (C₁-C₄) amino ou dialkyl (C₁-C₄) amino
ou
R⁶ et R⁷ sont liés l'un à l'autre et forment ensemble avec l'atome d'azote, auquel ils sont liés, un hétérocycle saturé à 5 ou 6 chaînons, qui peut contenir un autre hétéroatome formant le cycle, choisi dans la série N et O et porter un ou deux substituants, identiques ou différents, alkyle en C₁-C₄, amino, hydroxy et/ou alcoxy en C₁-C₄,
ou
R⁶ est lié à R³ et les deux forment ensemble avec les atomes, auxquels ils sont liés, un cycle pyrrolidine ou pipéridine
et
R⁸ représente un atome d'hydrogène ou le groupe carboxy,
ainsi que ses sels, produits de solvatation et produits de solvatation des sels.

2. Composé de formule (I) selon la revendication 1, dans lequel
R^{PD} représente un groupe de formule 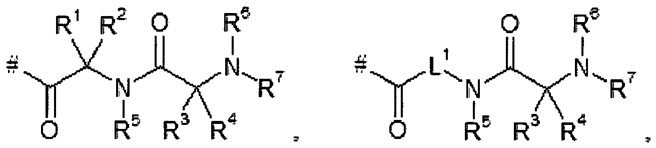 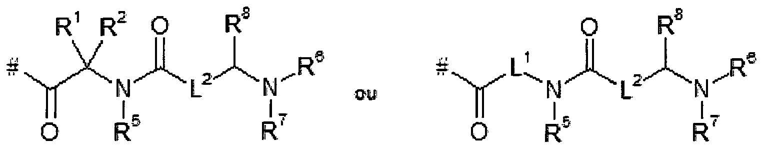 où
# représente la position de liaison à l'atome d'oxygène respectif,
L¹ représente le groupe éthane-1,2-diyle,
L² représente le groupe méthanediyle ou éthane-1,2-diyle,
R¹ représente un atome d'hydrogène, un groupe méthyle, propan-2-yle, 1-méthylpropan-1-yle, 2-méthylpropan-1-yle, benzyle, *p*-hydroxybenzyle, hydroxyméthyle ou 1-hydroxyéthyle,
R² représente un atome d'hydrogène,
R³ un atome d'hydrogène, un groupe méthyle, propan-2-yle, 1-méthylpropan-1-yle, 2-méthylpropan-1-yle, benzyle, imidazol-4-ylméthyle, hydroxyméthyle, 1-hydroxyéthyle, carbamoylméthyle, 2-carbamoyléthyle, carboxyméthyle, 2-carboxyéthyle, 4-aminobutan-1-yle, 3-aminopropan-1-yle, 2-aminoéthyle, aminométhyle ou 3-guanidinopropan-1-yle,
R⁴ représente un atome d'hydrogène,
R⁵ représente un atome d'hydrogène ou le groupe méthyle
ou
R⁵ est lié à R¹ et les deux forment ensemble avec les atomes, auxquels ils sont liés, un cycle pyrrolidine,
R⁶ représente un atome d'hydrogène ou le groupe méthyle
ou
R⁶ est lié à R³ et les deux forment ensemble avec les atomes, auxquels ils sont liés, un cycle pyrrolidine,
R⁷ représente un atome d'hydrogène ou le groupe méthyle
et
R⁸ représente un atome d'hydrogène ou le groupe carboxy,
ainsi que ses sels, produits de solvatation et produits de solvatation des sels.

3. Composé de formule (I) selon la revendication 1 ou 2, dans lequel
R^{PD} représente un groupe de formule 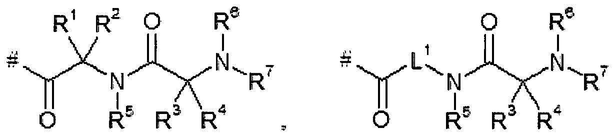 ou 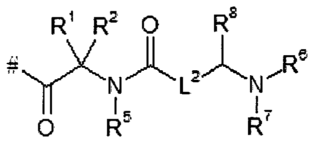 où
# représente la position de liaison à l'atome d'oxygène respectif,
L¹ représente le groupe éthane-1,2-diyle,
L² représente le groupe méthanediyle,
R¹ représente un atome d'hydrogène, le groupe méthyle, propan-2-yle, 1-méthylpropan-1-yle, 2-méthylpropan-1-yle, hydroxyméthyle ou 1-hydroxyéthyle,
R² représente un atome d'hydrogène,
R³ représente un atome d'hydrogène, le groupe méthyle, propan-2-yle, 1-méthylpropan-1-yle, 2-méthylpropan-1-yle, imidazol-4-ylméthyle, hydroxyméthyle, 1-hydroxyéthyle, 2-carboxyéthyle, 4-aminobutan-1-yle, 3-aminopropan-1-yle ou 2-aminoéthyle,
R⁴ représente un atome d'hydrogène,
R⁵ représente un atome d'hydrogène,
R⁶ représente un atome d'hydrogène ou le groupe méthyle
ou
R⁶ est lié à R³ et les deux forment ensemble avec les atomes, auxquels ils sont liés, un cycle pyrrolidine,
R⁷ représente un atome d'hydrogène
et
R⁸ représente un atome d'hydrogène,
ainsi que ses sels, produits de solvatation et produits de solvatation des sels.

4. Composé de formule (I) selon la revendication 1, 2 ou 3, dans lequel les deux groupes R^{PD} sont identiques, ainsi que ses sels, produits de solvatation et produits de solvatation des sels.

5. Composé selon la revendication 1, 2, 3 ou 4, de formule (I-A) 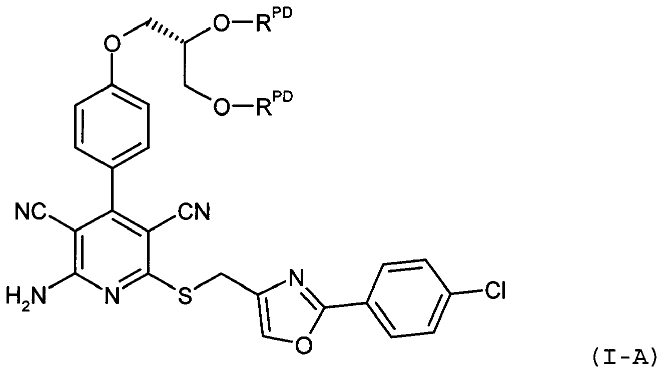 ainsi que ses sels, produits de solvatation et produits de solvatation des sels.

6. composé (2S,2'S)-bis{2-[(3-aminopropanoyl)-amino]propanoate} de (2S)-3-{4-[2-amino-6-({[2-(4-chlorophényl)-1,3-oxazol-4-yl]méthyl}sulfanyl)-3,5-dicyanopyridin-4-yl]phénoxy}propane-1,2-diyle-dichlorhydrate

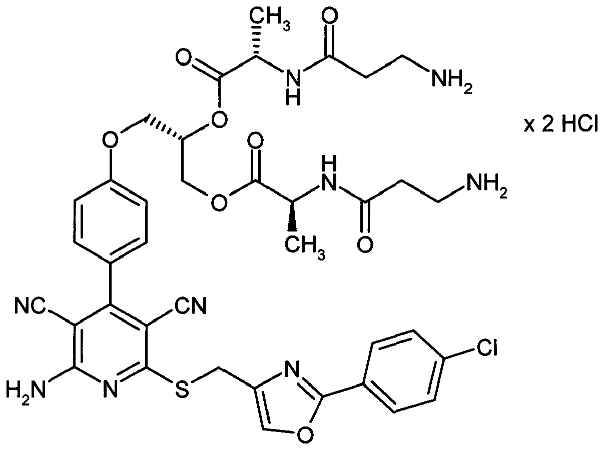

7. Composé (2S,2'S)-bis(2-{[(2S)-2-aminopropanoyl]amino}propanoate) de (2S)-3-{4-[2-amino-6-({[2-(4-chlorophényl)-1,3-oxazol-4-yl]méthyl}sulfanyl)-3,5-dicyanopyridin-4-yl]phénoxy}propane-1,2-diyle-dichlorhydrate

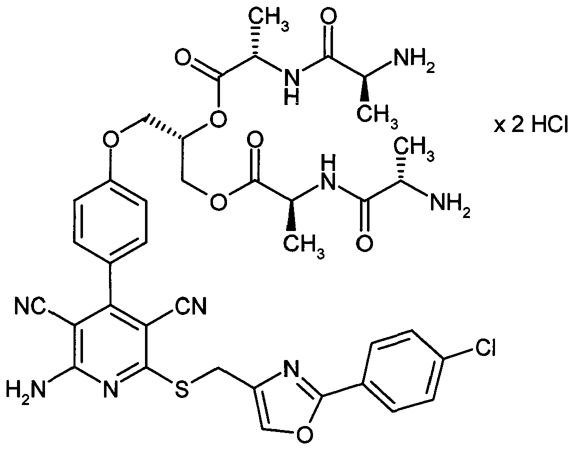

8. Composé *(2S,2'S)*-bis(2-{[(2S)-2-aminopropanoyl]amino}propanoate) de (2*S*)-3-[4-[2-amino-6-({[2-(4-chlorophényl)-1,3-oxazol-4-yl]méthyl}sulfanyl)-3,5-dicyanopyridin-4-yl]phénoxy]propane-1,2-diyle-sel d'acide bis(4-méthylbenzènesulfonique)

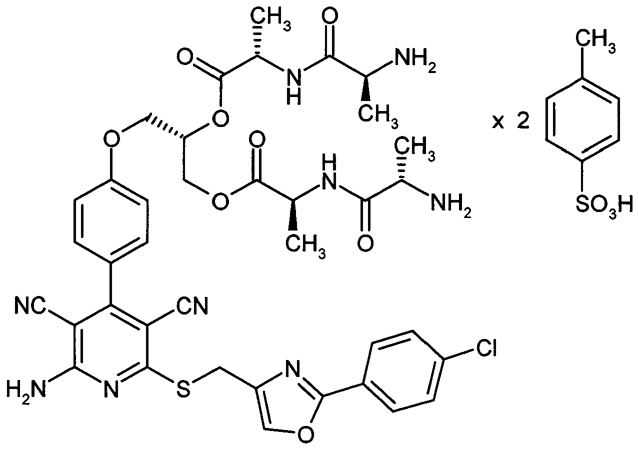

9. Composé (2*S*,2'*S*)-bis(2-{[(2*R*)-2-aminopropanoyl]amino}propanoate) de (2*S*)-3-{4-[2-amino-6-({[2-(4-chlorophényl)-1,3-oxazol-4-yl]méthyl}sulfanyl)-3,5-dicyanopyridin-4-yl]phénoxy}propane-1,2-diyle-dichlorhydrate

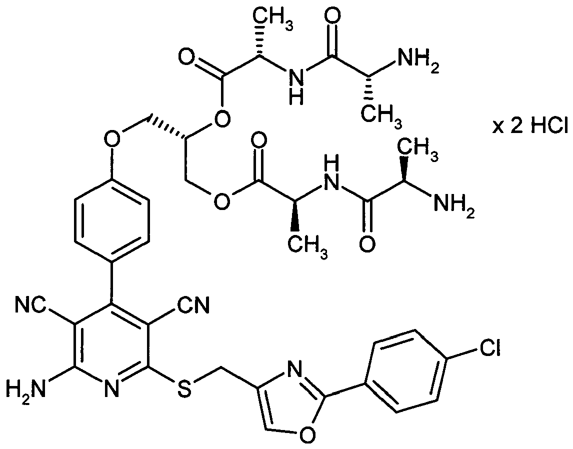

10. Composé (2*R*,2'*R*)-bis(2-([(2*R*)-2-aminopropanoyl]amino}propanoate) de (2*S*)-3-{4-[2-amino-6-({[2-(4-chlorophényl)-1,3-oxazol-4-yl]méthyl}-sulfanyl)-3,5-dicyanopyridin-4-yl]phénoxy}propane-1,2-diyle-dichlorhydrate

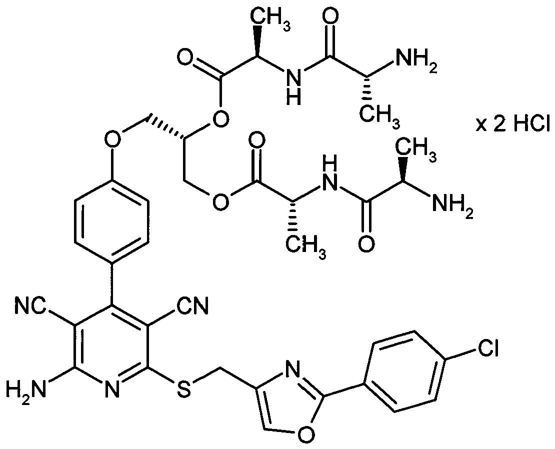

11. Procédé pour la préparation de composés de formule (I), tels que définis dans les revendications 1 à 10, dans lesquels les deux groupes R^{PD} sont chaque fois identiques, **caractérisé en ce qu'**on estérifie le composé (A) 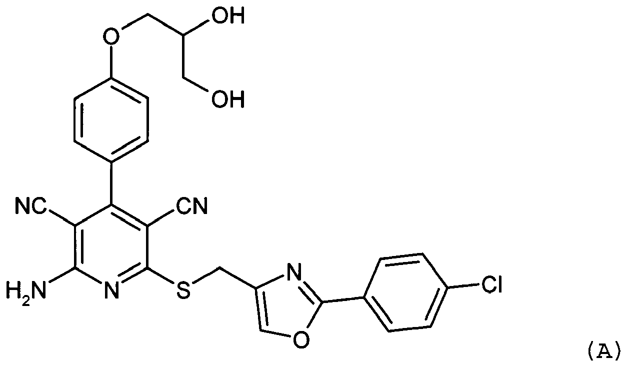 soit
[A] dans un solvant inerte, en présence d'un agent de condensation, d'abord avec deux ou plus de deux équivalents d'un acide aminé de formule (II) ou (III) 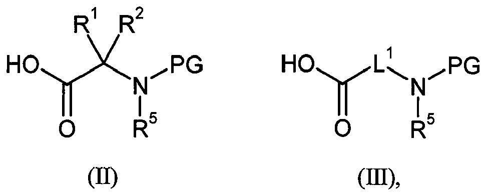 formules dans lesquelles L¹, R¹, R² et R⁵ ont les significations indiquées dans les revendications 1 à 5
et
PG représente un groupe protecteur temporaire de fonction amino, comme par exemple le groupe *tert*.-butoxycarbonyle,
pour obtenir un composé de formule (IV) ou respectivement (V) 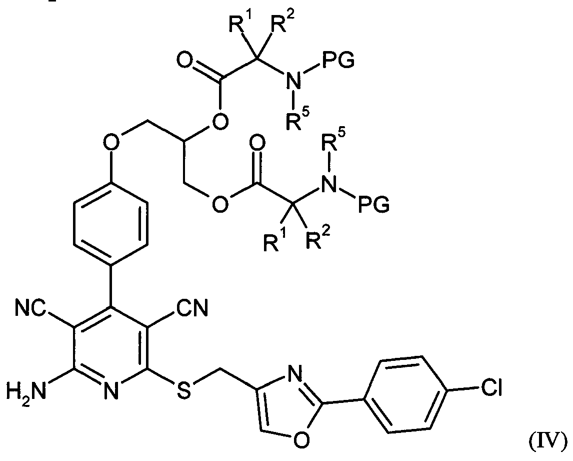 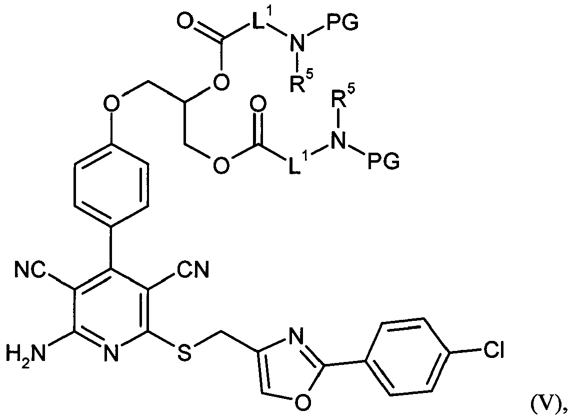 formules dans lesquelles L¹, PG, R¹, R² et R⁵ ont les significations indiquées plus haut, ensuite, après élimination des groupes protecteurs PG on le combine dans un solvant inerte, en présence d'un agent de condensation, avec deux ou plus de deux équivalents d'un acide aminé de formule (VI) ou (VII) 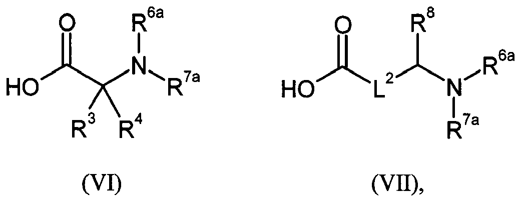 formules dans lesquelles L², R³, R⁴ et R⁸ ont les significations indiquées dans les revendications 1 à 5
et
R^{6a} et R^{7a} sont identiques ou différents et ont les significations de R⁶ ou respectivement R⁷ indiquées dans les revendications 1 à 5 ou représentent un groupe protecteur temporaire de fonction amino,
pour aboutir à un composé de formule (VIII), (IX), (X) ou (XI) 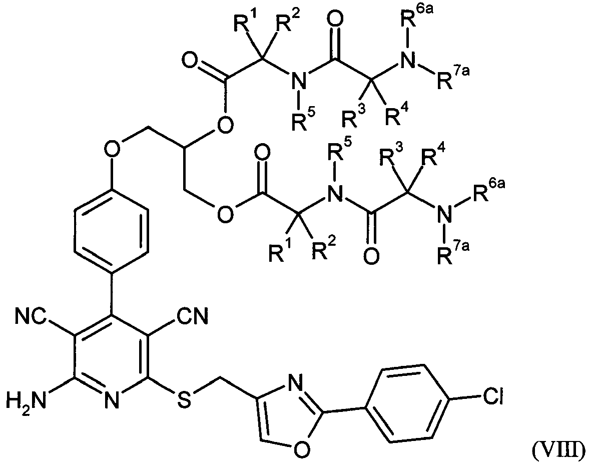 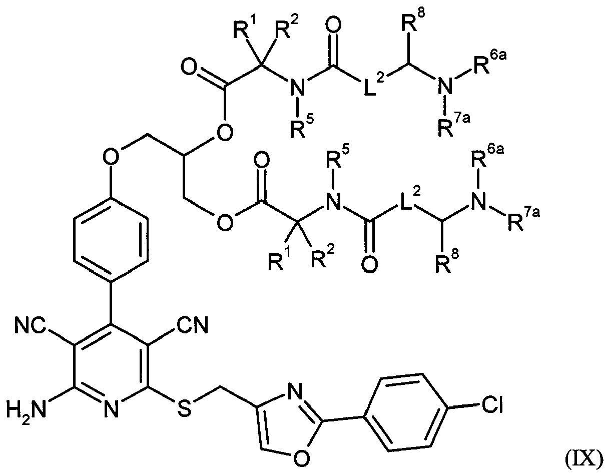 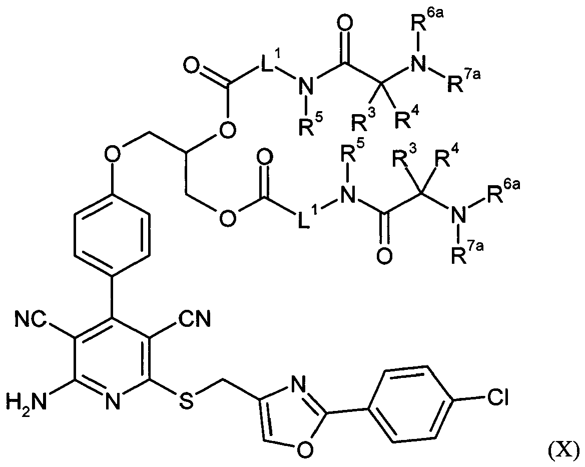 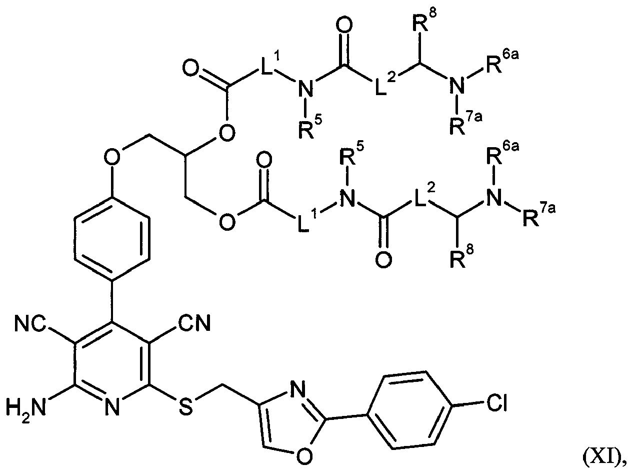 dans lesquelles L¹, L², R¹, R², R³, R⁴, R⁵, R^{6a}, R^{7a} et R⁸ ont chaque fois les significations indiquées plus haut,
et ensuite on élimine de nouveau les groupes protecteurs éventuellement présents soit
[B] dans un solvant inerte, en présence d'un agent de condensation, avec deux ou plus de deux équivalents d'un acide carboxylique de formule (XII), (XIII), (XIV) ou (XV) 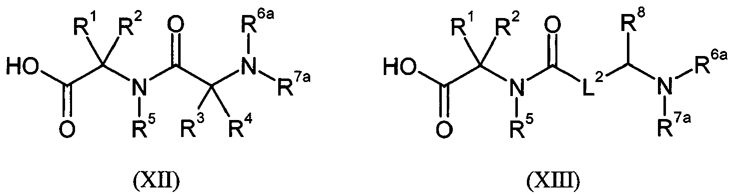 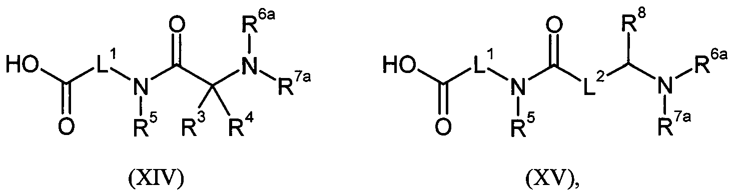 formules dans lesquelles L¹, L², R¹, R², R³, R⁴, R⁵ et R⁸ ont les significations indiquées dans les revendications 1 à 5
et
R^{6a} et R^{7a} sont identiques ou différents et ont les significations indiquées pour R⁶ ou respectivement R⁷ indiquées dans les revendications 1 à 5 ou représentent un groupe protecteur temporaire de fonction amino,
pour aboutir à l'un des composés (VIII), (IX), (X) ou (XI) indiqués dans la variante [A] et ensuite on élimine de nouveau les groupes protecteurs éventuellement présents
et éventuellement on convertit les composés de formule (I) résultants dans chaque cas, à l'aide des solvants (i) et/ou acides correspondants ou bases correspondantes (ii), en leurs produits de solvatation, sels et/ou produits de solvatation des sels.

12. Composé, tel que défini dans l'une quelconque des revendications 1 à 10, destiné au traitement et/ou à la prophylaxie de maladies.

13. Composé, tel que défini dans l'une quelconque des revendications 1 à 10, pour l'utilisation dans un procédé destiné au traitement et/ou à la prophylaxie de maladies cardiovasculaires.

14. Utilisation d'un composé, tel que défini dans l'une quelconque des revendications 1 à 10, pour la fabrication d'un médicament destiné au traitement et/ou à la prophylaxie de maladies cardiovasculaires.

15. Médicament contenant un composé tel que défini dans l'une quelconque des revendications 1 à 10, éventuellement en association avec un ou plusieurs adjuvants inertes, non toxiques, pharmaceutiquement appropriés.

16. Médicament contenant un composé tel que défini dans l'une quelconque des revendications 1 à 10, en association avec une ou plusieurs autres substances actives.

17. Médicament selon la revendication 15 ou 16, destiné au traitement et/ou à la prophylaxie de maladies cardiovasculaires.
